(19) Europäisches Patentamt / European Patent Office / Office européen des brevets

(11) **EP 4 752 902 A2**

(12) **EUROPEAN PATENT APPLICATION**

(43) Date of publication:
03.06.2026 Bulletin 2026/23

(51) International Patent Classification (IPC):
*G16H 40/63* (2018.01)

(21) Application number: 26162802.8

(22) Date of filing: 07.03.2025

(52) Cooperative Patent Classification (CPC):
G16H 40/40; G16H 10/40; G16H 40/63

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 07.03.2024 JP 2024035313
27.03.2024 JP 2024051365
27.03.2024 JP 2024051366

(62) Document number(s) of the earlier application(s) in accordance with Art. 76 EPC:
25162308.8 / 4 614 503

(71) Applicant: SYSMEX CORPORATION
Kobe-shi
Hyogo 651-0073 (JP)

(72) Inventors:
• **Tatsutani, Hiroo**
**Kobe-shi, Hyogo, 651-0073 (JP)**
• **Iwata, Hiroshi**
**Kobe-shi, Hyogo, 651-0073 (JP)**
• **Ariyoshi, Shunsuke**
**Kobe-shi, Hyogo, 651-0073 (JP)**
• **Kuwano, Keisuke**
**Kobe-shi, Hyogo, 651-0073 (JP)**
• **Kinishi, Motoi**
**Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

Remarks:
This application was filed on 06.03.2026 as a divisional application to the application mentioned under INID code 62.

(54) **SPECIMEN MEASUREMENT APPARATUS**

(57) Disclosed is a specimen measurement apparatus including a measurement unit configured to measure a specimen, the specimen measurement apparatus including: control software configured to control the measurement unit; data management software configured to manage data regarding the measurement unit that operates under control by the control software; and an interface configured to allow application software capable of being added to a system including the specimen measurement apparatus, to utilize the data, wherein a function of the system is expanded by the application software added to the system.

FIG. 3

**Description**

CROSS REFERENCE TO RELATED APPLICATIONS

This application claims priority from prior Japanese Patent Applications No. 2024-035313 filed on March 7, 2024, entitled "SYSTEM, APPLICATION SOFTWARE, AND LINKING METHOD" , No. 2024-051365 filed on March 7, 2024, entitled "SPECIMEN MEASUREMENT APPARATUS" , and No. 2024-051366 filed on March 7, 2024, entitled "DATA MANAGEMENT SYSTEM" , the entire contents of which are incorporated herein by reference.

FIELD OF THE INVENTION

The present invention relates to a specimen measurement apparatus that measures a specimen.

BACKGROUND OF THE INVENTION

International Publication No. WO2013/094485 discloses a specimen measurement system including a specimen measurement apparatus that performs specimen measurement and a control apparatus that is used for controlling the specimen measurement apparatus. The control apparatus displays information regarding the specimen measurement, and accepts a manipulation such as setting of the specimen measurement apparatus.

SUMMARY OF THE INVENTION

In the specimen measurement system of International Publication No. WO2013/094485, since information regarding a reagent is managed by software of the control apparatus and displayed on a display unit of the control apparatus, an user can grasp the information regarding the reagent displayed on the display unit and then manipulate the specimen measurement apparatus. In the International Publication No. WO2013/094485, programs regarding functions (e.g., the above mentioned function for managing the reagent) are embedded in the software. Therefore, if the software is to be expanded by adding a new function, in order to newly embed the new function into the software, a modification of the software may be necessary. In addition, when the software has been certified by a certification institution as a component of a medical device, it may become necessary to acquire the certification again in accordance with the modification. Due to these circumstances, expanding the functions of the software would not be easily performed in a conventional specimen measurement system.
An object of the present invention is to facilitate expanding the functions of the specimen measurement system.
A specimen measurement apparatus according to an aspect of the present invention is a specimen measurement apparatus including a measurement unit configured to measure a specimen. The specimen measurement apparatus includes: control software configured to control the measurement unit; data management software configured to manage data regarding the measurement unit that operates under control by the control software; and an interface configured to allow application software capable of being added to a system including the specimen measurement apparatus, to utilize the data. A function of the system is expanded by the application software added to the system.
According to the present invention, it is possible to facilitate expanding the functions of the specimen measurement system.

BRIEF DESCRIPTION OF THE DRAWINGS

[FIG. 1] FIG. 1 is a diagram showing components of a specimen measurement system according to the present embodiment.
[FIG. 2] FIG. 2 is a diagram showing a configuration example of a computer.
[FIG. 3] FIG. 3 is a diagram showing a configuration example 1 of the specimen measurement system according to the present embodiment.
[FIG. 4] FIG. 4 is a diagram showing a configuration example 2 of the specimen measurement system according to the present embodiment.
[FIG. 5] FIG. 5 is a diagram showing a configuration example 3 of the specimen measurement system according to the present embodiment.
[FIG. 6] FIG. 6 is a diagram showing a configuration example 4 of the specimen measurement system according to the present embodiment.
[FIG. 7] FIG. 7 is a diagram showing a configuration example 5 of the specimen measurement system according to the present embodiment.

[FIG. 8] FIG. 8 is a diagram showing a configuration example 6 of the specimen measurement system according to the present embodiment.

[FIG. 9] FIG. 9 is a diagram showing a configuration example 7 of the specimen measurement system according to the present embodiment.

[FIG. 10] FIG. 10 is a diagram showing a configuration example of a specimen measurement apparatus.

[FIG. 11] FIG. 11 is a diagram showing a hardware configuration example of a control unit.

[FIG. 12] FIG. 12 is a diagram showing configuration examples of control software and data management software.

[FIG. 13] FIG. 13 is a diagram showing a modification of the configuration example of the control unit.

[FIG. 14] FIG. 14 is a diagram showing a configuration example of a measurement unit.

[FIG. 15] FIG. 15 is a diagram showing a configuration example when the measurement unit is a blood cell analyzer.

[FIG. 16] Fig. 16 is a schematic diagram for explaining a specimen suction part and a sample preparation part when a measurement sample is supplied to an FCM detection part.

[FIG. 17] Fig. 17 is a schematic diagram for explaining a specimen suction part and a sample preparation part when a measurement sample is supplied to an RBC/PLT detection part.

[FIG. 18] FIG. 18 is a schematic diagram for explaining a specimen suction part and a sample preparation part when a measurement sample is supplied to an HGB detection part.

[FIG. 19] FIG. 19 is a diagram showing a configuration example when the measurement unit is a urine particle apparatus.

[FIG. 20] FIG. 20 is a diagram showing a configuration example of a detection part.

[FIG. 21] FIG. 21 is a diagram showing a configuration example when the measurement unit is a blood coagulation analyzer.

[FIG. 22] FIG. 22 is a diagram showing a configuration example of a light irradiation part of the detection part.

[FIG. 23] FIG. 23 is a diagram showing a configuration example of an optical detection part of the detection part.

[FIG. 24] FIG. 24 is a diagram showing a configuration example when the measurement unit is an immunoassay apparatus.

[FIG. 25] FIG. 25 is a diagram showing an example of the flow of a process performed by the sample preparation part.

[FIG. 26] FIG. 26 is a diagram showing a configuration example of an interface.

[FIG. 27] FIG. 27 is a flow chart showing an example of the flow (linking method) of the process performed by the interface of the specimen measurement apparatus.

[FIG. 28] FIG. 28 is a diagram showing a configuration example of an interface of a data management system.

[FIG. 29] FIG. 29 is a diagram showing an example of the data structure of an HTTP request body when lot information of quality control is updated.

[FIG. 30] FIG. 30 is a diagram showing examples of a request for information regarding the specimen measurement apparatus/a response to the request.

[FIG. 31] FIG. 31 is a diagram showing examples of a request for information regarding specimen measurement/a response to the request.

[FIG. 32] FIG. 32 is a diagram showing examples of a request for information regarding maintenance of the specimen measurement apparatus/a response to the request.

[FIG. 33] FIG. 33 is a diagram showing examples of a request regarding manipulation of the specimen measurement apparatus.

[FIG. 34] FIG. 34 is a diagram showing a configuration example of a PUSH notification from the specimen measurement apparatus to the data management system.

[FIG. 35] FIG. 35 is a diagram showing examples of an event and examples of information (information to serve as the target of PUSH notification) corresponding to the event.

[FIG. 36] FIG. 36 is a diagram showing a configuration example of an application.

[FIG. 37] FIG. 37 is a diagram showing a configuration example of when the application is a reagent management application.

[FIG. 38] FIG. 38 is a diagram showing an example of providing reagent information through a GUI.

[FIG. 39] FIG. 39 is a diagram showing a configuration example when the application is a specimen tracking application.

[FIG. 40] FIG. 40 is a diagram showing an example of providing measurement data through a GUI.

[FIG. 41] FIG. 41 is a diagram showing a configuration example when the application is a quality control application.

[FIG. 42] FIG. 42 is a diagram showing an example of providing quality control data through a GUI.

[FIG. 43] FIG. 43 is a diagram showing a configuration example when the application is a centralized management application.

[FIG. 44] FIG. 44 is a diagram showing an example of providing a centralized management data through a GUI.

[FIG. 45] FIG. 45 is a diagram showing an example of providing a centralized management data through a GUI.

[FIG. 46] FIG. 46 is a diagram showing a configuration example of providing the function of the application as a Web service.

[FIG. 47] FIG. 47 is a diagram showing another configuration example of providing the function of the application as a Web service.

[FIG. 48] FIG. 48 is a diagram showing still another configuration example of providing the function of the application as a Web service.

[FIG. 49] FIG. 49 is a diagram showing a configuration example of an application providing server that provides the application.

[FIG. 50] FIG. 50 is a diagram showing a configuration example of providing the application in a secure communication environment.

[FIG. 51] FIG. 51 is a diagram showing a configuration example of providing the application by utilizing MDM or MAM.

[FIG. 52] FIG. 52 is a diagram showing an example of a data structure when a database of an MDM/MAM system is a relational database.

DETAILED DESCRIPTION

(Components of specimen measurement system)

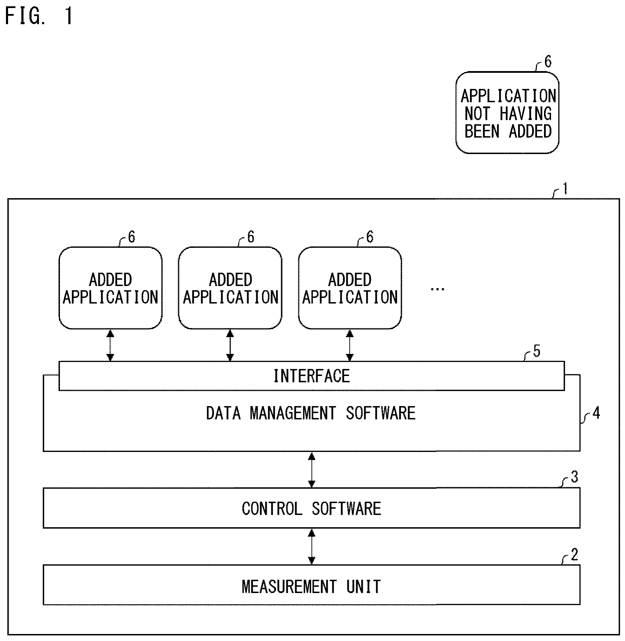

As shown in FIG. 1, a specimen measurement system 1 according to the present embodiment includes, as an example, a measurement unit 2, control software 3, data management software 4, and an interface 5, as components. The control software 3 and the data management software 4 may be integrally formed. For example, the specimen measurement system 1 is the specimen measurement apparatus including the measurement unit 2, the control software 3, the data management software 4, and the interface 5. For example, the specimen measurement system 1 is a system including: the specimen measurement apparatus including the measurement unit 2, the control software 3, the data management software 4, and the interface 5, other apparatuses; and other pieces of software.

The measurement unit 2 measures a specimen allocated to the measurement unit 2, and acquires data corresponding to a measurement result. For example, the measurement unit 2 may be (i) a unit configured to execute a measurement for blood cell analysis, (ii) a unit configured to execute a measurement for blood coagulation analysis, (iii) a unit configured to execute a measurement for urine particle analysis, (iv) a unit configured to execute a measurement for immunoassay, and the like. The measurement unit 2 operates under control by the control software 3. The measurement unit 2 provides acquired data to the data management software 4 via the control software 3.

The control software 3 controls the operation of the measurement unit 2 in cooperation with the data management software 4. The control software 3 orders the measurement unit 2 to execute measurement operation corresponding to a measurement order with respect to the specimen allocated to the measurement unit 2. The control software 3 may analyze data acquired by the measurement unit 2, and acquires measurement data (i.e., a measurement result) .

The data management software 4 manages data regarding the measurement unit 2. The data management software 4 has a function (for example, UI: User interface) of providing the measurement result acquired by the control software 3, to an operator. The data management software 4 has a function to operate the specimen measurement apparatus such as registration and acquisition of a measurement order, and a function of providing information regarding the measurement order to the control software 3. The data management software 4 may classify the data acquired by the measurement unit 2, and may manage the data based on the classification. For example, the data management software 4 may classify and manage the data according to a type of the measurement unit 2. For example, the type of the measurement unit 2 may include a unit configured to execute a measurement for blood cell analysis, a unit configured to execute a measurement for blood coagulation analysis, a unit configured to execute a measurement for urine particle analysis, and a unit configured to execute a measurement for immunoassay. The control software 3 and the data management software 4 may be different software which are independent with each other. Software having a function equivalent to that of the control software 3 and a function equivalent to that of the data management software 4 may execute control of the measurement unit 2 and management of data regarding the measurement unit 2.

The measurement unit 2, the control software 3, and the data management software 4 described above may require certification by a certification institution as a medical device, for example. A medical device requiring certification is an in vitro diagnostic medical device, for example. In this case, the measurement unit 2, the control software 3 and the data management software 4 provide functions corresponding to the intended use of the certified medical device, for example. The intended use of the medical device is to measure a specimen and provide a measurement result, for example. When the medical device is a blood cell analyzer, for example, the intended use is to measure a blood specimen and provide a measurement result (for example, red blood cell count, white blood cell count, white blood cell classification, and the like) regarding blood cells.

The application software (hereinafter, simply referred to as "application") 6 is software independent of the control software 3 and the data management software 4. For example, the application 6 has a function having been incorporated in software of a prior art regarding an apparatus for measuring a specimen. That is, the application 6 has a function separated from the software of the prior art regarding the apparatus for measuring a specimen. For example, the application 6 may have a new function that was not incorporated in the software of the prior art regarding the apparatus for measuring a specimen. For example, the application 6 provides a function different from the function corresponding to the "Intended Use" of the medical device. Examples of the application 6 are software for a healthcare usage other than a medical usage (hereinafter, referred to as "non-medical device software") and software for non-healthcare usage (hereinafter, referred to as "non-health software"). For example, the non-medical device software and the non-health software are software that does not require certification or that requires limited certification as a medical device. The application 6 may be an application requiring certification as the medical device.

The application 6 is capable of being added to the specimen measurement system 1. In other words, the application 6 is capable of being add-on to the specimen measurement system 1. The application 6 added to the specimen measurement system 1 serves as a component of the specimen measurement system 1. For example, the application 6 added to the specimen measurement system 1 provides a function of utilizing information (data regarding the measurement unit 2) managed by the data management software 4, and a function of utilizing the function of the data management software 4. For example, the functions provided by the application 6 are (i) a function that the data management software 4 does not have, and (ii) a function that expands an existing function of the data management software 4. The functions provided by the application 6 are new functions different from the function corresponding to the intended use as the medical device, for example. The application 6 added to the specimen measurement system 1 may provide a function for expanding the specimen measurement system 1. In other words, the application 6 added to the specimen measurement system 1 may be said to provide a function that complements the data management software 4.

Since the application 6 is separated/isolated from the data management software 4 and the control software 3, the application 6 may be easily added to the specimen measurement system 1 without modifying the data management software 4 and the control software 3. Moreover, for example, if the application 6 is the non-medical device software or the non-health software that does not require certification as the medical device, it may not be necessary to acquire the certification by the certification institution when the application 6 is added to the specimen measurement system 1. When the application 6 is to be deleted from the specimen measurement system 1 as well, it may not be necessary to modify the data management software 4 and the control software 3.

The application 6 separated/isolated from the data management software 4 is capable of communicating with the data management software 4 via the interface 5. For example, the interface 5 is a software interface. For example, the interface 5 is an API (Application Programming Interface). For example, the interface 5 allows the application 6 to utilize information (for example, data such as measurement data regarding the measurement unit 2) managed by the data management software 4 and the function of the data management software 4. For example, the interface 5 accepts a request from the application 6 and performs a response corresponding to the request against the application 6, thereby allowing the application 6 to utilize data corresponding to the request or utilize a function corresponding to the request. The request from the application 6 is a request for utilization of data managed by the data management software 4 and/or a request for utilization of a function of the data management software 4, for example. The response to the application 6 is provision of data corresponding to the request and/or provision of a function corresponding to the request, for example.

The application 6 may communicate with the interface 5 in accordance with a predetermined rule (hereinafter, referred to as "Rule R") defined in advance. The interface 5 may allow the application 6 to utilize the information/function regarding the data management software 4 according to the rule R. For example, when the interface 5 is configured as a Web API, the rule R is, as an example, determined as a combination of a URI (Uniform Resource Identifier) for designating a processing subject by a predetermined syntax, and a predetermined HTTP (Hyper Text Transfer Protocol) method indicating manipulation with respect to the processing subject. According to the rule R, the application 6 creates, as a request to the interface 5, a combination of a URI and an HTTP method that the processing subject and manipulation with respect to the processing subject are specified. Hereinafter, the request is also referred to as "command C". The application 6 transmits a command C created according to the rule R to the interface 5. The interface 5 provides a response according to the command C transmitted from the application 6, to the application 6. For example, according to the command C transmitted from the application 6, the interface 5 allows the application 6 to utilize at least one of: (i) data regarding the measurement unit 2, (ii) data regarding measurement operation by the measurement unit 2, (iii) data regarding maintenance of the measurement unit 2, and (iv) data regarding manipulation of the measurement unit 2. Further, for example, according to the command C transmitted from the application 6, the interface 5 allows the application 6 to execute at least one of: (i) acquisition of data regarding the measurement unit 2, (ii) registration of the data, (iii) update of the data, and (iv) deletion of the data. For example, there may be a plurality of types of rules R depending on the type of the specimen measurement apparatus 11. For example, manners of information management of the data management software 4 and the data management system 15 may differ depending on the type of the specimen measurement apparatus 11. In such a case, for example, if the application 6 uses information corresponding to a certain type of the specimen measurement apparatus 11, the application 6 communicates with the interface 5/5A according to a corresponding type of the rule R (i.e., the rule R corresponding to the certain type of the specimen measurement apparatus 11).

Therefore, the application 6 may be able to use a plurality of types of information each of which are respectively different with respect to the manners of the information management.

The interface 5 may allow a plurality of types of applications 6 to utilize the data regarding the measurement unit 2 according to the rule R which is common among the plurality of types of applications 6. The plurality of types of applications 6 may provide different functions. The rule 5 may be common among the applications 6 which operate in different operating systems, respectively. For example, the interface 5 may allow the applications 6 to utilize data regarding the measurement unit 2 according to the rule R which is common among a plurality of types of the applications 6 respectively created for a plurality of types of operating systems.

The interface 5 may be incorporated in the data management software 4 or may be incorporated in a software program different from the data management software 4. The interface 5 may be software independent of (i) the data management software 4, (ii) the control software 3, and (iii) other software programs. The data management software 4 may be configured as one piece of software (for example, single executable file whose extension is ".exe"), or may be configured as a plurality of pieces of software (for example, a plurality of executable files whose extensions are ".exe"). For example, the software configured as the interface 5 and the software configured as the data management software 4 may be configured as a plurality of different executable files.

(Example of software operating environment)

The control software 3, the data management software 4, the interface 5, and the application 6 are executed, for example, in one or more computers 900. For example, the computer 900 may be (i) a smartphone (for example, iPhone, Android terminal), (ii) a tablet (for example, iPad, Android tablet, Windows tablet), (iii) a Windows PC having Windows OS installed therein, or (iv) a Mac having Mac OS installed therein (iPhone, iPad, Android, Windows, and Mac OS are each a registered trademark). The application 6 may be created for a plurality of types of operating systems, for example. For example, with respect to a certain application 6, one for Windows OS and one for iOS may be respectively created. That is, the application 6 may be created for a plurality of operating systems.

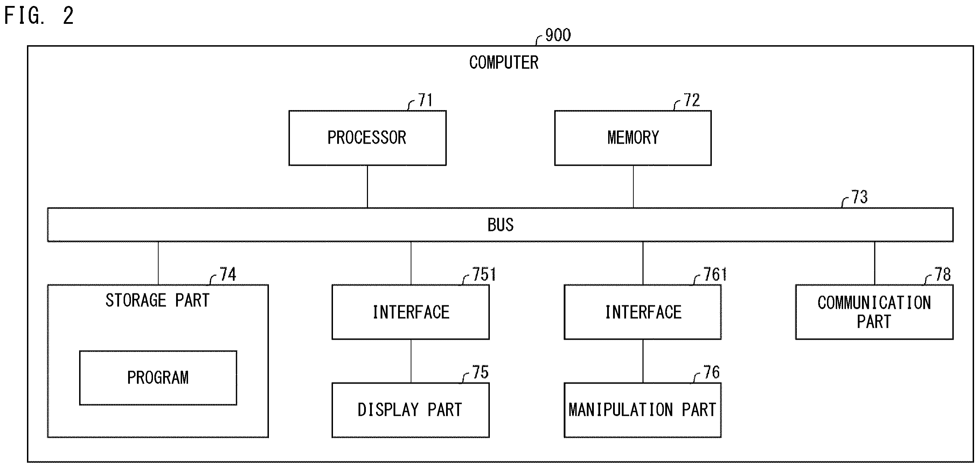

A configuration example of the computer 900 is shown in FIG. 2. As an example, the computer 900 includes a processor 71, a memory 72, a bus 73, a storage part 74, a display part 75, an interface 751, a manipulation part 76, an interface 761, and a communication part 78. A terminal apparatus 101 and a control unit 7, which will be described later, have a configuration similar to that of the computer 900 shown in FIG. 2.

The storage part 74 is, for example, a nonvolatile memory such as an SSD (Solid State Drive), a flash memory, an HDD (Hard Disk Drive), or a combination of these. The storage part 74 stores therein, for example, various types of programs including the control software 3, the data management software 4, the interface 5, and the application 6, and the like, and various types of data.

The processor 71 is, for example, an IPU (Infrastructure Processing Unit), a CPU (Central Processing Unit), a GPU (Graphic Processing Unit), a DSP (Digital Signal Processor), an MPU (Micro Processing Unit), or a combination of these. The processor 71 may be realized by a logic circuit, for example. The processor 71 reads out various types of programs from the storage part 74 to be expanded into the memory 72, to execute the programs. The memory 72 is, for example, a volatile memory such as a RAM (Random Access Memory) or a DRAM (Dynamic Random Access Memory).

The interface 751 connects the bus 73, and the display part 75 such as a display to each other. The interface 761 connects the bus 73, and a manipulation part 76 such as a keyboard and a mouse to each other. The communication part 78 is responsible for communication with an external apparatus.

Hereinafter, in order to simplify description of operation of the control software 3, the data management software 4, the interface 5, or the application 6, description may be given with omission of members respectively corresponding to the processor 71, the storage part 74, and the memory 72 for executing these.

The computer 900 may further include a communication interface for communicating with other apparatuses. The computer 900 may further include an input/output interface for connecting input/output devices such as a keyboard, a mouse, a display, and a printer.

(Configuration examples of specimen measurement system)

(Configuration Example 1)

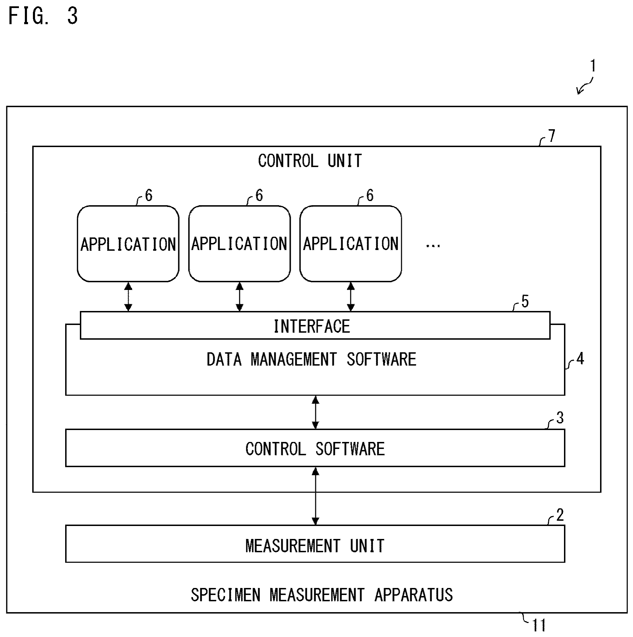

FIG. 3 shows a configuration example of the specimen measurement system 1. In the configuration example shown in the figure, the specimen measurement system 1 is configured as the specimen measurement apparatus 11 including a measurement unit 2, and the control unit 7 that executes the control software 3, the data management software 4, the interface 5, and the application 6. In the case of the present configuration example, the application 6 is executed in the specimen measurement apparatus 11, and utilizes, via the interface 5, information managed by the data management software 4 and the function of the data management software 4.

(Configuration Example 2)

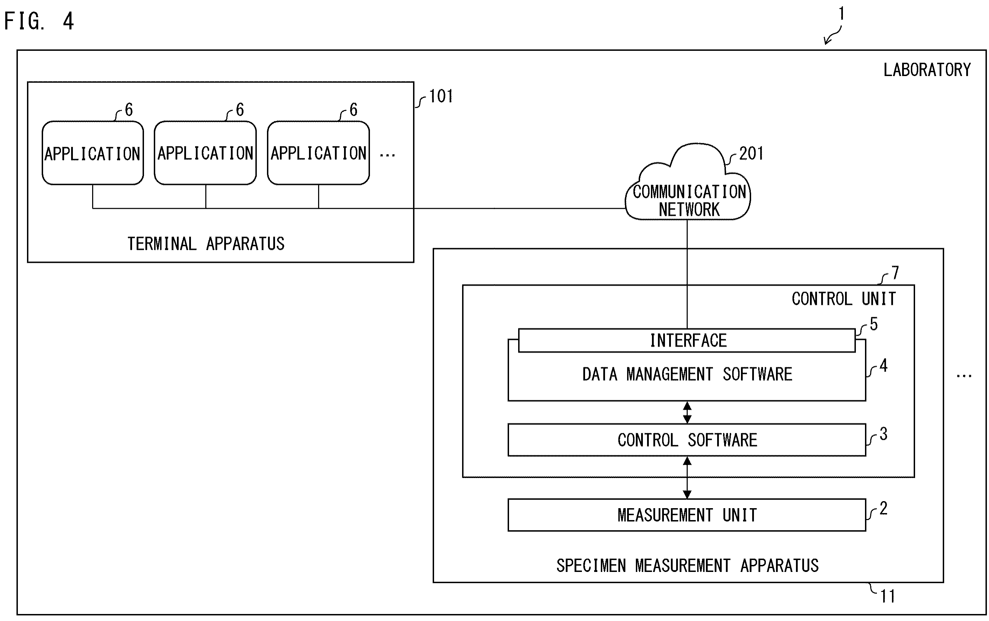

The application 6 may be installed in an apparatus different from the specimen measurement apparatus 11. In the configuration example shown in FIG. 4, the specimen measurement system 1 includes: a specimen measurement apparatus 11 placed in a laboratory; and a terminal apparatus 101 used in the laboratory and connected to the specimen measurement apparatus 11 via a communication network 201. The communication network 201 is, for example, an intra network.

The specimen measurement apparatus 11 includes: the measurement unit 2; and the control unit 7 that executes the control software 3, the data management software 4, and the interface 5. The terminal apparatus 101 is a computer used by a user of the specimen measurement system 1, and is, for example, a computer such as a PC, a smartphone, or a tablet. The terminal device 101 has the application 6 installed therein and executes the application 6.

In the case of the present configuration example, the application 6 executed in the terminal apparatus 101 accesses the interface 5 of the specimen measurement apparatus 11 via the communication network 201. The interface 5 allows the application 6 of the terminal apparatus 101 to utilize information managed by the data management software 4 and the function of the data management software 4, via the communication network 201.

(Configuration Example 3)

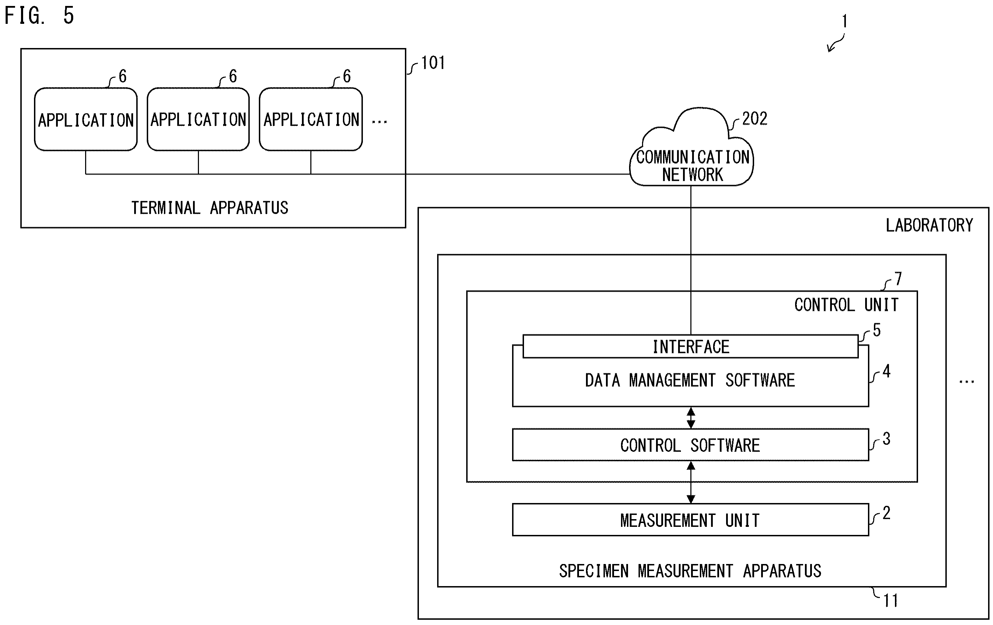

The terminal apparatus 101 including the application 6 installed therein may be used in a location (for example, outside the laboratory, another room in the same facility where the laboratory is present) different from the facility where the specimen measurement apparatus 11 is placed. In the configuration example shown in FIG. 5, the specimen measurement system 1 includes: the specimen measurement apparatus 11 placed in a laboratory; and a terminal apparatus 101 used in a facility different from the laboratory and connected to the specimen measurement apparatus 11 via a communication network 202. The communication network 202 is, for example, the Internet or an intranet. The specimen measurement apparatus 11 includes: the measurement unit 2; and the control unit 7 that executes the control software 3, the data management software 4, and the interface 5. The terminal apparatus 101 includes the application 6 installed therein and executes the application 6.

In the case of the present configuration example, the application 6 executed in the terminal apparatus 101 accesses the interface 5 of the specimen measurement apparatus 11 via the communication network 202. The interface 5 allows the application 6 of the terminal apparatus 101 to utilize information managed by the data management software 4 and the function of the data management software 4, via the communication network 202.

(Configuration Example 4)

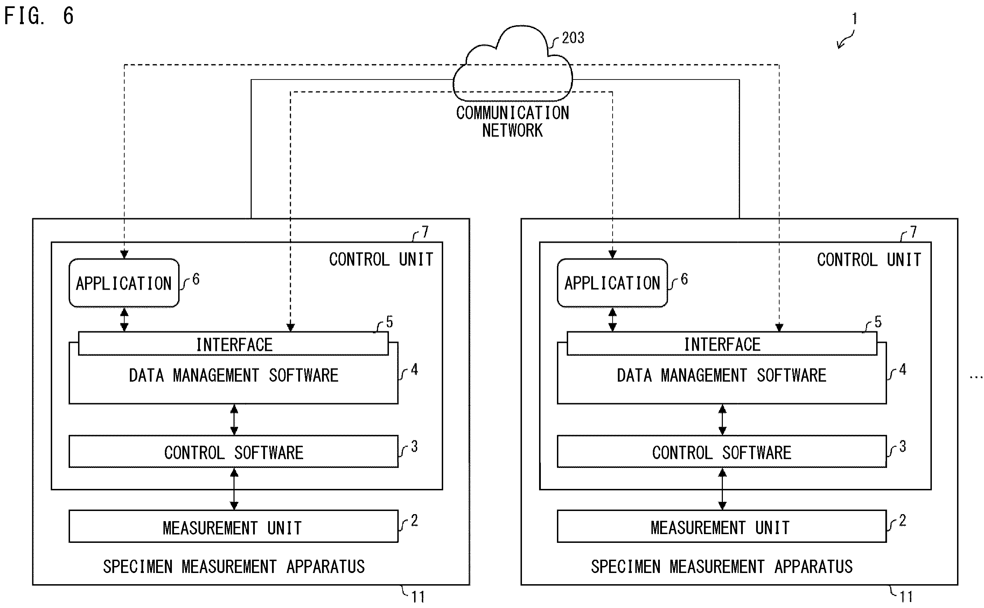

When the specimen measurement apparatus 11 includes the application 6 installed therein, the application 6 itself installed in the specimen measurement apparatus 11 may access another specimen measurement apparatus 11 (hereinafter, it may be called as "second specimen measurement apparatus 11") and utilize information managed by the data management software 4 of the other specimen measurement apparatus 11 and the function of the data management software 4 of the second specimen measurement apparatus 11. In the configuration example shown in FIG. 6, the specimen measurement system 1 has a configuration in which the specimen measurement apparatuses 11 that each include: the measurement unit 2; and the control software 3, the data management software 4, the interface 5, and the control unit 7 that executes the application 6 are communicably connected to each other via the communication network 203. The communication network 203 is, for example, an intra network or the Internet.

In the case of the present configuration example, the application 6 executed in the specimen measurement apparatus 11 can utilize, via the interface 5 of the specimen measurement apparatus 11, information managed by the data management software 4 of the specimen measurement apparatus 11 and the function of the data management software 4, and can also utilize, by accessing the interface 5 of the second specimen measurement apparatus 11 connected via the communication network 203, information managed by the data management software 4 of the second specimen measurement apparatus 11 and the function of the data management software 4 of the second specimen measurement apparatus 11.

(Configuration Example 5)

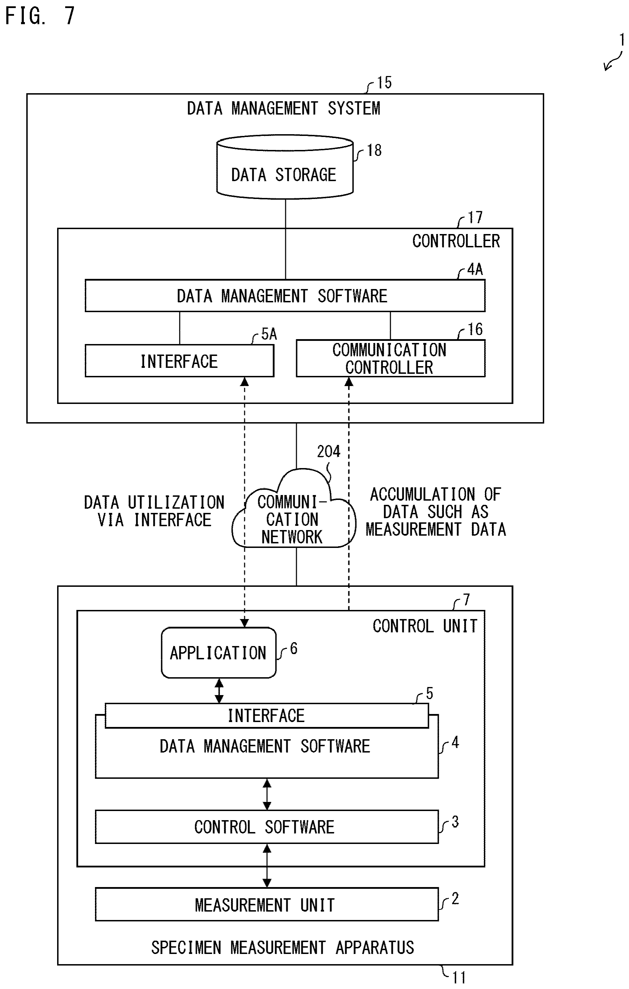

Information (for example, measurement data and the like) managed by the data management software 4 may be managed by an apparatus different from the specimen measurement apparatus 11. In the configuration example shown in FIG. 7, the specimen measurement system 1 includes: the specimen measurement apparatus 11; and the data management system 15 connected to the specimen measurement apparatus 11 via a communication network 204. The communication network 204 is, for example, an intra network or the Internet. Information (for example, measurement data and the like) managed by the data management software 4 may be managed by both the specimen measurement apparatus 11 and the data management system 15. For example, a duplicate of all or a part of the information managed by the data management software 4 of the specimen measurement apparatus 11 may be managed by the data management system 15.

The specimen measurement apparatus 11 includes: the measurement unit 2, the control software 3, the data management software 4, the interface 5, and the control unit 7 that executes the application 6. The specimen measurement apparatus 11 includes a function for providing information managed by the data management software 4 to the data management system 15. Although details will be described later, as an example of the function, the interface 5 may provide, in accordance with a predetermined event, information (measurement data and the like) corresponding to the event to the data management system 15 by a PUSH notification via the communication network 204. The PUSH notification may be executed by a component (for example, the data management software 4, another piece of software installed in the control unit 7) other than the interface 5. Instead of the PUSH notification, the specimen measurement apparatus 11 may provide, as a response to an inquiry from the data management system 15, information managed by the data management software 4 to the data management system 15. The process to provide the information to the data management system 15 is the same as Configuration Examples 6 and 7 described later.

The data management system 15 includes, for example, a controller 17 that executes data management software 4A, an interface 5A, and a communication controller 16. The controller 17 is equivalent to the computer 900 shown in FIG. 2. The data management system 15 includes a data storage 18 being a storage device. The data management software 4A manages the information provided by the PUSH notification from the specimen measurement apparatus 11. The information is stored in the data storage 18. The communication controller 16 includes a function of controlling communication regarding the PUSH notification from the specimen measurement apparatus 11.

The application 6 is capable of communicating with the data management software 4A via the interface 5A. The data management software 4A acquires, from the data storage 18, data corresponding to a request from the application 6 received by the interface 5A, and, as a response to the request, provides the data to the application 6. The interface 5A includes a function of allowing the application 6 executed in the specimen measurement apparatus 11 to utilize information managed in the data storage 18, via the data management software 4A. The interface 5A may be equivalent to the interface 5. For example, the interface 5A is a software interface, and is an API. The interface 5A allows the application 6 to utilize information managed by the data management software 4A. The application 6 communicates with the interface 5A according to the rule R, and the interface 5A allows the application 6 to utilize the function/information regarding the data management software 4A according to the rule R.

In the case of the present configuration example, the application 6 executed in the specimen measurement apparatus 11 accesses the interface 5A of the data management system 15 via the communication network 204, and requests utilization of information stored in the data storage 18 of the data management system 15. The data management system 15 may be placed in a laboratory or in a medical facility. In this case, the application 6 of the specimen measurement apparatus 11 accesses the interface 5A via the communication network 204 serving as an intranet, for example.

(Configuration Example 6)

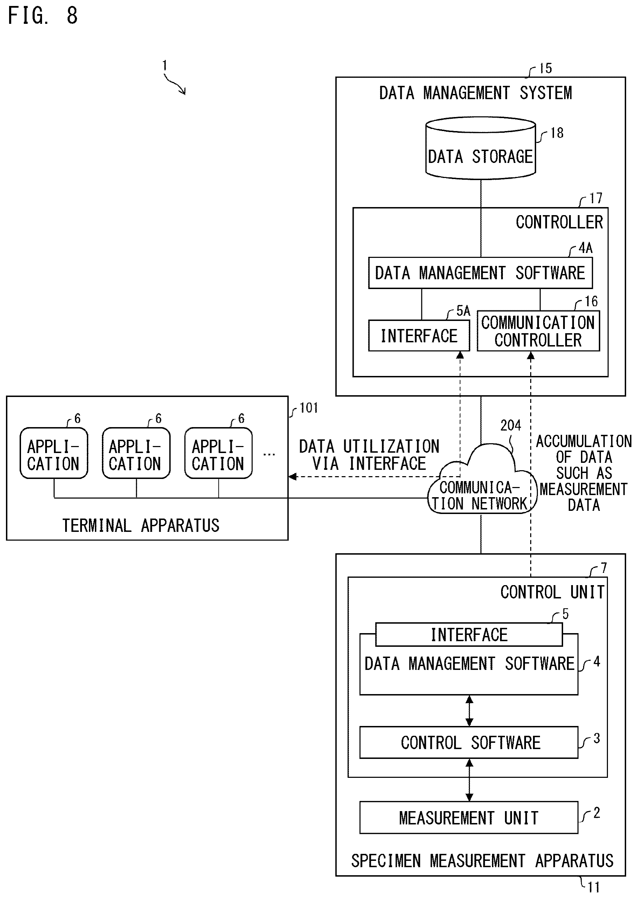

Information stored in the data storage 18 of the data management system 15 may be utilized by the application 6 executed in the terminal apparatus 101. In the configuration example shown in FIG. 8, the specimen measurement system 1 includes a configuration in which the specimen measurement apparatus 11, the data management system 15, and the terminal apparatus 101 are connected to each other via the communication network 204.

In the case of the present configuration example, the application 6 executed in the terminal apparatus 101 accesses the interface 5A of the data management system 15 via the communication network 204, and requests utilization of information stored in the data storage 18 of the data management system 15. The application 6 executed in the terminal apparatus 101 communicates with the interface 5A according to the rule R.

(Configuration Example 7)

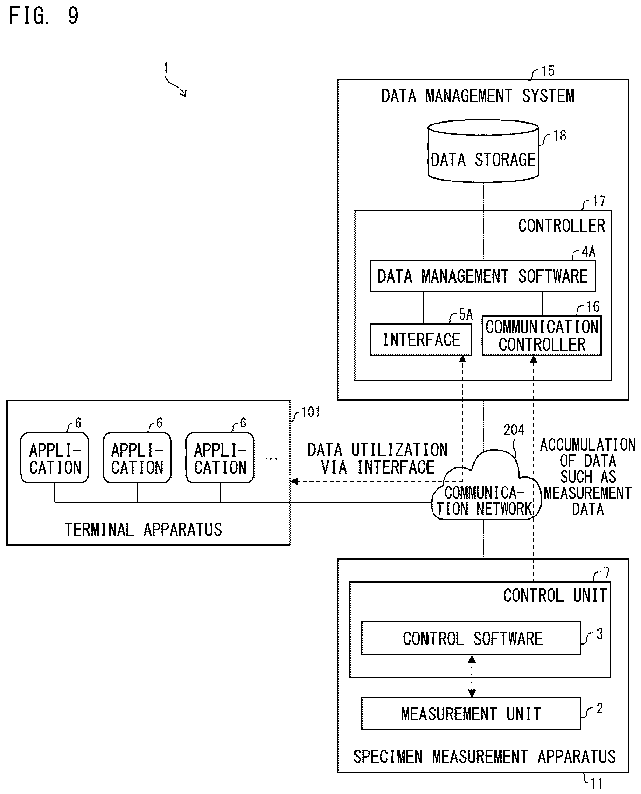

As a modification of the Configuration Example 6, the specimen measurement apparatus 11 may not include the data management software 4 and the interface 5. The configuration example shown in FIG. 9 is the same as the Configuration Example 6 except that the specimen measurement apparatus 11 may not include the data management software 4 and the interface 5.

In the case of the present configuration example, information such as measurement data acquired through control of the measurement unit 2 by the control software 3 is transmitted to the data management system 15 via the communication network 204, and is stored into the data storage 18 via the data management software 4A of the data management system 15. Similarly to the Configuration Example 6, the application 6 executed in the terminal apparatus 101 accesses the interface 5A of the data management system 15 via the communication network 204, and requests utilization of information stored in the data storage 18 of the data management system 15.

(Configuration Example of Sample Measuring Apparatus)

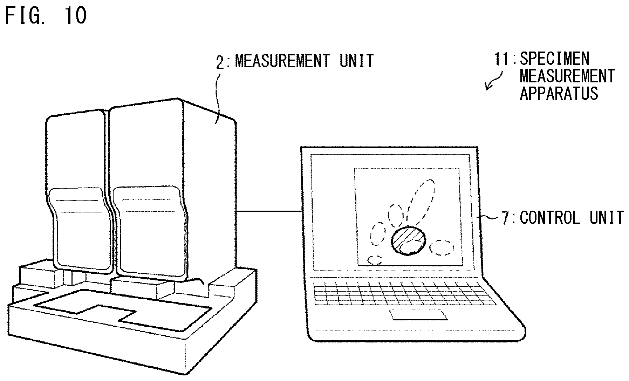

As described above, the specimen measurement apparatus 11 includes, as an example, the control unit 7 and the measurement unit 2 as shown in FIG. 10.

(Control Unit)

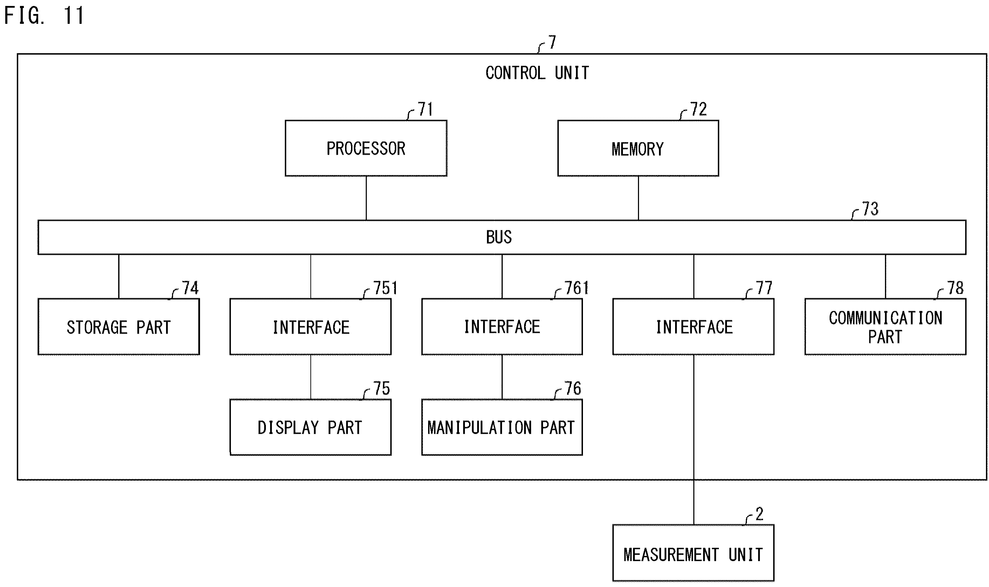

The control unit 7 is, for example, a computer. An example of a hardware configuration of the control unit 7 is shown in FIG. 11. The control unit 7 includes, as an example, the processor 71, the memory 72, the bus 73, the storage part 74, the display part 75, the interface 751, the manipulation part 76, the interface 761, an interface 77, and the communication part 78.

The processor 71 is a controller that comprehensively controls the functions of the control unit 7. The processor 71 is equivalent to the processor 71 shown in FIG. 2. The processor 71 reads out, for example, the control software 3 and the data management software 4 from the storage part 74 to be expanded into the memory 72, to execute the control software 3 and the data management software 4. The storage part 74 is equivalent to the storage part 74 shown in FIG. 2. The memory 72 is equivalent to the memory 72 shown in FIG. 2.

The interface 751 connects the bus 73 and the display part 75 such as a display to each other. The interface 761 connects the bus 73 and the manipulation part 76 such as a keyboard and a mouse to each other. The interface 77 connects the bus 73 and the measurement unit 2 to each other. The communication part 78 is responsible for communication with an external apparatus.

(Configuration Examples of Control Software and Data Management Software)

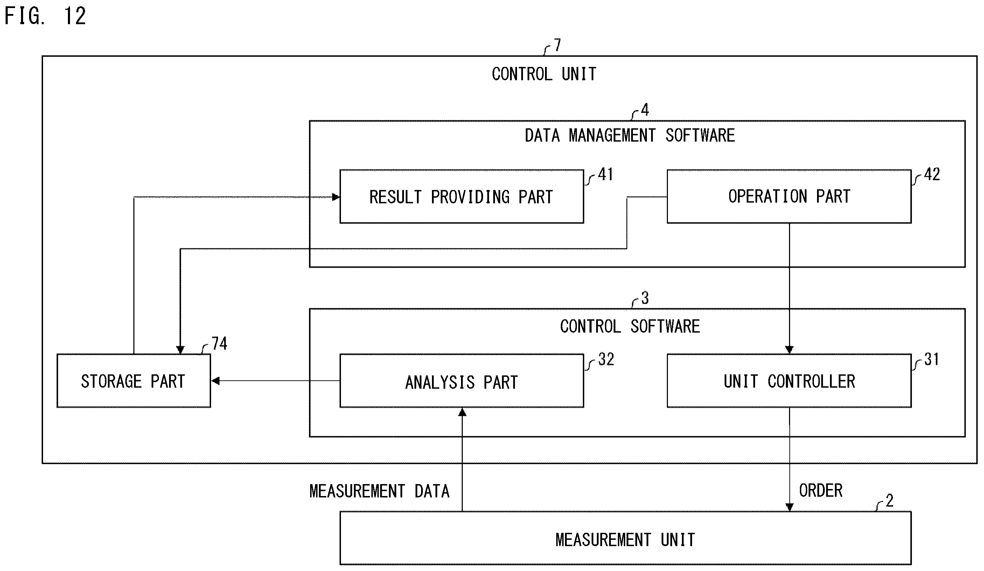

FIG. 12 shows configuration examples of the control software 3 and the data management software 4 executed in the control unit 7. The control software 3 includes, for example, a unit controller 31 and an analysis part 32. The unit controller 31 orders the measurement unit 2 to execute operation corresponding to a measurement order for a specimen serving as a measurement target. The analysis part 32 analyzes data acquired by the measurement unit 2 having operated in accordance with the order from the unit controller 31, generates measurement data as a measurement result, and stores the generated measurement data in the storage part 74.

The data management software 4 includes, for example, a result providing part 41 and an operation part 42. The result providing unit 41 includes a function (for example, GUI: Graphical User Interface) of acquiring, from the storage part 74, the measurement data generated by the analyzing unit 32, and providing the acquired measurement data to an operator. The operation part 42 includes functions such as a function (UI) of allowing registration of a measurement order/specimen information performed by the operator, a function of acquiring a measurement order and specimen information from a laboratory test information system (LIS: Laboratory Information System) and/or a hospital information system (HIS: Hospital Information System), a function of registering a measurement instruction based on the measurement order, various types of setting functions regarding the apparatus and the system, and an operation function such as a registration of reagent information and consumables information. The information that is set and registered by the operation part 42 is stored into the storage part 74. The operation function included in the data management software 4 is, for example, a function (for example, the necessary minimum of functions for measuring a specimen and providing a measurement result according to the intended use) required by the intended use (Intended Use) of the specimen measurement apparatus 11 approved as the medical device. The operation function included in the data management software 4 is, for example, a function required by the intended use (Intended Use) of the specimen measuring apparatus 11 approved as a medical device, and an additional function for enhancing convenience for a user. The operation function of the data management software 4 can be expanded, for example, by addition of the application 6.

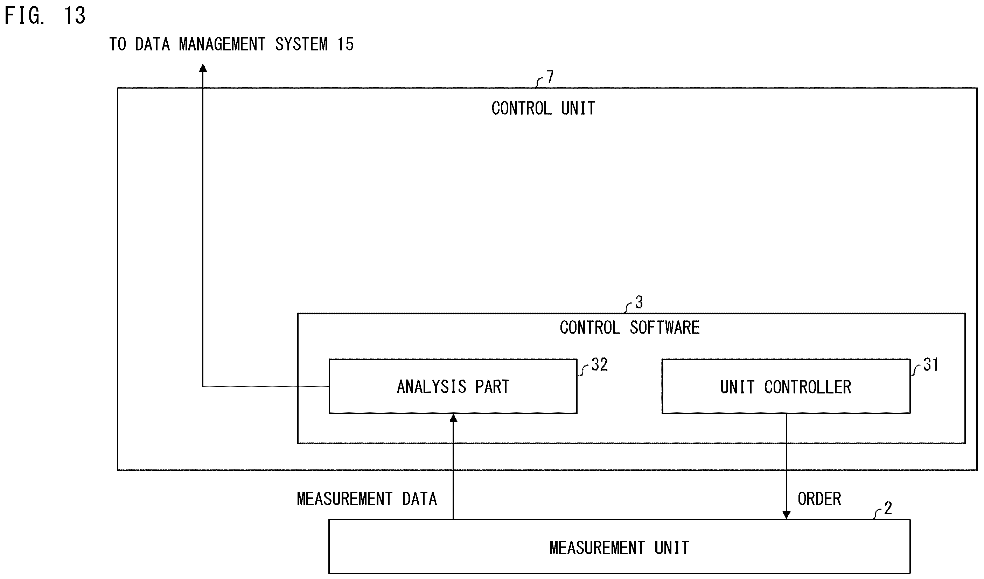

As a modification, as shown in FIG. 13, the control unit 7 need not necessary include the data management software 4. The control unit 7 shown as an example in FIG. 13 is used in a system shown as an example in FIG. 9, for example. In the case of this example, the unit controller 31 orders the measurement unit 2 to execute, for example, operation corresponding to a measurement order acquired from the LIS/HIS. The analysis part 32 analyzes data acquired by the measurement unit 2 having operated in accordance with the order from the unit controller 31, generates measurement data as a measurement result, and transmits the generated measurement data to the data management system 15 via the communication unit 78, for example.

(Measuring Unit)

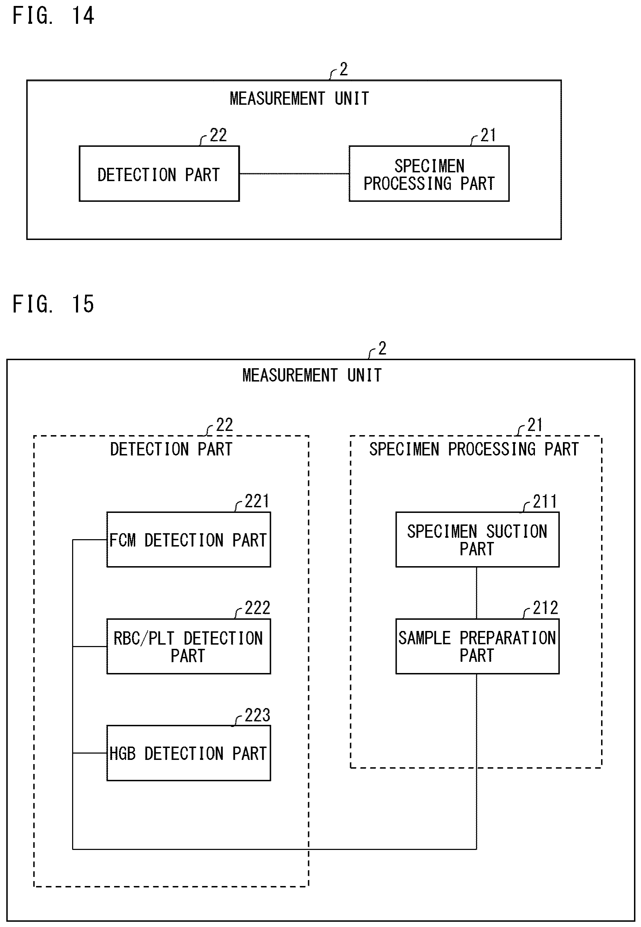

A configuration example of the measurement unit 2 is shown in FIG. 14. The measurement unit 2 includes, as an example, a specimen processing part 21 and a detection part 22. The specimen processing part 21 prepares a measurement sample on the basis of a specimen and a reagent. The detection part 22 measures the measurement sample prepared by the specimen processing part 21. The unit controller 31 of the control software 3 controls operation of the specimen processing part 21 and the detection part 22 linking with (according to an instruction from the operation part 42) the operation part 42 of the data management software 4. The unit controller 31 performs, for example, operation control of a mechanism and a fluid circuit forming the specimen processing part 21 and the detection part 22. The operation control may include, for example, at least one of: control of suction operation of a liquid including at least one of a specimen and a reagent; control of mixing the specimen and the reagent to prepare a measurement sample; control of causing the detection part 22 to detect the measurement sample; and control of causing the detection part 22 to detect at least one of optical information and an electric signal regarding the measurement sample, and analyzing a measurement result on the basis of a detection result having been detected.

(Blood Cell Analyzer)

FIG. 15 shows a configuration example when the measurement unit 2 is a blood cell analysis analyzer. In this case, the specimen processing part 21 includes, for example, a specimen suction part 211 and a specimen preparation part 212, and the detection part 22 includes, for example, an FCM detection part 221, an RBC/PLT detection part 222, and an HGB detection part 223.

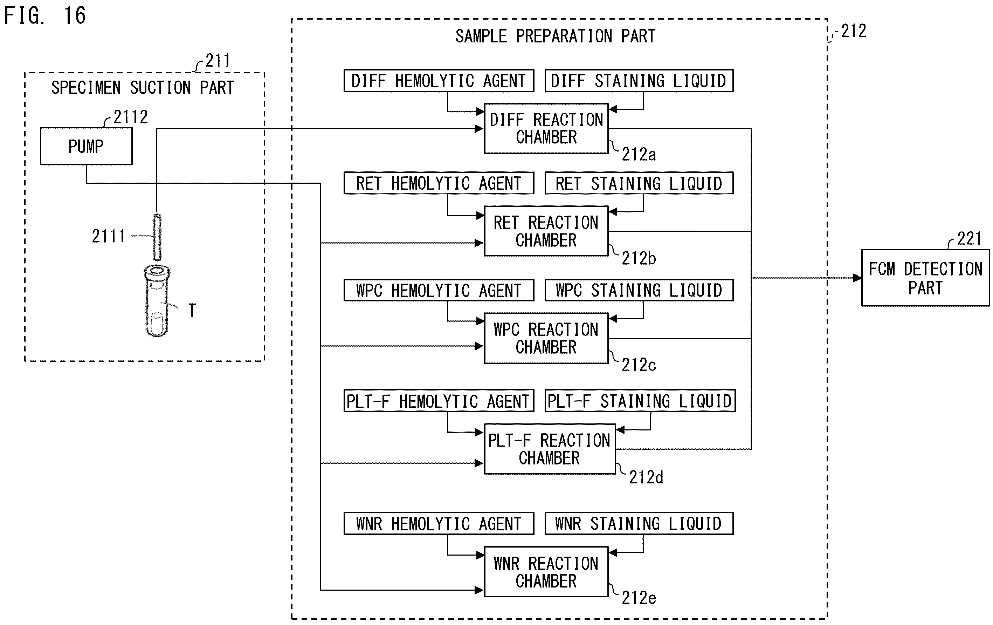

FIG. 16 is a schematic diagram for explaining the specimen suction part 211 and the sample preparation part 212 when a measurement sample is supplied to the FCM detection part 221. The specimen suction part 211 includes a nozzle 2111 for suctioning a blood specimen (whole blood) from a blood collection tube T, and a pump 2112 for applying a negative pressure/positive pressure to the nozzle 2111. The nozzle 2111 is moved upward and downward by an apparatus mechanism part (not shown), thereby being inserted into the blood collection tube T. When the pump 2112 applies a negative pressure in a state where the nozzle 2111 is inserted in the blood collection tube T, the blood specimen is suctioned via the nozzle 2111. The apparatus mechanism part may include a hand member configured to invert and agitate the blood collection tube T before suctioning blood from the blood collection tube T.

The sample preparation part 212 includes five reaction chambers 212a to 212e. The reaction chambers 212a to 212e are respectively used in DIFF, RET, WPC, PLT-F, and WNR measurement channels (also referred to as systems of measurement). Each reaction chamber has connected thereto, via flow paths, a hemolytic agent container containing a hemolytic agent being a reagent and a staining liquid container containing a staining liquid, which correspond to the corresponding measurement channel. One reaction chamber and a reagent (a hemolytic agent and a staining liquid) connected thereto from a measurement channel. For example, the DIFF measurement channel corresponds to a measurement item for classifying (five classification of white blood cells) white blood cells into a plurality of subpopulations, and is composed of a DIFF hemolytic agent and a DIFF staining liquid which are a DIFF measurement reagent, and a DIFF reaction chamber. The RET measurement channel corresponds to a measurement item regarding measurement of reticulocytes, the WPC measurement channel corresponds to a measurement item regarding measurement of abnormal white blood cells, the PLT-F measurement channel corresponds to a measurement item regarding optical measurement of platelets, and the WNR measurement channel corresponds to a measurement item regarding white blood cells and nucleated red blood cells. These measurement channels are also configured similarly to the DIFF measurement channels.

The nozzle 2111 having suctioned the blood specimen accesses, through horizontal/up-down movement by the apparatus mechanism part, a reaction chamber, among the reaction chambers 212a to 212e, that corresponds to the measurement channel corresponding to an order from above, and discharges the suctioned blood specimen. The sample preparation part 212 supplies a corresponding hemolytic agent and a corresponding staining liquid to the reaction chamber into which the blood specimen has been discharged, and mixes the blood specimen, the hemolytic agent, and the staining liquid in the reaction chamber, to prepare a measurement sample. The prepared measurement sample is supplied from the reaction chamber to the FCM detection part 221 via a flow path, and measurement of cells is performed by a flow cytometry method.

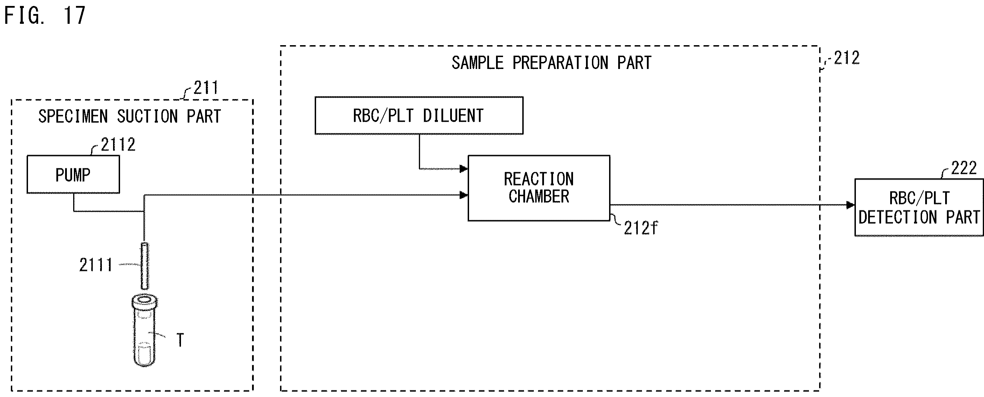

FIG. 17 is a schematic diagram for explaining the specimen suction part 211 and the sample preparation part 212 when a measurement sample is supplied to the RBC/PLT detection part 222. The specimen suction part 211 is the same as the specimen suction part 211 shown in FIG. 16. The sample preparation part 212 includes a reaction chamber 212f. The reaction chamber 212f is connected to a diluent container containing a diluent corresponding to RBC/PLT via a flow path. The measurement sample is prepared by mixing the blood specimen and the diluent in the reaction chamber 212f. The prepared measurement sample is supplied from the reaction chamber to the RBC/PLT detection part 222 via a path.

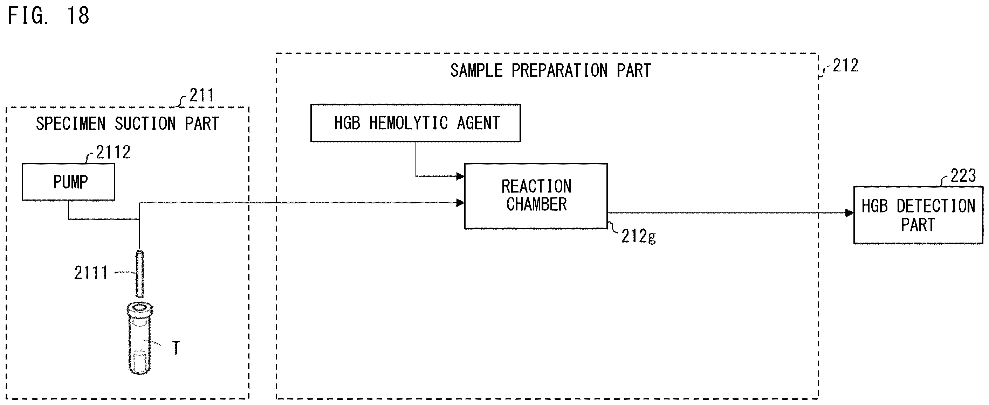

FIG. 18 is a schematic diagram for explaining the specimen suction part 211 and the sample preparation part 212 when a measurement sample is supplied to the HGB detection part 223. The specimen suction part 211 is the same as the specimen suction part 211 shown in FIG. 16. The sample preparation part 212 includes a reaction chamber 212g. The reaction chamber 212g is connected to a hemolytic agent container containing a hemolytic agent being a reagent corresponding to HGB via a flow path. The measurement sample is prepared by mixing the blood specimen and the hemolytic agent in the reaction chamber 212g. The prepared measurement sample is supplied from the reaction chamber to the HGB detection part 223 via a flow path.

(Urine particle apparatus)

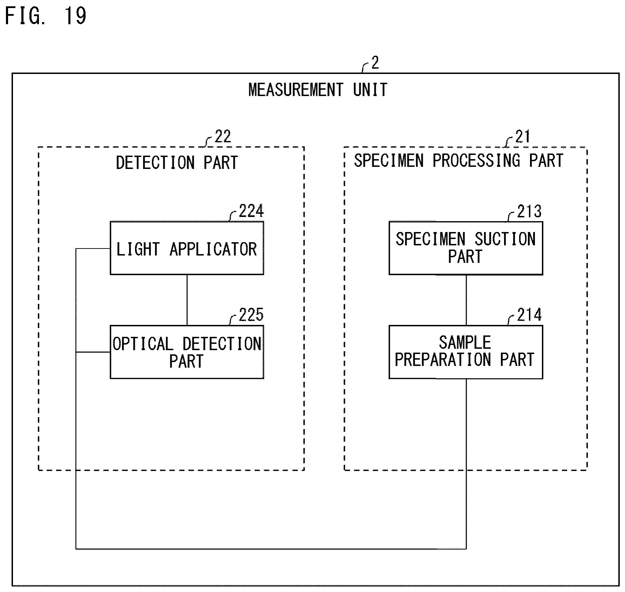

FIG. 19 shows a configuration example when the measurement unit 2 is a urine particle apparatus. In this case, the specimen processing part 21 includes, for example, a specimen suction part 213 and a specimen preparation part 214, and the detection part 22 includes, for example, a light applicator 224 and an optical detection part 225.

The sample preparation part 214 suctions a urine specimen from a specimen container by the sample suction part 213. The specimen suction part 213 is composed of a pipe-shaped suction tube. The sample preparation part 214 prepares a measurement sample by mixing a reagent with the urine specimen suctioned by the specimen suction part 213. The reagent to be mixed with the urine specimen is a staining liquid containing a dye that stains particles in the urine specimen, and/or a diluent.

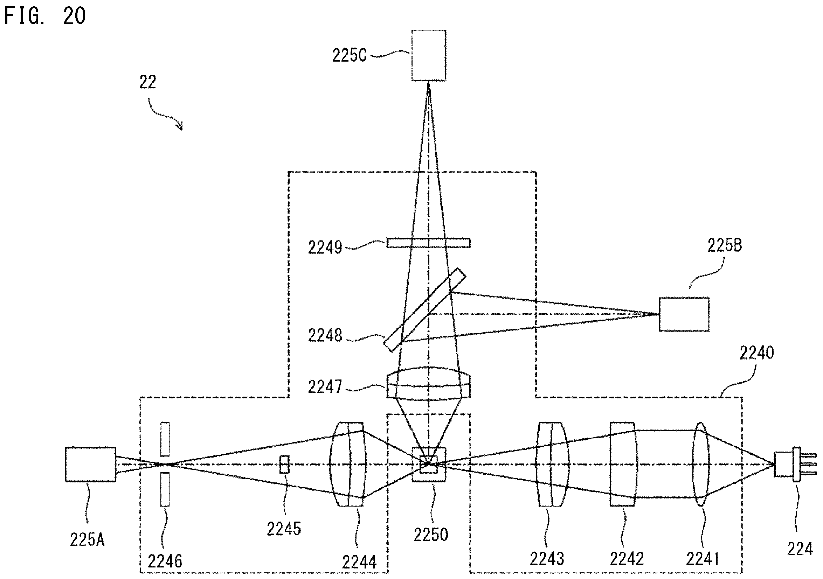

The detection part 22 measures the measurement sample prepared by the sample preparation part 214. FIG. 20 shows a configuration example of the detection part 22. The detection part 22 includes a flow cell 2250, the light applicator 224, an optical system 2240, and the optical detection part 225 (225A, 225B, and 225C). In the flow cell 2250, a measurement sample in a state of being surrounded by a sheath liquid is caused to flow in one direction. The light applicator 224 is composed of, for example, a laser diode, and emits the light having a predetermined wavelength. The optical system 2240 applies the light emitted from the light applicator 224, to a sample flow in the flow cell 2250. The optical system 2240 guides forward scattered light generated from each particle in the flow cell 2250 to the optical detection part 225A. The optical system 2240 guides side scattered light and side fluorescence generated from each particle to the respective optical detection parts 225B, 225C.

The optical system 2240 includes a collimator lens 2241, a cylindrical lens 2242, a condenser lens 2243, a condenser lens 2244, a beam stopper 2245, a pinhole 2246, a condenser lens 2247, a dichroic mirror 2248, and an optical filter 2249.

The collimator lens 2241 converts light emitted from the light applicator 224 into parallel light. The cylindrical lens 2242 and the condenser lens 2243 shape light transmitted through the collimator lens 2241 into a shape having a large width in a direction perpendicular to the flow of the measurement sample, and apply the light to the sample flow in the flow cell 2250. Accordingly, forward scattered light is generated to the forward direction of the particle flowing in the flow cell 2250, and side scattered light and fluorescence are generated to the side direction of the particle flowing in the flow cell 2250.

The condenser lens 2244 condenses the forward scattered light to the position of the pinhole 2246. The beam stopper 2245 blocks light transmitted through the flow cell 2250 without being applied to the particle in the measurement sample. The optical detection part 225A receives the forward scattered light having passed through the pinhole 2246. The optical detection part 225A includes, for example, a photodiode. The optical detection part 225A amplifies a detection signal by an amplifier, to generates a forward scattered light signal based on the forward scattered light, and outputs the generated forward scattered light signal.

The condenser lens 2247 converges each of the side scattered light and the fluorescence. The dichroic mirror 2248 reflects the side scattered light transmitted through the condenser lens 2247. The optical detection part 225B receives the side scattered light reflected by the dichroic mirror 2248. The optical detection part 225B includes, for example, a photodiode or a photomultiplier tube. The optical detection pert 225B amplifies a detection signal by an amplifier, to generate a side scattered light signal based on the side scattered light, and outputs the generated side scattered light signal.

The dichroic mirror 2248 allows transmission therethrough of the fluorescence transmitted through the condenser lens 2247. The optical filter 2249 removes light in a wavelength band that may become noise, from the fluorescence transmitted through the dichroic mirror 2248. The optical detection part 225C receives the fluorescence transmitted through the optical filter 2249. The optical detection part 225C includes, for example, a photomultiplier. The optical detection part 225C amplifies a detection signal by an amplifier, to generate a fluorescence signal based on the fluorescence, and outputs the generated fluorescence signal.

The optical detection part 225 (225A, 225B, 225C) can switch the light receiving sensitivity between a low sensitivity and a high sensitivity, by switching the driving voltage at the time of photoelectric conversion, or by an amplifier. While a measurement sample flows in the flow cell 2250, the optical detection parts 225 (225A, 225B, 225C) each generate and output a signal of corresponding light when the light receiving sensitivity is a low sensitivity, and a signal of corresponding light when the light receiving sensitivity is a high corresponding.

(Blood Coagulation Analyzer)

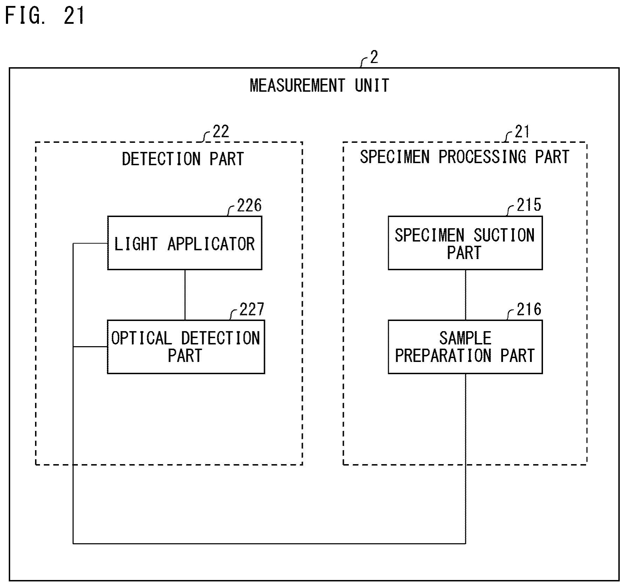

FIG. 21 shows a configuration example when the measurement unit 2 is a blood coagulation analyzer. In this case, the specimen processing part 21 includes, for example, a specimen suction part 215 and a specimen preparation part 216, and the detection part 22 includes, for example, a light applicator 226 and an optical detection part 227.

The specimen suction part 215 suctions a specimen from a specimen container. The sample preparation part 216 prepares a measurement sample by mixing a reagent with a specimen suctioned by the specimen suction part 215.

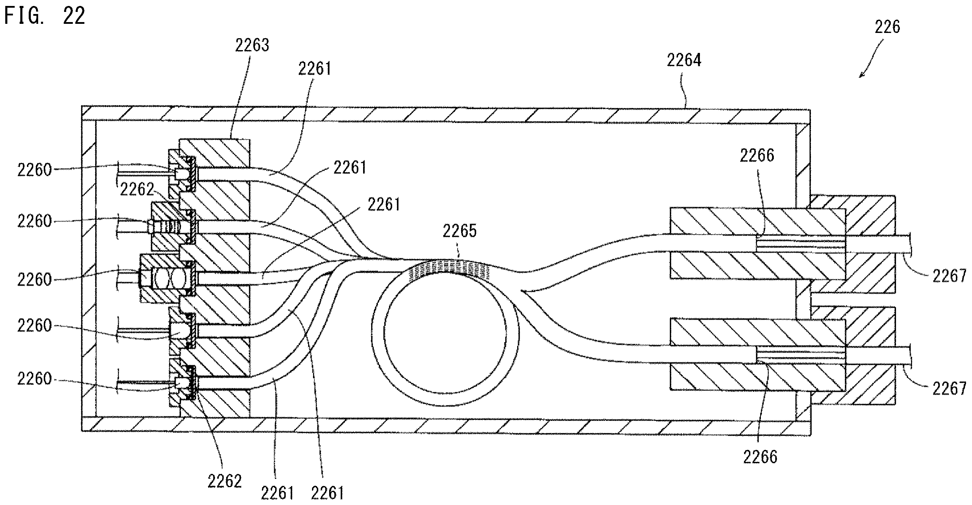

The detection part 22 measures the measurement sample prepared by the sample preparation part 216. FIG. 22 shows a configuration example of the light applicator 226 of the detection part 22. The light applicator 226 includes five light sources 2260, five optical fiber parts 2261 provided so as to correspond to the five light sources 2260, and one holding member 2263 for holding the respective light sources 2260 and incident ends 2262 of the respective optical fiber parts 2261. The light sources 2260, the optical fiber parts 2261, and the holding member 2263 are housed in a metal-made housing 2264, for example.

The plurality of optical fiber portions 2261 each include a plurality of optical fibers 2265. The plurality of optical fiber parts 2261 are, at emission ends 2266, bundled such that the plurality of optical fiber parts 2261 corresponding to the respective light sources 2260 are mixed so as to be approximately uniformly distributed. The five optical fiber portions 2261 are intertwisted at a portion to be integrated, so as to have two emission ends 2266. The two emission ends 2266 are provided so as to correspond to the optical detection part 227. The light applicator 226 includes light distribution members 2267 configured to distribute light from the emission end 2266 in the form of bundles.

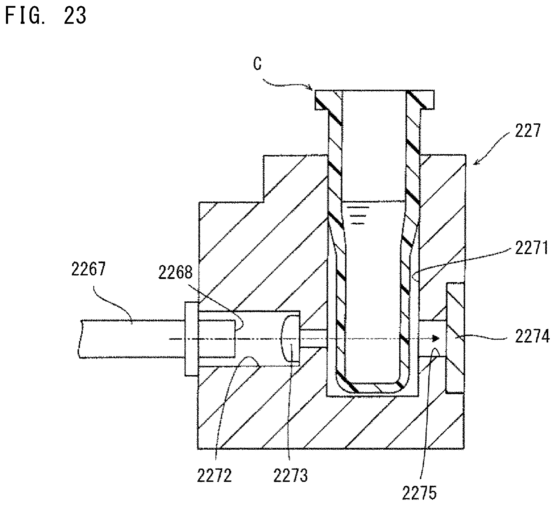

FIG. 23 shows a configuration example of the optical detection part 227 of the detection part 22. The optical detection part 227 includes a container placement part 2271 as a hole part extending in the up-down direction, and in a hole 2272 extending from the container placement part 2271 sideways, an emission end 2268 of the light distribution member 2267 is disposed. A condenser lens 2273 is disposed inside the hole 2272. A light receiver 2274 is provided at an end portion of the hole 2275 formed so as to face the hole 2272 across the container placement part 2271. Accordingly, the emission end 2268 of the light distribution member 2267, the condenser lens 2273, the container placement part 2271, and the light receiver 2274 are disposed so as to be linearly arranged. Light outputted from the emission end 2268 is transmitted, via the condenser lens 2273, through a container C containing a measurement sample in the container placement part 2271 and the measurement sample in the container C, to be detect by the light receiver 2274.

(Immunoassay Analyzer)

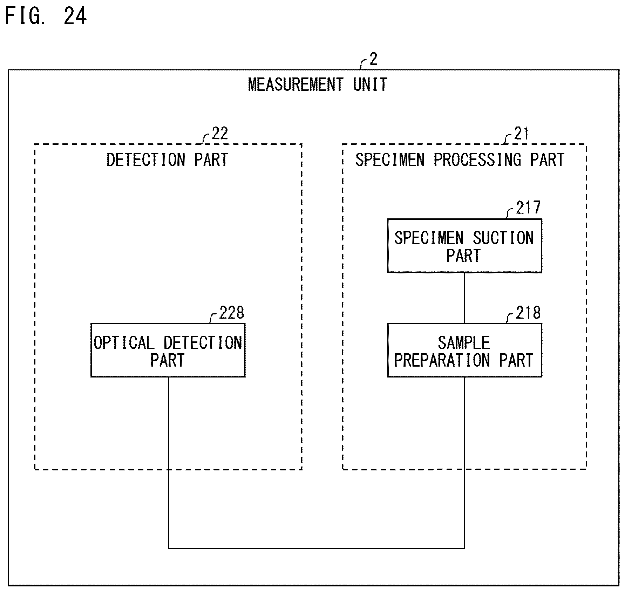

FIG. 24 shows a configuration example when the measurement unit 2 is an immunoassay apparatus. In this case, the specimen processing part 21 includes, for example, a specimen suction part 217 and a specimen preparation part 218, and the detection part 22 includes, for example, an optical detection part 228.

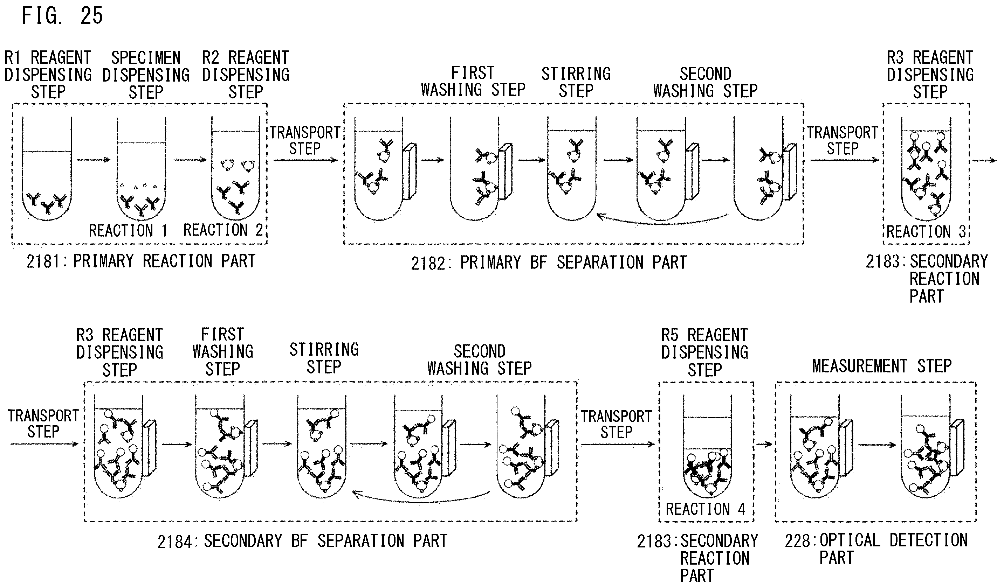

The specimen suction part 217 suctions a specimen from a specimen container. The sample preparation part 218 includes, for example, a primary reaction part 2181, a primary BF (Bound Free) separation part 2182, a secondary reaction part 2183, and a secondary BF separation part 2184. An example of the flow of the process performed by the sample preparation part 218 will be described with reference to FIG. 25. In the primary reaction part 2181, magnetic particles (R2 reagent) are caused to be bound to a capture antibody (R1 reagent) bound to an antigen contained in a specimen suctioned by the specimen suction part 217, and then, the antigen, the capture antibody, and the magnetic particles bound to each other are attracted by a magnet of the primary BF separation part 2182, to remove the R1 reagent containing the capture antibody that has not reacted (free). Then, in the secondary reaction part 2183, the antigen having the magnetic particles bound thereto and a labeled antibody (R3 reagent) are caused to be bound to each other, and then, the magnetic particles, the antigen, and the labeled antibody bound to each other are attracted by a magnet of the secondary BF separation part 2184, to remove the R3 reagent containing unreacted (free) labeled antibody. Further, in the secondary reaction part 2183, a dispersion liquid (R4 reagent) is added, and then, a luminescent substrate (R5 reagent) which emits light in the course of reaction with the labeled antibody is added, and then, in the optical detection part 228, the amount of light (the amount proportional to the number of photons) generated in the course of reaction process between the labeled antibody of the R3 reagent and the luminescent substrate of the R5 reagent is obtained by a photomultiplier tube (Photo Multiplier Tube).

(Configuration Example of Interface)

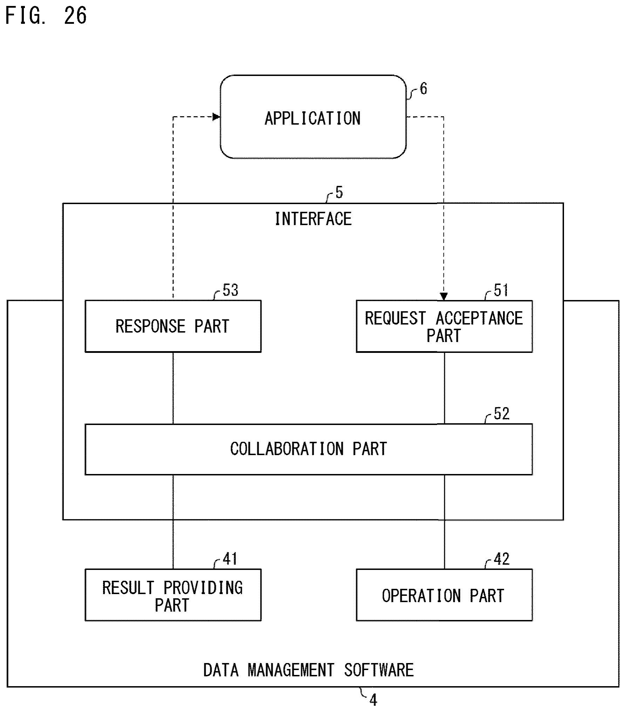

The interface 5 receives a request from the application 6, communicates with the data management software 4 (the result providing part 41 and the operation part 42), and performs a response corresponding to the request to the application 6. FIG. 26 shows a configuration example of the interface 5. The interface 5 includes, as an example, a request acceptance part 51, a collaboration part 52, and a response part 53. The request acceptance part 51 accepts a request from the application 6. The request acceptance part 51 analyzes the content of the request according to the rule R, and instructs the collaboration part 52 to communicate with the data management software 4 in order to acquire a response to the request. The collaboration part 52 communicates with the data management software 4 in accordance with the instruction, and acquires a response corresponding to the request. The response part 53 provides the response acquired by the collaboration unit 52, to the application 6.

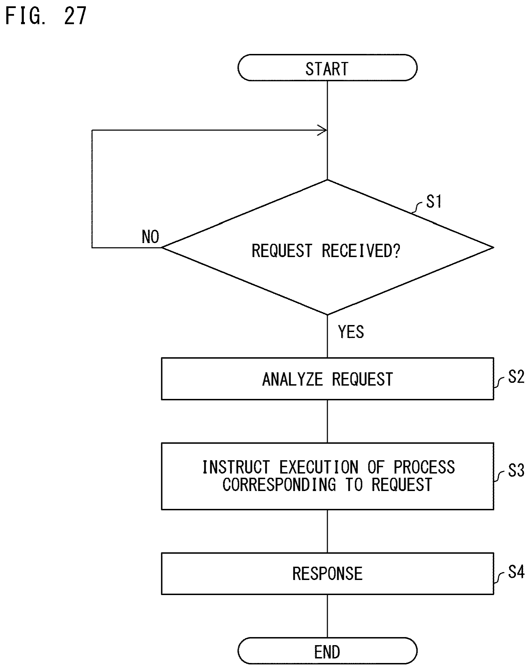

FIG. 27 is a flowchart showing an example of the flow (method of communication between the application and the interface) of the process performed by the interface 5 of the specimen measurement apparatus 11. First, the interface 5 waits for a request from the application 6 (S1). In response to receiving the request from the application 6 (Yes in S1), the interface 5 analyzes the content of the request according to the rule R (S2). The interface 5 instructs the data management software 4 to perform a process corresponding to the analyzed request (S3). The data management software 4 executes the process according to the instruction. For example, if the request instructs to acquire information regarding a measurement result, the result providing part 41 of the data management software 4 identifies the requested information and provides the identified information to the application 6 via the interface 5. The measurement result, for example, may be measurement data measured by the measurement unit 2 of a specific specimen measurement apparatus 11, a measurement results of a quality control substance in a time period (for example, the time period may be per day, per week, per month) designated by the request. The operation part 42 of the data management software 4 registers, for example, a measurement order in accordance with the request for registration of the measurement order from the application 6, and provides a response indicating that the registration has been completed, to the application 6 via the interface 5. In response to executing the process by the data management software 4, the interface 5 returns a response to the application 6 (S4). Thus, the interface 5 allows the application 6 to utilize the data and/or function corresponding to the request.

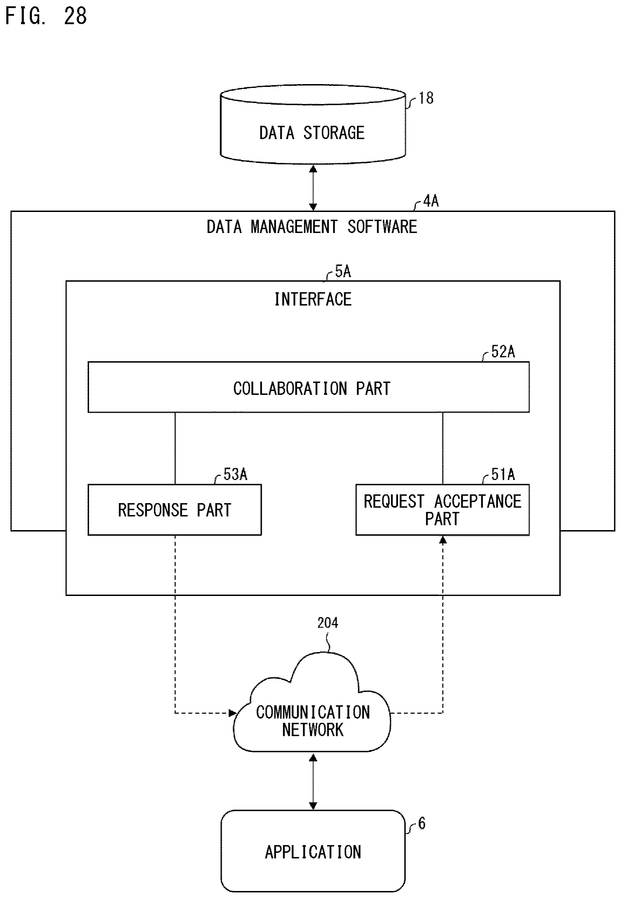

FIG. 28 shows a configuration example of the interface 5A of the data management system 15. The interface 5A includes, as an example, a request acceptance part 51A, a collaboration part 52A, and a response part 53A. The request acceptance part 51A accepts a request from the application 6 via the communication network 204. The request acceptance part 51A analyzes the content of the request, and instructs, on the basis of the analyzed request, the collaboration part 52A to link with the data management software 4A in order to acquire a response to the request. The collaboration part 52A communicates with the data management software 4A in accordance with the instruction and acquires a response corresponding to the request. The response part 53A provides the response acquired by the collaboration part 52A, to the application 6 via the communication network 204.

When the interface 5, 5A is configured as a Web API, the application 6 accesses the interface 5, 5A by using a URI defined according to the rule R. An example of the syntax of the URI defined according to the rule R is shown in Formula 1.

$$\text{http://[server address]/[interface ID]/[apparatus ID]\{/[resource]/[query parameter]\}} \cdots \text{(Formula 1)}$$

The "Server Address" in Formula 1 is a communication address corresponding to the interface 5, 5A. The server address is designated by, for example, an IP address and a port number.

For example, a plurality of types of the interfaces 5, 5A may be provided. For example, the type of the interface 5/5A may correspond to the type of the specimen measurement apparatus 11. In this case, the "interface ID" in Formula 1 is designated in order to identify the interface 5, 5A corresponding to a certain type of the specimen measurement apparatus 11. The interface ID is, for example, an ID corresponding to the type of the specimen measurement apparatus 11. The designation of the "interface ID" in Formula 1 may not mandatory, and if the "interface ID" is not designated, for example, a wildcard (blank, *, etc.) may be used. The interface ID may correspond to, for example, a type of the rule R. For example, there are a plurality of types of the rules R according to the types of the specimen measurement apparatuses 11. In other words, the interface ID corresponds to, for example, the type of the specimen measurement apparatus 11. For example, a format of information management of the data management software 4 and the data management system 15 may differ depending on the type of the specimen measurement apparatus 11. In such a case, for example, by collaborating the interface 5/5A and the application 6 with each other according to a plurality of types of the rules R each corresponding to the formats of information management (e.g., each format corresponds to the type of the specimen measurement apparatus 11), the application 6 can utilize a plurality of types of information for which the formats of information management are respectively different.

The "apparatus ID" in Formula 1 is the ID of the specimen measurement apparatus 11 whose information and function are to be used by the application 6. The apparatus ID is, for example, the serial number of the specimen measurement apparatus 11. Designation of "apparatus ID" in Formula 1 may not be mandatory. For example, if the "apparatus ID" is not designated, a wildcard (blank, *, etc.) is used.

The "resource" in Formula 1 is the resource of the API endpoint. The resource is, for example, the directory on a database corresponding to the information to be used by the application 6.

The "query parameter" in Formula 1 is a query, when a plurality of data and control subjects are present on the resource designated by the "resource" parameter, for uniquely identifying a certain data and a certain subject. For example, the "query parameter" is used for identifying a certain measurement data among a plurality of measurement data. To identify the certain measurement data, at least one condition (for example, specific time period, specific specimen, measurement data in which a measurement abnormality has occurred) may be designated as the query parameter.

Designation of the "resource" in Formula 1 may not be mandatory, and if the "resource" is not designated, for example, a wildcard (blank, *, etc.) may be used. The designation formats of the "resource" and the "query parameter" may differ depending on, for example, the type of the specimen measurement apparatus 11. For example, with respect to acquisition of measurement data, the designation format of the "resource" and/or the "query parameter" for acquisition of the measurement data of the blood cell analysis apparatus, and the designation format of the "resource" and/or the "query parameter" for acquisition of the measurement data of an immunoassay apparatus may be different from each other. With respect to the measurement data, in accordance with the type of the specimen measurement apparatus 11, for example, the resource as the API endpoint may be different, or a specific query parameter may be required for each type of the specimen measurement apparatus 11. In such a case, the designation formats of the "resource" and the "query parameter" may be different depending on, for example, the type of the specimen measurement apparatus 11. The designation formats of the "resource" and the "query parameter" are a part of the rule R. Therefore, in other words, the "resource" and/or the "query parameter" may be designated on the basis of a plurality of types of the rules R according to the type of the specimen measurement apparatus 11.

Manipulation with respect to the resource designated by the URI is defined by, for example, an HTTP method. Examples of the HTTP method include methods such as "GET", "PUT", "DELETE", and "POST". Information designated by the "resource" and the "query parameter" of the URI is acquired by using the "GET" method.

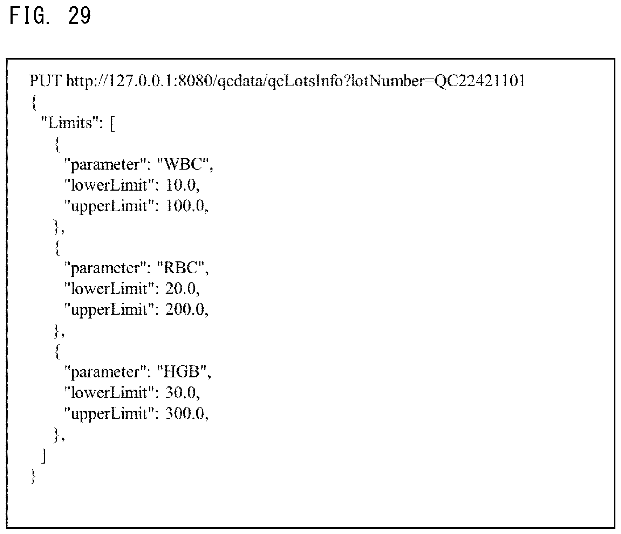

By use of the "PUT" method, information designated by the "resource" and the "query parameter" of the URI is updated or edited. In this case, new data (for example, the content of the resource to be updated or edited) is caused to be included in the HTTP request body. FIG. 29 shows an example of the data structure of the HTTP request body when lot information on quality control is updated. This example is described in the JSON (JavaScript Object Notation) format (JavaScript is a registered trademark). For example, the URI is designated as "http://127.0.0.1:8080/qcdata/qcLotsInfo?lotNumber=QC22421101". The "server address" of the control unit 7 to serve as the target of the "PUT" method is designated as "127.0.0.1". The "resource" for the QC (quality control) data held by the control unit 7 is designated as the "qcdata". A QC lot information is designated by the "query parameter". In this example, the "query parameter" is designated as "QC22421101" which represents a QC lot number. In this example, the URI is modified by the "PUT" method in order to update the QC date corresponding to the lot number "QC22421101". The contents (i.e., the QC data) to be updated is designated by HTTP request body. In this example, the HTTP request body is defined as "{"Limits":[{ "parameter": "WBC", "lowerLimit":10.0, "upperLimit": 100.0}, {"parameter": "RBC", "lowerLimit":20.0, "upperLimit":200.0},{"parameter": "HGB", "lowerLimit":30.0, "upperLimit":300.0},]}",. With such a combination of the URI and the HTTP request body, update is performed with respect to the designated QC lot information, such that: as for WBC (white blood cell count), lower limit value is updated as 10.0, upper limit value is updated as 100.0; as for RBC (red blood cell count), lower limit value is updated as 20.0, upper limit value is updated as 200.0; and as for HGB (hemoglobin concentration), lower limit value is updated as 30.0, upper limit value is updated as 300.0. Here, since the lower limit value and the upper limit value of each of WBC (white blood cell count), RBC (red blood cell count), and HGB (hemoglobin concentration) are updated, QC can be performed by using the updated lower limit value and upper limit value. The lower limit value and the upper limit value as above are also referred to as management values. The management values are set for guaranteeing that the measurement result is accurate and for proving the validity of the measurement method and the management method. In addition, when the measurement result is outside the lower limit value or the upper limit value, the lower limit value or the upper limit value is also used for indicating that the measurement result is abnormal or that the measurement failed to be correctly performed.

By use of the "DELETE" method, information designated by the "resource" and the "query parameter" of the URI is deleted.

When new information is to be registered or created, the "POST" method is used. When the "POST" method is used, the "resource" and the "query parameter" may not be designated. In this case, new data (the content of the resource to be registered or created) is caused to be included in the HTTP request body.

The request acceptance part 51 of the interface 5 accepts, for example, the URI and the HTTP method from the application 6. The collaboration part 52 of the interface 5 communicates with the data management software 4 according to the URI and the HTTP method accepted by the request acceptance part 51, for example. For example, the collaboration part 52 communicates with the result providing part 41 in accordance with an acquisition request ("GET" of the HTTP method) for the measurement result in the specimen measurement apparatus 11 designated by the "apparatus ID" of the URI, and acquires corresponding measurement result. The response part 53 of the interface 5 transmits a response according to the received URI and HTTP method, to the application 6.

Similarly, the request acceptance part 51A of the interface 5A accepts, for example, the URI and the HTTP method from the application 6. The collaboration unit 52A of the interface 5A communicates with the data management software 4A according to the URI and the HTTP method accepted by the request acceptance unit 51A, for example. For example, the collaboration part 52A communicates with the data management software 4A in accordance with an acquisition request ("GET" of the HTTP method) for the measurement result in the specimen measurement apparatus 11 designated by the "apparatus ID" of the URI, and acquires corresponding measurement result. The response part 53A of the interface 5A transmits a response according to the received URI and HTTP method, to the application 6.

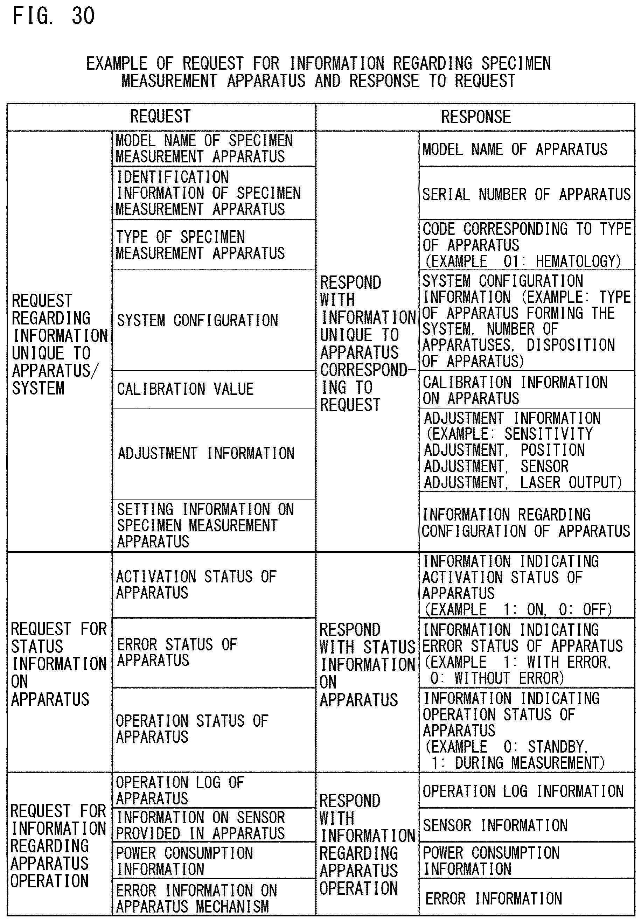

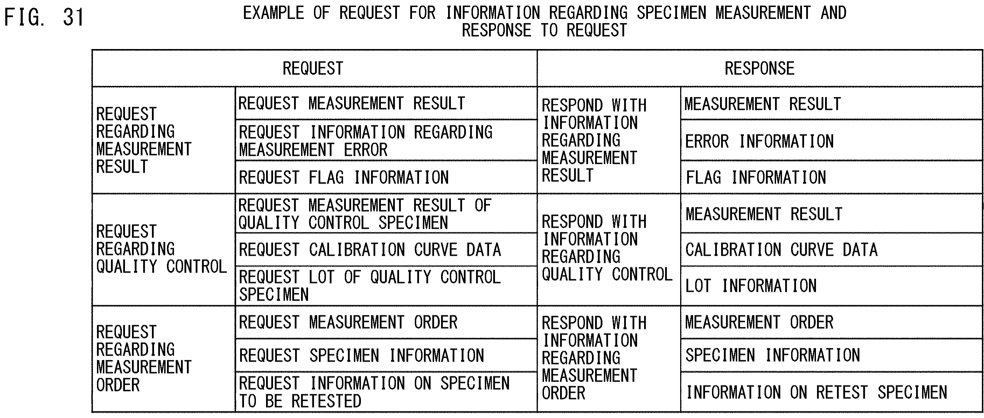

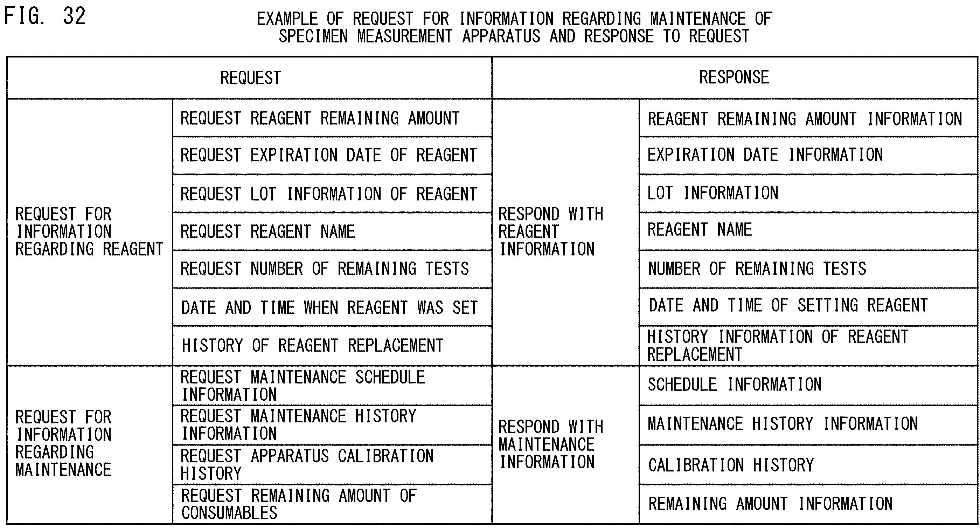

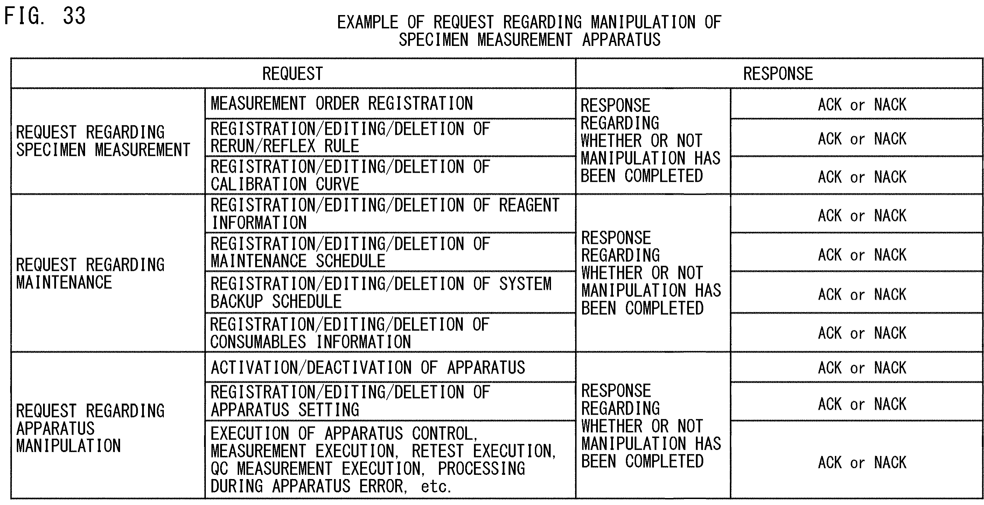

Examples of requests and responses to the interface 5, 5A are shown in FIGS. 30 to 33. FIG. 30 shows examples of the request for information regarding the specimen measurement apparatus 11 / the response to the request. For example, FIG. 30 shows examples of: (1) the request for information unique to the specimen measurement apparatus 11 and the system including the specimen measurement apparatus 11 and a response thereto; (2) the request for information regarding the status of the specimen measurement apparatus 11 and the response thereto; and (3) the request for information regarding operation of the specimen measurement apparatus 11 and the response thereto. Examples of various types of information corresponding to the request and the response are shown in the table of FIG. 30. However, the various types of information corresponding to the request and the response are not limited to the examples shown in the table. The interface 5 can collectively provide, for example, information regarding a plurality of the specimen measurement apparatuses 11 (for example, serial numbers of a plurality of the specimen measurement apparatuses 11 placed in a laboratory, error statuses of the plurality of the specimen measurement apparatuses 11 placed in the laboratory, and the like), to the application 6. FIG. 31 shows examples of the request for information regarding specimen measurement / the response to the request. For example, FIG. 31 shows examples of: (1) the request for information regarding the measurement result and the response thereto; (2) the request regarding a quality control measurement and the response thereto; and (3) the request for information regarding the measurement order and the response thereto. Examples of various types of information corresponding to the request and the response are shown in the table of FIG. 31. However, the various types of information corresponding to the request and the response are not limited to the examples shown in the table. The interface 5 can collectively provide, for example, information regarding a plurality of the specimen measurement apparatuses 11 (for example, results of measurement of specimens performed in a predetermined period by a plurality of the specimen measurement apparatuses 11 placed in a laboratory, results of measurement of quality control specimens performed in a predetermined period by the plurality of the specimen measurement apparatuses 11 placed in the laboratory, and the like), to the application 6. FIG. 32 shows examples of the request for information regarding maintenance of the specimen measurement apparatus 11 / the response to the request. For example, FIG. 32 shows examples of: (1) the request for information regarding a reagent and the response thereto; and (2) the request for information regarding maintenance and the response thereto. Examples of various types of information corresponding to the request and the response are shown in the table of FIG. 32. However, the various types of information corresponding to the request and the response are not limited to the examples shown in the table. The consumables described in FIG. 32 are, for example, cuvettes to be used in measurement, washing liquids for washing flow paths in the apparatus, pipette tips, and the like. The interface 5 can collectively provide, for example, information regarding a plurality of the specimen measurement apparatuses 11 (for example, reagent remaining amount information for a plurality of the specimen measurement apparatuses 11 placed in a laboratory, maintenance schedule information for the plurality of the specimen measurement apparatuses 11 placed in the laboratory, and the like), to the application 6. FIG. 33 shows examples of the request regarding manipulation of the specimen measurement apparatus 11. For example, FIG. 33 shows examples of: (1) the request for manipulation regarding the specimen measurement and the response thereto; (2) the request for manipulation regarding maintenance and the response thereto; and (3) the request regarding manipulation of the specimen measurement apparatus 11 and the response thereto. Examples of various types of information corresponding to the request and the response are shown in the table of FIG. 33. However, the various types of information corresponding to the request and the response are not limited to the examples shown in the table. The interface 5 can cause, for example, the application 6 to collectively execute manipulation with respect to a plurality of the specimen measurement apparatuses 11 (for example, registration of a Rerun/Reflex rule with respect to a plurality of the specimen measurement apparatuses 11 placed in a laboratory, an activation/deactivation request for the plurality of the specimen measurement apparatuses 11 placed in the laboratory, and the like). In the example of FIG. 33, as the response to a manipulation request, for example, information (for example, ACK corresponding to completion of manipulation and NACK corresponding to incompletion manipulation (for example, error)) indicating whether or not manipulation has been completed is shown.

(PUSH notification)

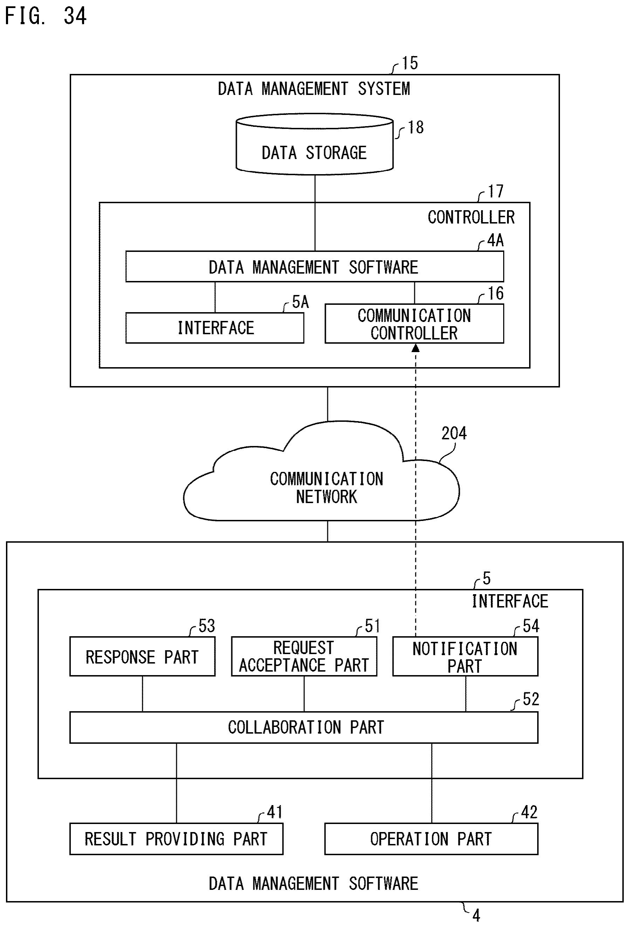

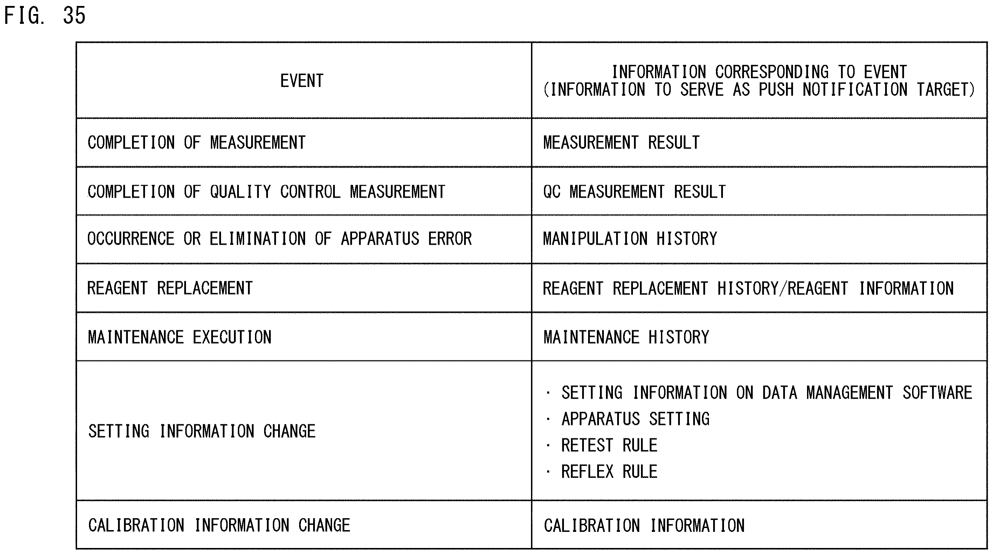

FIG. 34 shows a configuration example of the PUSH notification from the data management software 4 of the specimen measurement apparatus 11 to the data management system 15. The interface 5 may include a notification part 54 including a function of performing the PUSH notification. For example, at the time of occurrence of a predetermined event, the notification part 54 provides information corresponding to the event, to the data management system 15 by using the PUSH notification via the communication network 204. FIG. 35 shows examples of events and examples of information (information to serve as the subject of the PUSH notification) corresponding to the event. For example, upon acquisition of the measurement result of the specimen by the specimen measurement apparatus 11, the notification part 54 provides the measurement result to the data management system 15. For example, upon execution of predetermined manipulation (for example, reagent replacement, maintenance, information setting, and the like) on the specimen measurement apparatus 11, the notification part 54 provides information related to the manipulation, to the data management system 15. For example, in accordance with change (occurrence of an error, elimination of the error, and the like) in the status of the specimen measurement apparatus 11, the notification part 54 provides information (for example, error information, manipulation history regarding the error, and the like) related to the change in the status, to the data management system 15.

The notification part 54 may be provided to the data management software 4 instead of the interface 5. Alternatively, the notification part 54 may be provided to the control unit 7 as software independent of the interface 5 and the data management software 4.

(Application)

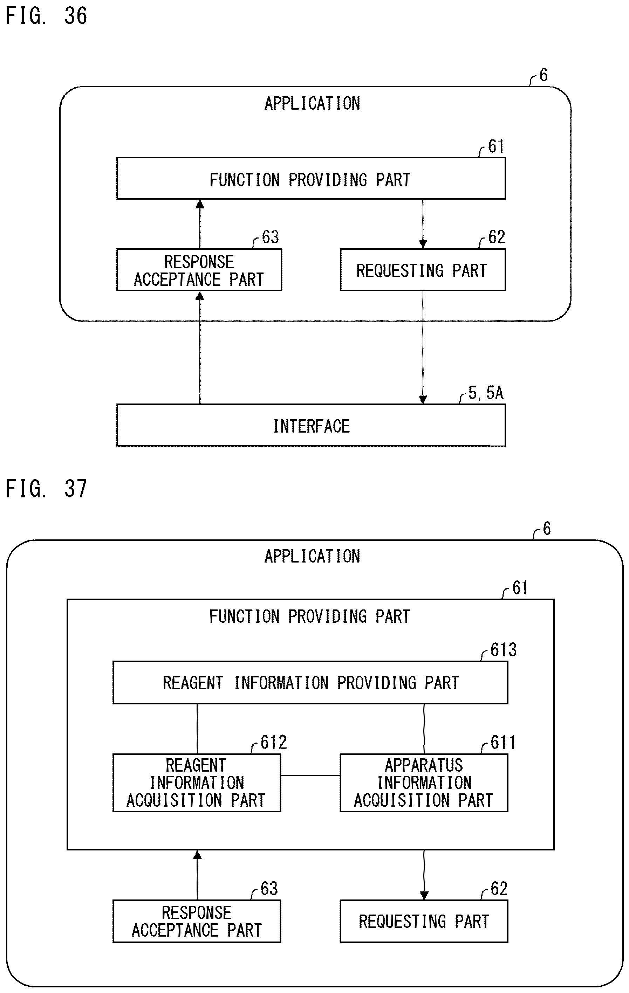

FIG. 36 shows a configuration example of the application 6. The application 6 includes, for example, a function providing part 61, a requesting part 62, and a response acceptance part 63.

The function providing part 61 provides a function for expanding the specimen measurement system 1. The function providing part 61 provides a function according to the type of the application 6. For example, according to the type of the application 6, the function providing part 61 provides a function of providing a QC result (QC chart and the like), a function of managing a reagent placed in the specimen measurement apparatus 11, a function regarding managing of an error having occurred in the specimen measurement apparatus 11, a function regarding maintenance of the specimen measurement apparatus 11, a function regarding operation management of the specimen measurement apparatus 11, and the like.

The function providing part 61 may have a function of allowing the user to log-in by using an account of a service provider of the function provided by the application 6. In this case, only when log-in has been established using the account of the service provider, the application 6 provides the function by the function providing part 61.

For example, when information regarding the specimen measurement apparatus 11 is necessary, the function providing part 61 requests the requesting part 62 to acquire the information. The requesting part 62 requests information to the interface 5, 5A in accordance with the request from the function providing part 61. For example, as described above, the requesting part 62 requests the information by a command C defined by a combination of the URI and the HTTP method. In accordance with operation of the function providing part 61, for example, when control of the apparatus 11 (for example, registration/change of configuration of the specimen measurement apparatus 11) become necessary, the function providing part 61 makes the request (for example, the request for registration/change of the configuration) regarding the control of the apparatus 11 to the requesting part 62. The requesting part 62 makes the request regarding the control of the apparatus 11 to the interface 5, 5A. For example, as described above, the requesting part 62 makes the request regarding the control of the apparatus 11 by the command C defined by the combination of the URI and the HTTP method. For example, the requesting part 62 generates the command C including the URI on the basis of information provided by the function providing part 61, and transmits the request to the interface 5/5A. For example, when requesting information regarding the specimen measurement apparatus 11, the function providing part 61 notifies the requesting part 62 of an instruction (for example, result of measurement in a predetermined period performed by a certain specimen measurement apparatus 11 with the apparatus ID) regarding the information is required. The requesting part 62 generates the command C according to the notified instruction. The instruction from the function providing part 61 to the requesting part 62 is notified, for example, according to manipulation of the application 6 by the user. For example, when the user has requested the measurement result acquired on the specific day by a certain specimen measurement apparatus 11, the function providing part 61 notifies the requesting part 62 of the instruction corresponding to the request. When generating the command C, the requesting part 62 generates the command C with reference to, for example, configuration information (for example, IP address of the request destination server) regarding creation of the URI. As the configuration information, the IP address itself of the request destination server may be set, or the URL (Uniform Resource Locator) of the server may be set. The IP address of the request destination server is, for example, the IP address of the control unit 7 of the specimen measurement apparatus 11 set in the laboratory, or the IP address of the data management system 15. When the address of the request destination server is set by the URL, the requesting part 62 inquires the DNS (Domain Name System) about the IP address on the basis of the domain name included in the URL, for example. The requesting part 62 generates the URI on the basis of the IP address notified from the DNS. The IP address or the URL may be input by the user via a GUI of the application 6. The process of generation of the URI or the command C by the requesting part 62 is the same in the examples of the application described later.

The response acceptance part 63 accepts, from the interface 5, 5A, the response corresponding to the request by the requesting part 62, and passes the response content to the function providing part 61.

(Example of application)

(Example 1)

FIG. 37 shows a configuration example when the application 6 is a reagent management application. The reagent management application provides, for example, a function for the user to manage information (for example, lot information of a reagent, the day when the reagent was placed in the specimen measurement apparatus 11, the reagent remaining amount) regarding the reagent placed in the specimen measurement apparatus 11. The function providing part 61 of the application 6 of the present configuration example includes, for example, an apparatus information acquisition part 611, a reagent information acquisition part 612, and a reagent information providing part 613.

The acquisition information acquisition unit 611 asks the requesting part 62 to acquire information regarding the specimen measurement apparatus 11 to serve as the target of reagent management. For example, in order to acquire reagent information on the management target, the apparatus information acquisition part 611 requests the requesting part 62 to acquire information regarding the specimen measurement apparatus 11 in which reagents are placed. For example, when the application 6 is used in a laboratory, the apparatus information acquisition part 611 asks the requesting part 62 to acquire information (for example, the apparatus ID of the specimen measurement apparatus 11, the name of the specimen measurement apparatus 11, and the like) regarding the specimen measurement apparatus 11 placed in the laboratory. For example, the apparatus information acquisition part 611 may ask the requesting part 62 to acquire information (for example, information regarding the kind of the specimen measurement apparatus 11, the apparatus ID of the specimen measurement apparatus 11, the name of the specimen measurement apparatus 11, and the like) regarding a predetermined kind of the specimen measurement apparatus 11 among a plurality of kinds of the specimen measurement apparatuses 11 placed in the laboratory. For example, on the basis of the content of such asking from the apparatus information acquiring part 611, the requesting part 62 generates a URI, and requests information acquisition to interface 5, 5A, by a command C generated by combining the generated URI and "GET" of the HTTP method. The URI that is generated by the requesting part 62 is, for example, "http://127.0.0.1:8080/XR//XXXX". In this URI example, the "server address" is designated as "127.0.0.1:8080", "interface ID" is designated as "XR", "apparatus ID" is not designated, "resource" is designated as "XXXX", and "query parameter" is not designated. "XXXX" is the API endpoint corresponding to information (for example, information regarding the kind of the specimen measurement apparatus 11, the apparatus ID of the specimen measurement apparatus 11, the name of the specimen measurement apparatus 11, and the like) regarding the specimen measurement apparatus 11. "127.0.0.1:8080", which is "server address", is the address corresponding to the interface 5A of the data management system 15 that manages information regarding a plurality of the specimen measurement apparatuses 11, for example. When "server address" is present in a plural number, a plurality of URIs (a plurality of addresses respectively corresponding to the URIs) may be generated. "XR", which is "interface ID", is set according to the kind of the specimen measurement apparatus 11 to serve as the target of information acquisition, for example. The URI may be generated in a plural number in according to the kind of information that is requested. The response acceptance part 63 accepts a response corresponding to the request from the requesting part 62, and passes the accepted information to the apparatus information acquisition part 611.

The reagent information acquisition part 612 asks the requesting part 62 to acquire information regarding reagents on the basis of information on the specimen measurement apparatus 11 acquired by the apparatus information acquisition part 611. For example, the reagent information acquisition part 612 asks the requesting part 62 to acquire reagent information on each of the specimen measurement apparatuses 11. For example, the requesting part 62 generates a URI in accordance with such asking, and requests information acquisition to the interface 5, 5A by a command C generated by combining the generated URI and "GET" of the HTTP method. The URI that is generated by the requesting part 62 is, for example, 7'http://127.0.0.1:8080/XR/XR-20^AB123456^11000/YYYY". In this URI, "server address" is designated as "127.0.0.1:8080", "interface ID" is designated as "XR", "apparatus ID" is designated as "XR-20^AB123456^11000", "resource" is designated as "YYYY", and "query parameter' is not designated. "YYYY" is the API endpoint corresponding to the reagent information. "XR-20^AB123456^11000", which is "apparatus ID", is set on the basis of the information (apparatus ID of the specimen measurement apparatus 11) acquired by the apparatus information acquisition unit 611, for example.

The requesting part 62 may generate a plurality of URIs respectively corresponding to a plurality of the specimen measurement apparatuses 11. In this case, the requesting part 62 generates a URI having a different "apparatus ID" for each specimen measurement apparatus 11. For example, the requesting part 62 can also collectively request, by one URL, reagent information of a plurality of the specimen measurement apparatuses 11. In "apparatus ID" of the URI in this case, for example, a plurality of apparatus IDs are enumerated by being separated with a predetermined delimiter (for example, [ ], |, and the like). The response acceptance part 63 accepts a response corresponding to the request from the requesting part 62, and passes the accepted information to the reagent information acquiring part 612.

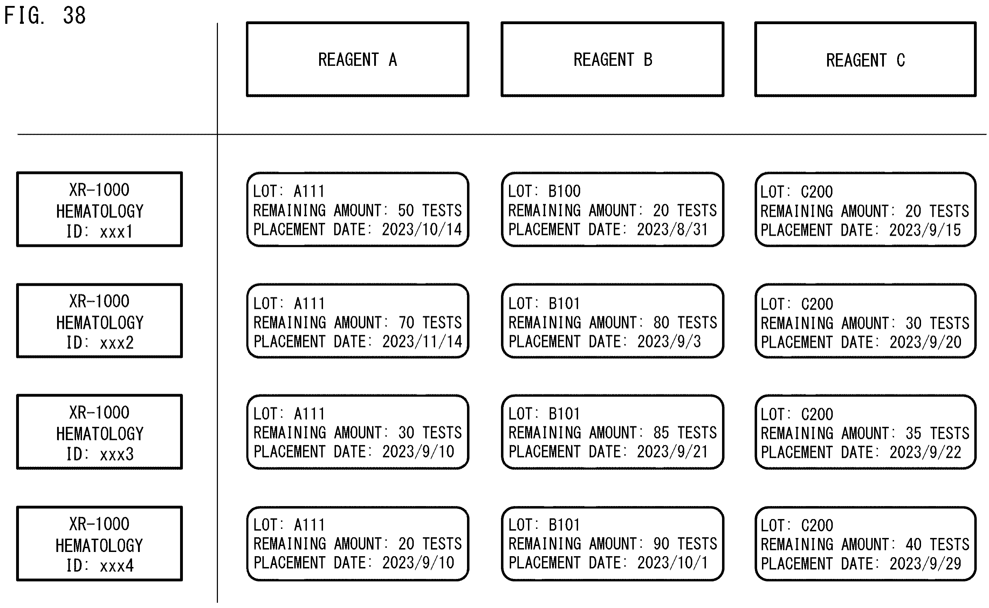

The reagent information providing part 613 provides the information regarding reagent management on the basis of the information acquired by the reagent information acquisition part 612. The reagent information providing part 613 provides the information regarding reagent management through a GUI, for example. FIG. 38 is an example of providing reagent information through a GUI. In this example, with respect to each of four apparatuses (ID: xxx1 to xxx4), information (lot, remaining amount, placement date) regarding three kinds of reagents (A to C) is shown.

(Example 2)

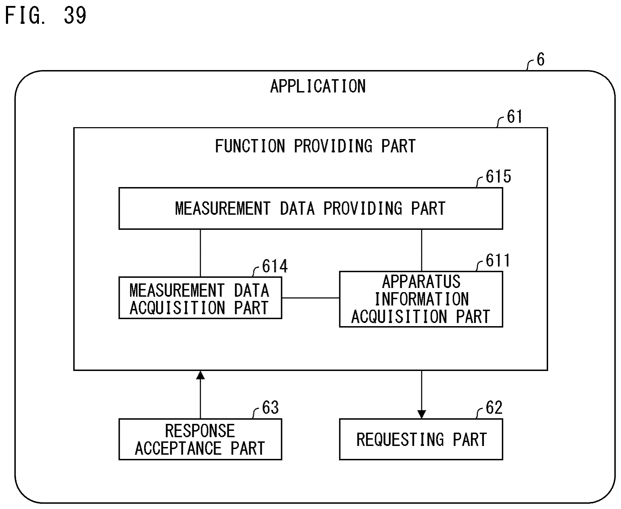

FIG. 39 shows a configuration example when the application 6 is a specimen tracking application. The specimen tracking application has a function of providing the status of a test target specimen to the user. The function providing part 61 of the application 6 of the present configuration example includes, for example, the apparatus information acquisition part 611, a measurement data acquisition part 614, and a measurement data providing part 615.

The apparatus information acquisition part 611 asks the requesting part 62 to acquire information regarding the specimen measurement apparatus 11 to serve as the target of specimen tracking. For example, similar to Example 1, the apparatus information acquisition part 611 requests the requesting part 62 to acquire information regarding the specimen measurement apparatus 11 that measures the specimen, in order to acquire information on a test target specimen. For example, when the application 6 is used in a laboratory, the apparatus information acquisition part 611 asks the requesting part 62 to acquire information (for example, the apparatus ID of the specimen measurement apparatus 11, the name of the specimen measurement apparatus 11, and the like) regarding the specimen measurement apparatus 11 placed in the laboratory. For example, the apparatus information acquisition part 611 may ask the requesting part 62 to acquire information (for example, information regarding the kind of the specimen measurement apparatus 11, the apparatus ID of the specimen measurement apparatus 11, the name of the specimen measurement apparatus 11, and the like) regarding a predetermined kind of the specimen measurement apparatus 11 among a plurality of kinds of the specimen measurement apparatuses 11 placed in the laboratory. The requesting part 62 requests the interface 5/5A to acquire information regarding the specimen measurement apparatus 11, by the same method as in Example 1. The response acceptance part 63 accepts a response corresponding to the request from the requesting part 62, and passes the accepted information to the apparatus information acquisition part 611.

The measurement data acquisition part 614 asks the request part 62 to acquire measurement data on the basis of the information of the specimen measurement apparatus 11 acquired by the apparatus information acquisition part 611. For example, the measurement data acquisition part 614 asks the request part 62 to acquire measurement data of each of the specimen measurement apparatuses 11. For example, the requesting part 62 generates a URI on the basis of such asking, and requests information acquisition to the interface 5, 5A, by a command C generated by combining the generated URI and "GET" of the HTTP method. The URI that is generated by the requesting part 62 is, for example, "http://127.0.0.1:8080/XR/XR-20^AB123456^11000/MMMM". In this URI, "server address" is designated as "127.0.0.1:8080", "interface ID" is designated as "XR", "apparatus ID" is designated as "XR-20^AB123456^11000", "resource" is designated as "MMMM", and "query parameter" is not designated. "MMMM" is the API endpoint corresponding to the measurement data. "XR-20^AB123456^11000", which is "apparatus ID" is set on the basis of the information (apparatus ID of the specimen measurement apparatus 11) acquired by the apparatus information acquisition part 611, for example. The measurement data acquired via the interface 5/5A includes, for example, information such as measurement date and time, and specimen number corresponding to the measurement data.

The requesting part 62 may generate a plurality of URIs respectively corresponding to a plurality of the specimen measurement apparatuses 11. In this case, the requesting part 62 generates a URI having a different "apparatus ID" for each specimen measurement apparatus 11. For example, the requesting part 62 can also collectively request, by one URI, measurement data of a plurality of the specimen measurement apparatuses 11. In "apparatus ID" of the URI in this case, for example, a plurality of apparatus IDs are enumerated by being separated with a predetermined delimiter (for example, [] , |, and the like). The response acceptance part 63 accepts a response corresponding to the request from the requesting unit 62, and passes the accepted information to the measurement data acquisition part 614.

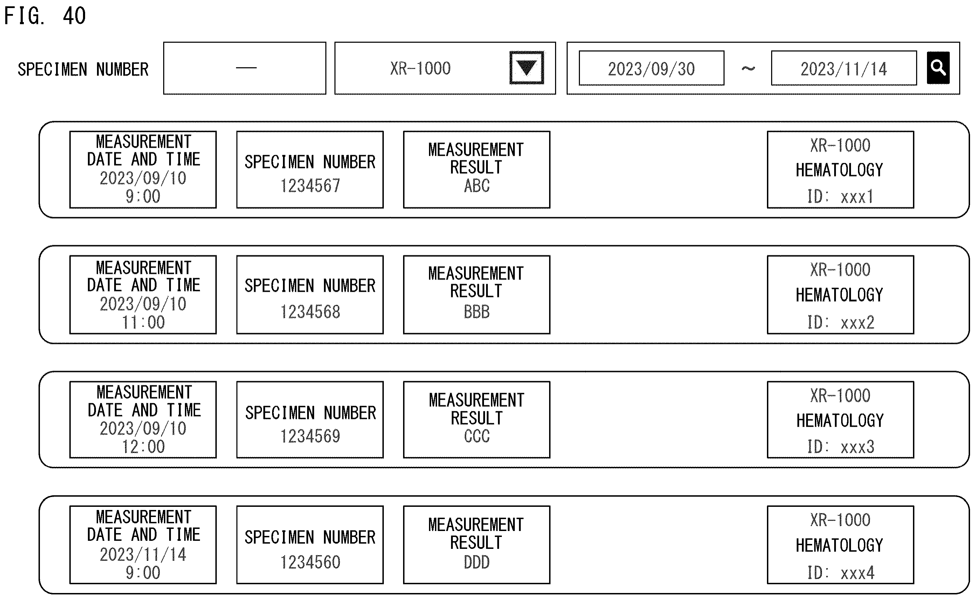

The measurement data providing part 615 provides the measurement data on the basis of the information acquired by the measurement data acquisition part 614. The measurement data providing part 615 provides the measurement data through a GUI, for example. FIG. 40 is an example of providing measurement data through a GUI. In this example, measurement date and time, specimen number, measurement result, and the kind of apparatus are shown. In this GUI, search for measurement data can be performed using a specimen number, the kind of apparatus, and a measurement period as a search key.

(Example 3)

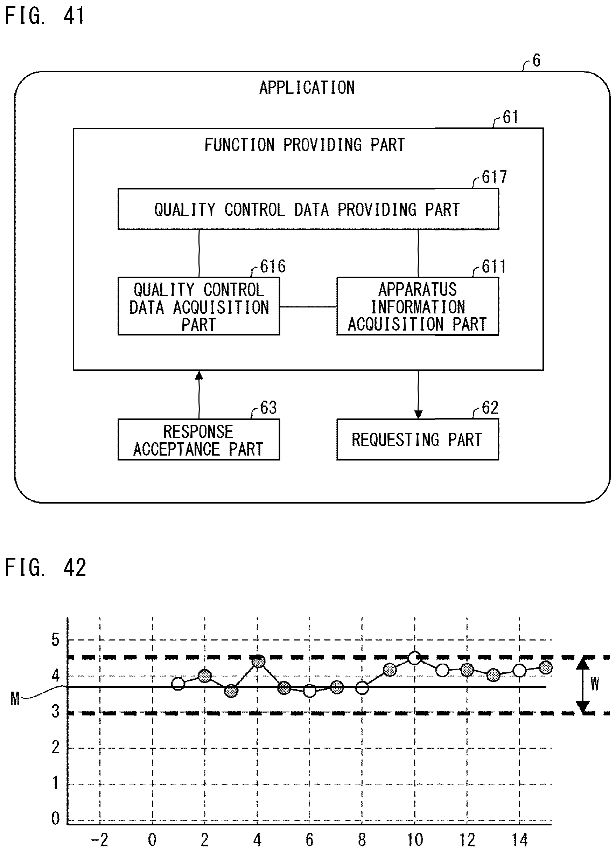

FIG. 41 shows a configuration example when the application 6 is a quality control application. The quality control application has a function of providing information regarding quality control of the specimen measurement apparatus 11 to the user. The function providing part 61 of the application 6 of the present configuration example includes, for example, the apparatus information acquisition part 611, a quality control data acquisition part 616, and a quality control data providing part 617.

The apparatus information acquisition part 611 asks the requesting part 62 to acquire information regarding the specimen measurement apparatus 11. For example, similar to Example 1, the apparatus information acquisition part 611 requests the request part 62 to acquire information regarding the specimen measurement apparatus 11, in order to acquire information regarding quality control. For example, when the application 6 is used in a laboratory, the apparatus information acquisition part 611 asks the requesting part 62 to acquire information (for example, the apparatus ID of the specimen measurement apparatus 11, the name of the specimen measurement apparatus 11, and the like) regarding the specimen measurement apparatus 11 placed in the laboratory. For example, the apparatus information acquisition part 611 may ask the requesting part 62 to acquire information (for example, information regarding the kind of the specimen measurement apparatus 11, the apparatus ID of the specimen measurement apparatus 11, the name of the specimen measurement apparatus 11, and the like) regarding a predetermined kind of the specimen measurement apparatus 11 among a plurality of kinds of the specimen measurement apparatuses 11 placed in the laboratory. The requesting part 62 requests the interface 5/5A to acquire information regarding the specimen measurement apparatus 11, by the same method as in Example 1. The response acceptance part 63 accepts a response corresponding to the request from the requesting part 62, and passes the accepted information to the apparatus information acquisition part 611.

The quality control data acquisition part 616 asks the requesting part 62 to acquire quality control data on the basis of the information of the specimen measurement apparatus 11 acquired by the apparatus information acquisition part 611. For example, the quality control data acquisition unit 616 asks the requesting part 62 to acquire quality control data of each of the specimen measurement apparatuses 11. For example, the requesting part 62 generates a URI on the basis of such asking, and requests information acquisition to the interface 5, 5A, by a command C generated by combining the generated URI and "GET" of the HTTP method. The URI that is generated by the requesting part 62 is, for example, "http://127.0.0.1:8080/XR/XR-20^AB123456^11000/QQQQ". In this URI, "server address" is designated as "127.0.0.1:8080", "interface ID" is designated as "XR", "apparatus ID" is designated as "XR-20^AB123456^11000", "resource" is designated as "QQQQ", and "query parameter" is not designated. "QQQQ" is the API endpoint corresponding to the quality control data. In the example of the URI above, the requesting part 62 requests acquisition of the measurement result of the quality control specimen executed by the specimen measurement apparatus 11 designated by the apparatus ID.

The requesting part 62 may generate a plurality of URIs respectively corresponding to a plurality of the specimen measurement apparatuses 11. In this case, the requesting part 62 generates a URI having a different "apparatus ID" for each specimen measurement apparatus 11. For example, the requesting part 62 can also collectively request, by one URL, quality control data of a plurality of the specimen measurement apparatuses 11. In "apparatus ID" of the URI in this case, for example, a plurality of apparatus IDs are enumerated by being separated with a predetermined delimiter (for example, [ ], |, and the like). The response acceptance part 63 accepts a response corresponding to the request from the requesting part 62, and passes the accepted information to the quality control data acquisition part 616.

The quality control data providing part 617 provides the quality control data on the basis of the information acquired by the quality control data acquisition part 616. The quality control data providing part 617 provides the quality control data through a GUI, for example. FIG. 42 is an example of providing quality control data through a GUI. In this example, a plurality of QC measurement results in a certain specimen measurement apparatus 11 are shown on a graph. The value of M shown in FIG. 42 indicates the median value or the average value calculated from the plurality of QC measurement results. W shown in FIG. 42 indicates a management width, and is $\pm 2$ SD with respect to M, for example.

(Example 4)

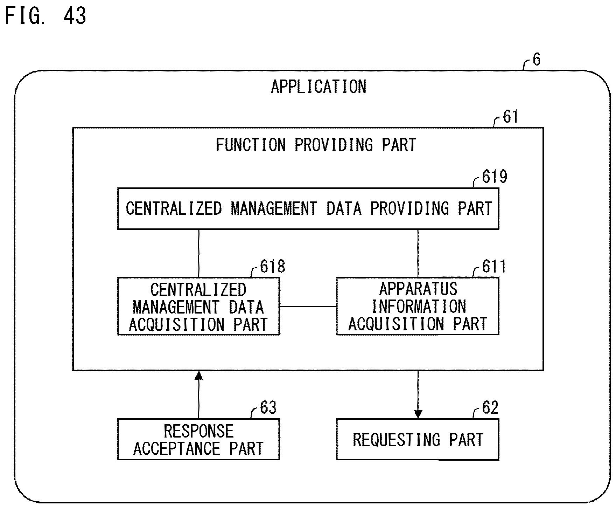

FIG. 43 shows a configuration example when the application 6 is a centralized management application. The centralized management application provides, for example, a function for performing centralized management of operation of a system including a plurality of the specimen measurement apparatuses 11 placed in a laboratory. The function providing part 61 of the application 6 of the present configuration example includes, for example, the apparatus information acquisition part 611, a centralized management data acquisition part 618, and a centralized management data providing part 619.

The apparatus information acquisition part 611 asks the requesting part 62 to acquire information regarding the specimen measurement apparatus 11 to serve as the target of centralized management. For example, similar to Example 1, the apparatus information acquisition part 611 requests the requesting part 62 to acquire information regarding the specimen measurement apparatus 11 to serve as the target of centralized management. For example, when the application 6 is used in a laboratory, the apparatus information acquisition part 611 asks the requesting part 62 to acquire information (for example, information regarding the kind of the specimen measurement apparatus 11, the apparatus ID of the specimen measurement apparatus 11, the name of the specimen measurement apparatus 11, and the like) regarding the specimen measurement apparatus 11 placed in the laboratory. For example, the apparatus information acquisition part 611 may ask the requesting part 62 to acquire information (for example, information regarding the kind of the specimen measurement apparatus 11, the apparatus ID of the specimen measurement apparatus 11, the name of the specimen measurement apparatus 11, and the like) regarding a predetermined kind of the specimen measurement apparatus 11 among a plurality of kinds of the specimen measurement apparatuses 11 placed in a laboratory. The apparatus information acquisition part 611 may ask the requesting part 62 to acquire information regarding all of the specimen measurement apparatuses 11 placed in the laboratory. The requesting part 62 requests the interface 5/5A to acquire information regarding the specimen measurement apparatus 11, by the same method as in Example 1. The response acceptance part 63 accepts a response corresponding to the request from the requesting part 62, and passes the accepted information to the apparatus information acquisition part 611.

The centralized management data acquisition part 618 asks the requesting part 62 to acquire data to serve as the target of centralized management, on the basis of the information on the specimen measurement apparatus 11 acquired by the apparatus information acquisition part 611. For example, the centralized management data acquisition part 618 asks the requesting part 62 to acquire data (for example, measurement result, running status, apparatus error information, reagent information, consumables information, and the like) of each of the specimen measurement apparatuses 11. For example, the requesting part 62 generates a URI on the basis of such asking, and requests information acquisition to the interface 5, 5A, by a command C generated by combining the generated URI and "GET" of the HTTP method. The URI that is generated by the quality control data acquisition part 616 is, for example, "http://127.0.0.1:8080/XR/XR-20^AB123456^11000/GGGG". In this URI example, "server address" is designated as "127.0.0.1:8080", "interface ID" is designated as "XR", "apparatus ID" is designated as "XR-20^AB123456^11000", "resource" is designated as "GGG", and "query parameter" is not designated. "GGGG" is the API endpoint corresponding to data for centralized management.

The requesting part 62 may generate a plurality of URIs respectively corresponding to a plurality of the specimen measurement apparatuses 11. In this case, the centralized management data acquisition part 618 generates a URI having a different "apparatus ID" for each specimen measurement apparatus 11. For example, the centralized management data acquisition part 618 can also collectively request, by one URI, quality control data of a plurality of specimen measurement apparatuses 11. In "apparatus ID" of the URI in this case, for example, a plurality of apparatus IDs are enumerated by being separated with a predetermined delimiters (for example, [ ], |, and the like). As for "GGGG" as "resource" described above, a plurality of resources (for example, a plurality of resources respectively corresponding to measurement result, running status, apparatus error information, reagent information, and consumables information) may be designated according to the kind of data to serve as the target of centralized management. For example, the requesting part 62 may generate a plurality of URIs respectively corresponding to a plurality of resources. The response acceptance part 63 accepts a response corresponding to the request from the requesting part 62, and passes the accepted information to the central management data acquisition part 618.

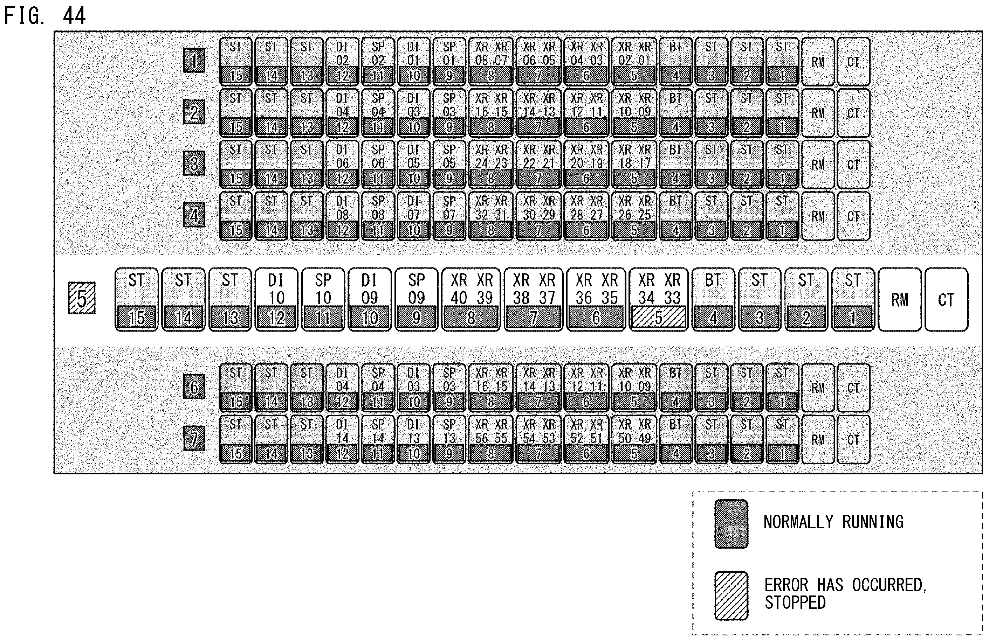

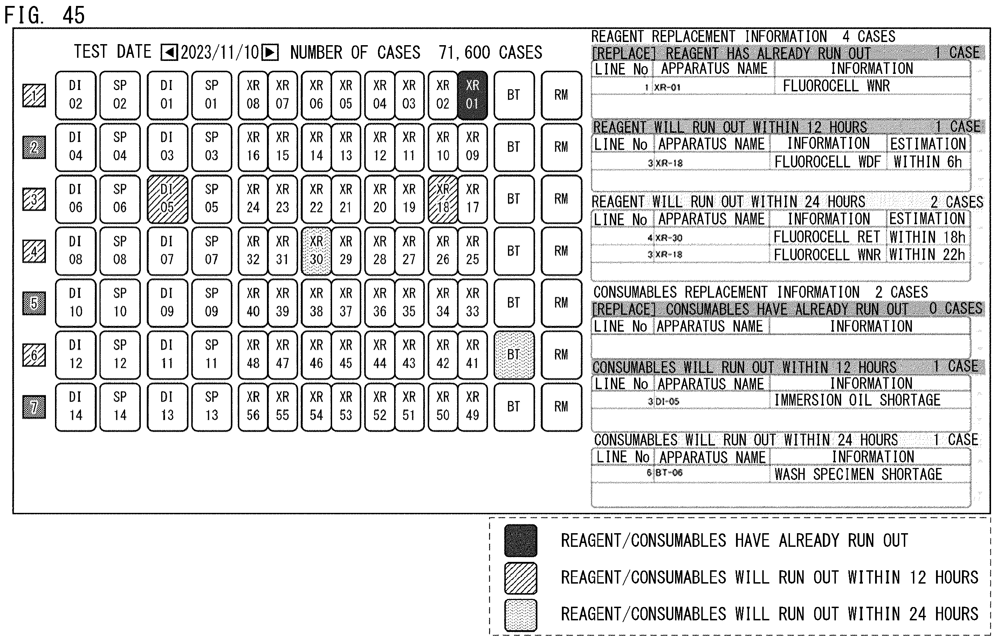

The centralized management data providing part 619 provides a function of performing centralized management of a system including a plurality of the specimen measurement apparatuses 11, on the basis of the information acquired by the centralized management data acquisition part 618. The centralized management data providing part 619 provides, for example, a GUI for performing centralized management of a system including a plurality of the specimen measurement apparatuses 11. FIG. 44 and FIG. 45 are examples of providing a GUI for performing centralized management of the system. The example shown in FIG. 44 is an example of displaying, in a list, the running state (normally running, error has occurred, stopped) of the system including a plurality of the specimen measurement apparatuses 11. In the example of FIG. 44, a GUI schematically showing lines for tests (in the example in FIG. 44, lines "1" to "7" are shown) formed by a plurality of apparatuses is provided. In the example shown in FIG. 44, each apparatus in the system is displayed in a display manner according to the running state, and a line that includes an apparatus in which an error has occurred and that has stopped is highlighted. In the example in FIG. 44, lines "1" to "7" are displayed, and line "5" having an apparatus where an error has occurred is highlighted. In the example in FIG. 44, "CT", "RM", "ST", "BT", "XR", "SP", and "DI" are symbols respectively representing the types of the apparatuses included in the system. The numeral displayed on the icon representing each apparatus is the identification number of the apparatus on the GUI. In the example in FIG. 44, two kinds of numbers (an identification number for each apparatus type such as "XR", and a serial number of "1" to "15" in each line) are displayed. The example shown in FIG. 45 is an example of a GUI that displays, in a list, the estimated remaining amounts (already run out/will run out within 12 hours/will run out within 24 hours) of reagents or consumables of a plurality of the specimen measurement apparatuses 11. In the example shown in FIG. 45, each of the specimen measurement apparatuses 11 is displayed in a display manner according to the estimated remaining amount. In the example in FIG. 45, lines "1" to "7" are displayed. In the example in FIG. 45, "RM", "BT", "XR", "SP", and "DI" are respectively symbols representing the types of the apparatuses included in the system. The numeral displayed on the icon representing each apparatus is the identification number of the apparatus on the GUI. In the example in FIG. 45, the display manner of the icon of each apparatus changes in accordance with the remaining amount of a reagent or a consumable .

(Configuration example of providing application function as Web service)

The function of the application 6 need not necessarily be provided as stand-alone software to be executed in the terminal apparatus 101 and the specimen measurement apparatus 11, and may be provided as a Web service.

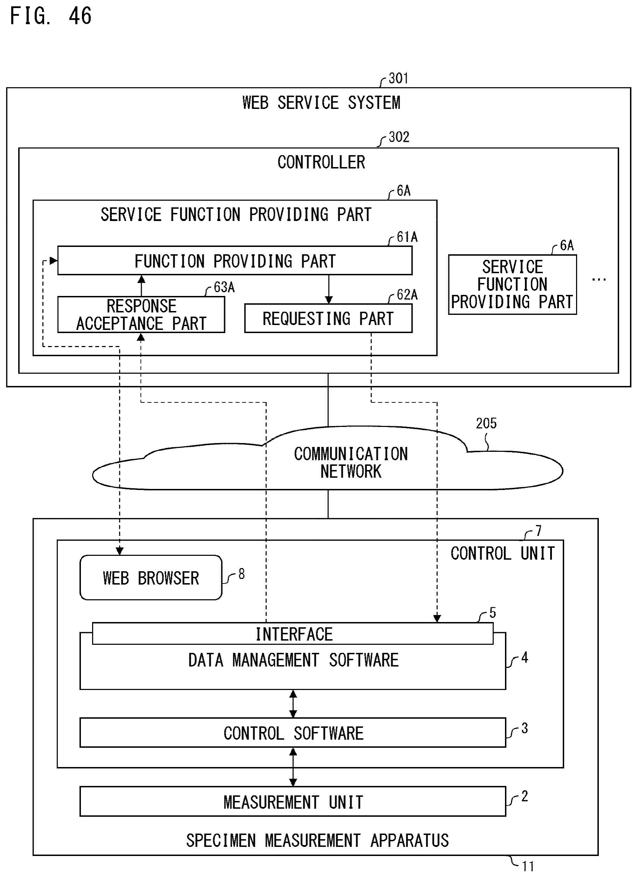

FIG. 46 shows a configuration example of providing the function of the application 6 as a Web service. In the configuration example shown in FIG. 46, the specimen measurement system 1 includes: a specimen measurement apparatus 11 having a Web browser 8 installed therein; and a Web service system 301 connected to the specimen measurement apparatus 11 via a communication network 205. The communication network 205 is, for example, the Internet. The specimen measurement apparatus 11 includes: the measurement unit 2; and the control unit 7 having the processor 71 that executes the control software 3, the data management software 4, the interface 5, and the Web browser 8.

The Web service system 301 includes a controller 302 having a processor that executes a service function providing part 6A functioning as a Web service. The service function providing part 6A has a function equivalent to that of the application 6. The service function providing part 6A includes, for example, a function providing part 61A, a requesting part 62A, and a response acceptance part 63A. The function providing part 61A, the requesting part 62A, and the response acceptance part 63A respectively have the functions equivalent to those of the function providing part 61, the requesting part 62, and the response acceptance part 63.

In the case of the present configuration example, when the service function providing part 6A functioning as a Web service is accessed by the Web browser 8 of the specimen measurement apparatus 11, information and function can be utilized via the interface 5 of the specimen measurement apparatus 11.

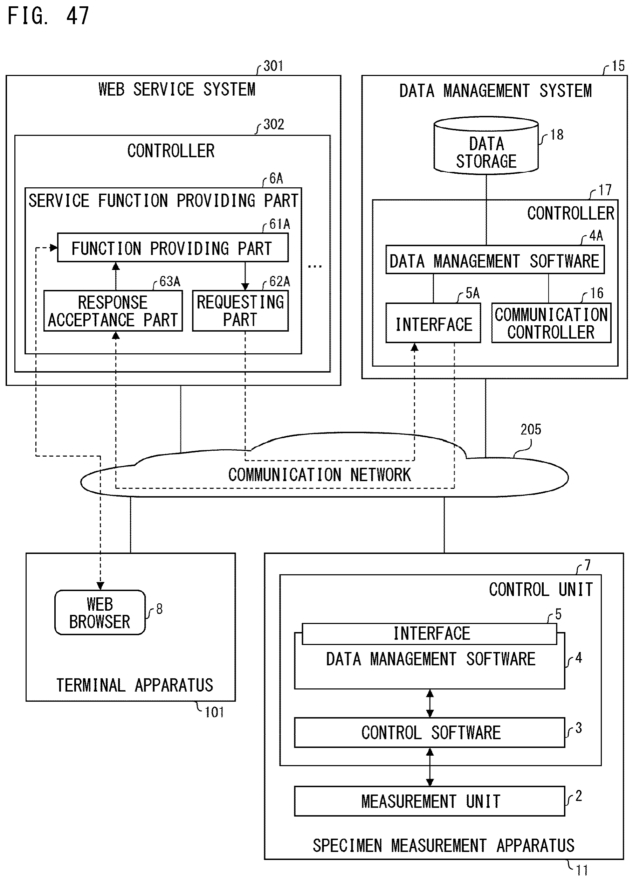

FIG. 47 shows another configuration example for providing the function of the application 6 as a Web service. In the configuration example shown in FIG. 47, the specimen measurement system 1 has a configuration in which the specimen measurement apparatus 11, the Web service system 301, the data management system 15, and the terminal apparatus 101 having the Web browser 8 installed therein are connected to each other via the communication network 205.

In the case of the present configuration example, when the service function providing part 6A functioning as a Web service is accessed by the Web browser 8 of the terminal apparatus 101, information managed in the data storage 18 can be utilized via the interface 5A of the data management system 15.

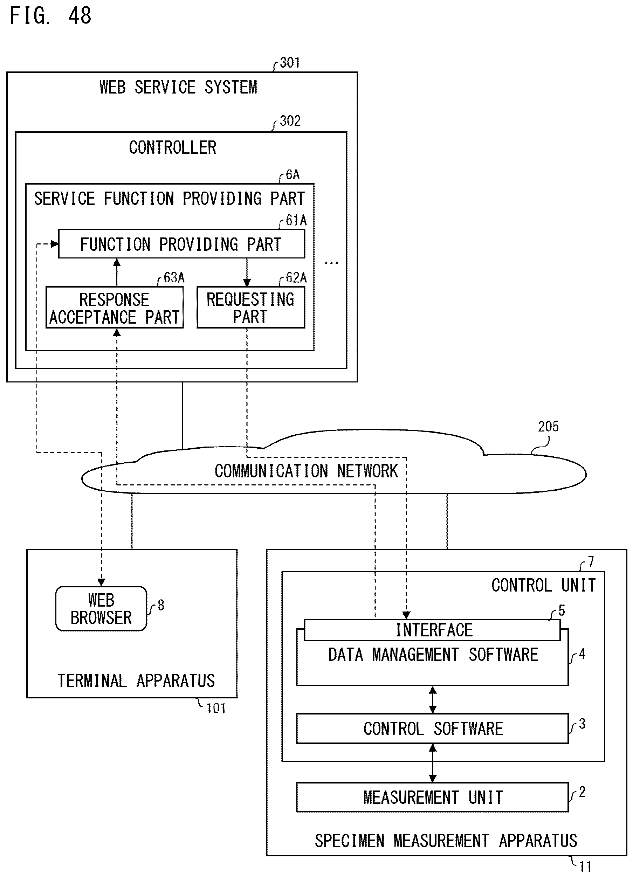

FIG. 48 shows still another configuration example for providing the function of the application 6 as a Web service. In the configuration example shown in FIG. 48, the specimen measurement system 1 has a configuration in which the specimen measurement apparatus 11, the Web service system 301, and the terminal apparatus 101 having the Web browser 8 installed therein are connected to each other via the communication network 205.

In the case of the present configuration example, when the service function providing part 6A functioning as a Web service is accessed by the Web browser 8 of the terminal apparatus 101, information and function can be utilized via the interface 5 of the specimen measurement apparatus 11.


(Provision form of application)


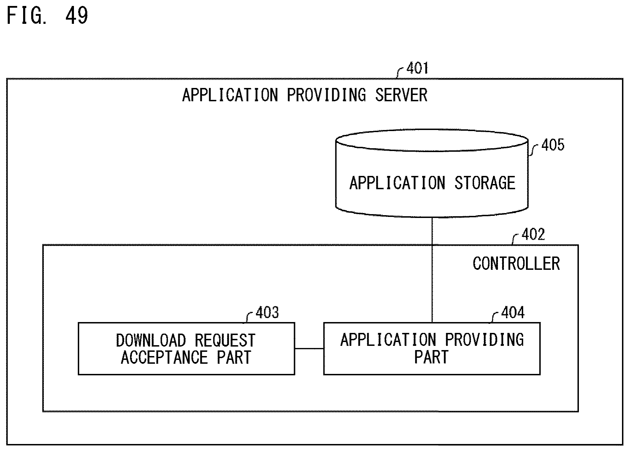

A provision form of the application 6 will be described. FIG. 49 shows a configuration example of an application providing server 401 that provides the application 6. The application providing server 401 is arranged on, for example, the cloud, and is communicably connected to the terminal apparatus 101 and the control unit 7 via a communication network. The terminal apparatus 101 and the control unit 7 are each, for example, a smartphone (iPhone, Android terminal), a tablet (iPad, Android tablet, Windows tablet), a Windows PC having Windows OS installed therein, or a Mac having Mac OS installed therein. The application providing server 401 includes: a controller 402 having a processor that executes a download request acceptance part 403 and an application providing part 404; and an application storage 405 having the application 6 stored therein.

The operator of the terminal apparatus 101 and the control unit 7 logs into a predetermined download application (for example, App Store, Google Play Store, Windows Store) using an account ID (for example, Apple ID, Google account, Microsoft account, or the like) managed by an application provider, for example, and requests download of a desired application 6 to the application providing server 401 via the download application.

For example, the download request acceptance part 403 notifies the application providing part 404 of information regarding the requested application 6. The application providing part 404 searches and acquires the application 6 notified from the application storage 405, and transmits the application 6 to the request source (the terminal apparatus 101 or the control unit 7). The application providing part 404 may transmit, for example, an installer of the application 6 to the request source (the terminal apparatus 101 or the control unit 7). The terminal apparatus 101 or the control unit 7 having received the installer installs the application 6 on the basis of the installer.

The application providing server 401 may be compatible with a multi-platform (e.g., Windows, MacOS, iOS, Android). A plurality of the application providing servers 401 may be present according to the kind of the platform. For example, the application providing server 401 (for example, App Store) that provides the application 6 for iOS, the application providing server 401 (for example, Google Play) that provides the application 6 for Android, the application providing server 401 (for example, Microsoft Store) that provides the application 6 for Windows, and the like. In this case, the terminal apparatus 101 and the control unit 7 request provision of the application 6 by a method according to the platform.

The terminal apparatus 101 and the control unit 7 having iOS installed therein request, for example, to the application providing server 401 that provides the application 6 for iOS, download of the desired application 6 searched for by a predetermined application (for example, an App Store). The terminal apparatus 101 and the control unit 7 having Android OS installed therein request, for example, to the application providing server 401 that provides the application 6 for Android, download of the desired application 6 searched for by a predetermined application (for example, Google Play store). The terminal apparatus 101 and the control unit 7 having Windows OS installed therein request, for example, to the application providing server 401 that provides the application 6 for Windows, download of the desired application 6 searched for by a predetermined application (for example, Microsoft Store).

Depending on the medical facility or the laboratory, it is assumable that the terminal apparatus 101 and the control unit 7 are not connected to the Internet or the like. In such cases, for example, from a medium (for example, CD/DVD/USB memory/external HDD/SDD) having stored therein an installer for the application 6, a maintenance person may copy the installer into the terminal apparatus 101 and the control unit 7, and may install the application 6 into the terminal apparatus 101 and the control unit 7.

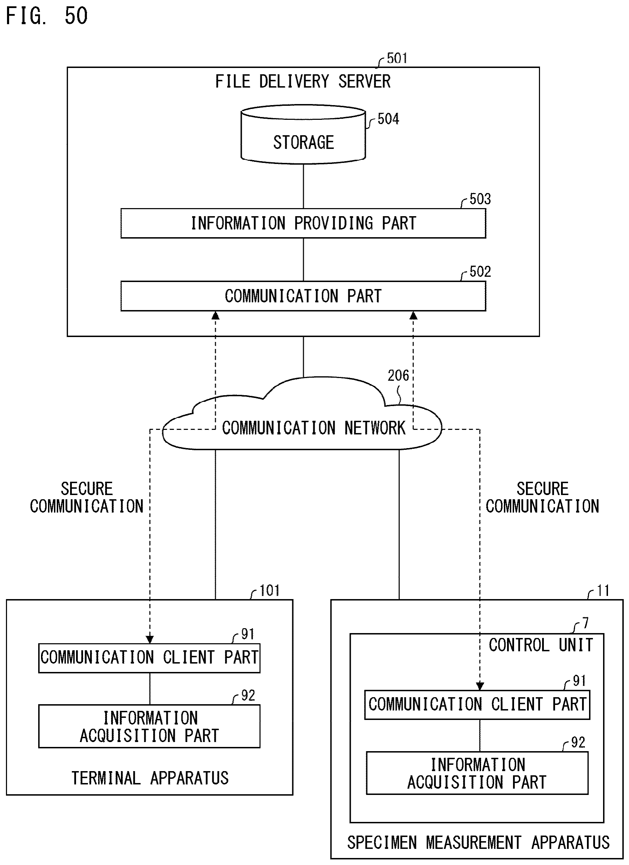

Depending on the medical facility or the laboratory, the terminal apparatus 101 and the control unit 7 may be allowed to perform communication with the outside only in a communication network (for example, VPN: Virtual Private Network) in which security is ensured. In this case, for example, from a server for which a secure communication network with respect to the terminal apparatus 101 and the control unit 7 can be set, the application 6 may be delivered. FIG. 50 shows a configuration example of providing the application 6 in a secure communication environment. The secure communication environment includes an environment in which a file delivery server 501 is placed in an intranet in a facility and can be accessed via the intranet from the terminal apparatus 101 and the control unit 7.

The file delivery server 501 includes: a processor that executes a communication part 502 and an information providing part 503; and a storage 504 having stored therein data (for example, installers and setting files of the applications 6) necessary for installing the application 6. The communication part 502 has, for example, a VPN server function. The information providing part 503 has, for example, a Web server function.

The terminal apparatus 101 and the control unit 7 each have a processor that executes a communication client part 91 and an information acquisition part 92. The communication client part 91 has, for example, a VPN client function, and sets a secure communication network 206 with the communication patt 502. The information acquisition part 92 is, for example, a Web browser.

The information acquisition part 92 of the terminal apparatus 101 and the control unit 7 accesses the information providing part 503 of the file delivery server 501 via the secure communication network 206. The information providing part 503 provides, for example, data that can be downloaded by the terminal apparatus 101 and the control unit 7, to the information acquisition part 92. The information acquisition part 92 presents the data in a Web page. When the operator of the terminal apparatus 101 and the control unit 7 selects data to be downloaded from the Web page, the information providing part 503 acquires corresponding data from the storage 504 and transmits the data to the information acquisition unit 92. The information acquisition part 92 installs the application 6 on the basis of the acquired data.

The terminal apparatus 101 and the control unit 7 may download an installer of the application 6 via the Internet and install the application 6 using the installer.

(Web Clip)

The function of the application 6 may be provided to the terminal apparatus 101 and the control unit 7 as a Web clip (a short-cut to a Web site). The service provider notifies, for example, the terminal apparatus 101 and the control unit 7 of a URL (Uniform Resource Locator) to serve as the access determination of the Web clip. The operator of the terminal apparatus 101 and the control unit 7 bookmarks the URL notified of, as a Web clip onto the home screen or the desktop of the terminal apparatus 101 and the control unit 7, for example. The operator of the terminal apparatus 101 and the control unit 7 may access a Website of the service provider by the Web browser installed in the terminal apparatus 101 and the control unit 7, access a menu providing a desired service from the Website, and bookmark the site of the menu as a Web clip, for example.

The system configuration example for utilizing the function of the application 6 by using a Web clip is similar to those of FIGS. 46 to 48. In the configuration example shown in FIG. 46, when the service function providing part 6A functioning as the Web service corresponding to the URL designated by the Web clip is accessed by the Web browser 8 of the control unit 7, information and functions can be utilized via the interface 5 of the specimen measurement apparatus 11. Similarly, in the configuration example shown in FIG. 47, when the service function providing part 6A functioning as the Web service corresponding to the URL designated by the Web clip is accessed by the Web browser 8 of the terminal apparatus 101, information managed in the data storage 18 can be utilized via the interface 5A of the data management system 15. Similarly, in the configuration example shown in FIG. 48, when the service function providing part 6A functioning as the Web service corresponding to the URL designated by the Web clip is accessed by the Web browser 8 of the terminal apparatus 101, information and functions can be utilized via the interface 5 of the specimen measurement apparatus 11.

(Provision of application by MDM/MAM)

The application 6 may be provided not via a predetermined application provider (For example, Apple Store, Google Play Store, Microsoft Store). For example, the application 6 may be provided to the terminal apparatus 101 and the control unit 7, by utilizing the mechanism of MDM (Mobile Device Management) or MAM (Mobile Application Management), and not via a predetermined application provider.

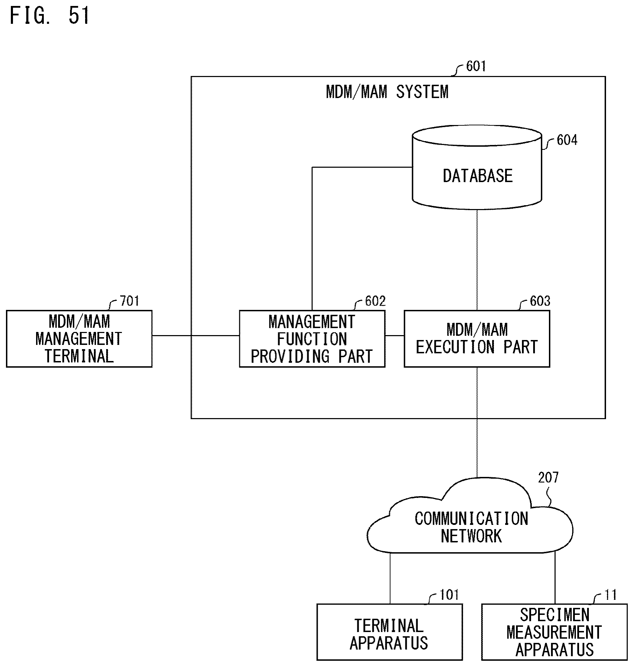

FIG. 51 shows a configuration example for providing the application 6 by utilizing MDM or MAM. The terminal apparatus 101 and the control unit 7 are communicably connected to an MDM/MAM system 601 via a communication network 207. The terminal apparatus 101 and the control unit 7 are registered in the MDM/MAM system 601 and managed by the MDM/MAM system 601 in accordance with a management policy. In order to manage the terminal apparatus 101 and the control unit 7 in the MDM/MAM system 601, for example, a management profile is installed into the terminal apparatus 101 and the control unit 7. For example, information (for example, a URL for downloading an installer) for installing the management profile is notified of (for example, by an electronic mail or the like) to the terminal apparatus 101 and the control unit 7.

When the management profile has been installed in the terminal apparatus 101 and the control unit 7, the terminal apparatus 101 and the control unit 7 are registered into the MDM/MAM system 601, and information regarding the terminal apparatus 101 and the control unit 7 is registered into a database 604 of the MDM/MAM system 601.

An MDM/MAM execution part 603 acquires information on the application 6 installed in the terminal apparatus 101 and the control unit 7, and registers the information into an application list of the database 604. The MDM/MAM execution part 603 can deliver the application 6 to the terminal apparatus 101 and the control unit 7. The terminal apparatus 101 and the control unit 7 install the delivered application 6. Accordingly, the application 6 can be provided not via a predetermined application provider (App Store or the like).

A management function providing part 602 provides a management function of the terminal apparatus 101 and the control unit 7 to an MDM/MAM management terminal 701. For example, the management function providing part 602 provides, to the MDM/MAM management terminal 701, a Website for managing the terminal apparatus 101 and the control unit 7. Via the Web site, the operator of the MDM/MAM management terminal 701 executes: registration and change of the management policy to the terminal apparatus 101 and the control unit 7; delivery of the application 6 to the terminal apparatus 101 and the control unit 7; and management of the application 6 installed in the terminal apparatus 101 and the control unit 7, for example.

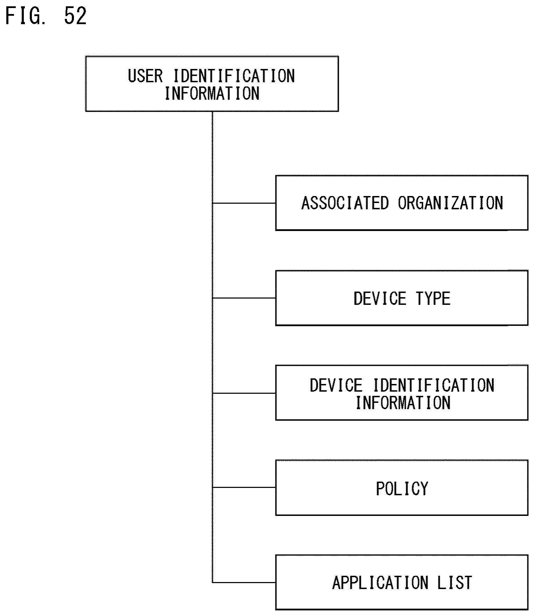

FIG. 52 shows an example of the data structure of the database 604 when the database 604 is a relational database. As shown in FIG.52, the information managed by the database 604 includes, for example, user identification information, associated organization, device type, device identification information, policy, and application list.

The user identification information is information for identifying the user of the terminal apparatus 101 and the control unit 7. For example, an ID assigned to the user from the MDM/MAM system 601, the name of the user, a combination thereof, and the like are included.

The associated organization is identification information of the organization to which the terminal apparatus 101 and the control unit 7 are associated. For example, an ID assigned to the organization from the MDM/MAM system 601, the organization name, a combination thereof, and the like are included.

The device type is information for identifying the platform of the device. For example, iOS, Android, Windows, Mac, and the like are included.

The device identification information is identification information unique to the terminal apparatus 101 and the control unit 7 to serve as the management target in the MDM/MAM system 601. Examples include IMEI (International Mobile Equipment Identifier), serial numbers, SIM (Subscriber Identity Module) card number, telephone numbers, MAC (Media Access Control) addresses, combinations thereof, and the like.

The policy is the management policy by the MDM/MAM system 601. For example, setting information of the management policy is included.

The application list is information regarding the application 6 installed in the terminal apparatus 101 and the control unit 7 corresponding to the user identification information. For example, a list identification information (application name, application ID) of the application 6 and the version of the application 6 are included.

The present invention is not limited to the embodiments described above, and various modifications can be made within the scope of the claims. Embodiments obtained by combining, as appropriate, the technological means disclosed in different embodiments are also included in the technological scope of the present invention.

(Remarks)

The present disclosure includes following items 1-38.

Item 1. A system including a measurement unit configured to measure a specimen, the system comprising:

control software configured to control the measurement unit;

data management software configured to manage data regarding the measurement unit that operates under control by the control software; and

an interface configured to allow application software capable of being added to the system, to utilize the data, wherein a function of the system is expanded by the application software added to the system.

Item 2. The system of item 1, wherein

the interface allows the application software to utilize the data, on the basis of a predetermined rule.

Item 3 The system of item 1, wherein

the application software includes a plurality of kinds of the application software that provide different functions, and

the interface allows the plurality of kinds of the application software to utilize the data on the basis of a predetermined rule being in common with respect to the plurality of kinds of the application software.

Item 4. The system of item 1, wherein

the interface provides, in accordance with a request from the application software, a response corresponding to the request, to the application software.

Item 5. The system of item 1, wherein

the interface allows, on the basis of a command created by the application software in accordance with a predetermined rule, the application software to utilize the data.

Item 6. The system of item 1, wherein

the interface allows, on the basis of a command created by the application software in accordance with a predetermined rule, the application software to utilize at least one of the data regarding the measurement unit, the data regarding measurement operation by the measurement unit, the data regarding maintenance of the measurement unit, and the data regarding manipulation of the measurement unit.

Item 7. The system of item 1, wherein

the interface allows, on the basis of a command created by the application software in accordance with a predetermined rule, the application software to execute at least one of acquisition of the data, registration of the data, update of the data, and deletion of the data.

Item 8. The system of item 1, wherein

the interface allows, on the basis of a predetermined rule being in common with respect to a plurality of kinds of the application software that respectively operate on a plurality of kinds of operating systems, the plurality of kinds of the application software to utilize the data.

Item 9. The system of item 1, wherein

the data management software manages the data on the basis of classification according to a kind of the data.

Item 10. The system of item 1, wherein

the interface allows the application software to utilize the data on the basis of a predetermined rule, and

the data management software manages the data on the basis of classification corresponding to the predetermined rule.

Item 11. The system of item 1, wherein

the system comprises a specimen measurement apparatus including the measurement unit, the control software, and the data management software, and

the interface allows the application software to utilize the data, on the basis of a plurality of predetermined rules respectively corresponding to kinds of the specimen measurement apparatus.

Item 12. The system of item 1, wherein

the system comprises a specimen measurement apparatus including the measurement unit, the control software, and the data management software, and

the specimen measurement apparatus has been certified as a medical device.

Item 13. The system of item 1, wherein
the interface allows the application software being a non-medical device to utilize the data.
Item 14. The system of item 1, wherein
the control software and the data management software are software independent of the application software.
Item 15. The system of item 1, wherein

the measurement unit includes a specimen processing part configured to prepare a measurement sample on the basis of the specimen and a reagent, and a detection part configured to measure the prepared measurement sample,
the control software controls operation of the specimen processing part and the detection part linking with the data management software, and
the data management software manages the data including a measurement result obtained through operation of the specimen processing part and the detection part.

Item 16. The system of item 1, wherein

the measurement unit includes a specimen processing part configured to prepare a measurement sample from the specimen and a reagent, and a detection part configured to measure the prepared measurement sample,
the control software controls operation of the specimen processing part and the detection part linking with the data management software,
the data management software manages the data including a measurement result obtained through operation of the specimen processing part and the detection part, and
the control software and the data management software are software independent of the application software.

Item 17. The system of item 1, wherein

the measurement unit includes a specimen processing part configured to prepare a measurement sample from the specimen and a reagent, and a detection part configured to measure the prepared measurement sample,
the control software controls operation of the specimen processing part and the detection part linking with the data management software,
the data management software manages the data including a measurement result obtained through operation of the specimen processing part and the detection part,
the control software and the data management software are software independent of the application software, and
the data management software is capable of linking with the application software via the interface.

Item 18. The system of item 1, wherein

the system comprises a specimen measurement apparatus including the measurement unit, the control software, and the data management software,
the measurement unit includes a specimen processing part configured to prepare a measurement sample from the specimen and a reagent, and a detection part configured to measure the prepared measurement sample,
the control software controls operation of the specimen processing part and the detection part linking with the data management software,
the data management software manages the data including a measurement result obtained through operation of the specimen processing part and the detection part,
the data management software is capable of linking with the application software via the interface,
the measurement unit, the control software, and the data management software are components of a certified medical device, and
the control software and the data management software are software independent of the application software.

Item 19. The system of item 1, wherein

the system comprises a specimen measurement apparatus including the measurement unit, the control software, and the data management software,
the measurement unit includes a specimen processing part configured to prepare a measurement sample from the specimen and a reagent, and a detection part configured to measure the prepared measurement sample,
the control software controls operation of the specimen processing part and the detection part linking with the data management software,
the data management software manages the data including a measurement result obtained through operation of the specimen processing part and the detection part,

the data management software is capable of linking with the application software via the interface,

the measurement unit, the control software, and at least a part of the data management software provide a function corresponding to an intended use of the specimen measurement apparatus having been certified as a medical device, and

the function of the system is expanded by the application software capable of providing a function different from the function corresponding to the intended use.

Item 20. The system of items 15 to 19, wherein operation control, performed by the control software, of the specimen processing part and the detection part includes at least one of:

control of suction operation of a liquid including at least one of the specimen and the reagent;

control of mixing the specimen and the reagent to prepare the measurement sample;

control of causing the detection part to detect the measurement sample; and

control of causing the detection part to detect at least one of optical information and an electric signal regarding the measurement sample, and analyzing a measurement result on the basis of a result of a detection.

Item 21. Application software to be used in a system including a measurement unit configured to measure a specimen, control software configured to control the measurement unit, and data management software configured to manage data regarding the measurement unit that operates under control by the control software,

the application software comprising:

a requesting part configured to request utilization of the data to an interface;

a response receiving part configured to receive a response corresponding to the request; and

a function providing part configured to provide a function for expanding the system, on the basis of the utilization of the data.

Item 22. The application software of item 21, wherein the requesting part requests the utilization of the data to the interface on the basis of a predetermined rule.

Item 23. The application software of item 21, wherein the requesting part requests, on the basis of a predetermined rule being in common with respect to a plurality of kinds of the application software that provide different functions, the utilization of the data to the interface.

Item 24. The application software of item 21, wherein the requesting part requests, on the basis of a command created in accordance with a predetermined rule, the utilization of the data to the interface.

Item 25. The application software of item 21, wherein the requesting part requests, on the basis of a command created in accordance with a predetermined rule, to the interface, utilization of at least one of the data regarding the measurement unit, the data regarding measurement operation by the measurement unit, the data regarding maintenance of the measurement unit, and the data regarding manipulation of the measurement unit.

Item 26. The application software of item 21, wherein the requesting part requests, on the basis of a command created in accordance with a predetermined rule, to the interface, utilization of at least one of acquisition of the data, registration of the data, update of the data, and deletion of the data.

Item 27. The application software of item 21, wherein the requesting part requests, on the basis of a predetermined rule being in common with respect to a plurality of kinds of the application software that respectively operate on a plurality of kinds of operating systems, the utilization of the data to the interface.

Item 28. The application software of item 21, wherein

the system comprises a specimen measurement apparatus including the measurement unit, the control software, and the data management software, and

the requesting part requests the utilization of the data to the interface, on the basis of a plurality of predetermined rules respectively corresponding to kinds of the specimen measurement apparatus.

Item 29. The application software of item 21, wherein

the system comprises a specimen measurement apparatus including the measurement unit, the control software, and the data management software, and
the specimen measurement apparatus has been certified as a medical device.

Item 30. The application software of item 21, wherein
the application software is software independent of the control software and the data management software.

Item 31. A linking method implemented in a system including a measurement unit configured to measure a specimen, control software configured to control the measurement unit, and data management software configured to manage data regarding the measurement unit that operates under control by the control software; and application software that is added to the system, the linking method comprising

receiving a request from the application software configured to provide a function for expanding the system,
analyzing the request on the basis of a predetermined rule, and
providing, on the basis of the request having been analyzed, a response corresponding to utilization of the data according to the request, to the application software.

Item 32. The linking method of item 31, wherein
the request is analyzed on the basis of the predetermined rule being in common with respect to a plurality of kinds of the application software that provide different functions.

Item 33. The linking method of item 31, wherein
a response corresponding to the utilization of the data is provided to the application software according to a command created by the application software in accordance with the predetermined rule.

Item 34. The linking method of item 31, wherein
a response is provided to the application software, according to a command created by the application software in accordance with the predetermined rule, the response corresponding to utilization of at least one of the data regarding the measurement unit, the data regarding measurement operation by the measurement unit, the data regarding maintenance of the measurement unit, and the data regarding manipulation of the measurement unit.

Item 35. The linking method of item 31, wherein
a response is provided to the application software, according to a command created by the application software in accordance with the predetermined rule, the response corresponding to execution of at least one of acquisition of the data, registration of the data, update of the data, and deletion of the data.

Item 36. The linking method of item 31, wherein
the request is analyzed on the basis of the predetermined rule being in common with respect to a plurality of kinds of the application software that respectively operate on a plurality of kinds of operating systems.

Item 37. The linking method of item 31, wherein

the system comprises a specimen measurement apparatus including the measurement unit, the control software, and the data management software, and
the specimen measurement apparatus has been certified as a medical device.

Item 38. The linking method of item 31, wherein
the application software is software independent of the control software and the data management software. Further preferred embodiments of the present invention are defined as E1-E20 as follows:

E1. A specimen measurement apparatus including a measurement unit configured to measure a specimen, the specimen measurement apparatus comprising:

control software configured to control the measurement unit;
data management software configured to manage data regarding the measurement unit that operates under control by the control software; and
an interface configured to allow application software capable of being added to a system including the specimen measurement apparatus, to utilize the data, wherein
a function of the system is expanded by the application software added to the system.

E2. The specimen measurement apparatus of embodiment E1, wherein
the interface allows the application software to utilize the data, on the basis of a predetermined rule.

E3. The specimen measurement apparatus of embodiment E1, wherein

the application software includes a plurality of kinds of the application software that provide different functions, and

the interface allows the plurality of kinds of the application software to utilize the data on the basis of a predetermined rule being in common with respect to the plurality of kinds of the application software.

E4. The specimen measurement apparatus of embodiment E1, wherein
the interface provides, in accordance with a request from the application software, a response corresponding to the request, to the application software.

E5. The specimen measurement apparatus of embodiment E1, wherein
the interface allows, on the basis of a command created by the application software in accordance with a predetermined rule, the application software to utilize the data.

E6. The specimen measurement apparatus of embodiment E1, wherein
the interface allows, on the basis of a command created by the application software in accordance with a predetermined rule, the application software to utilize at least one of the data regarding the measurement unit, the data regarding measurement operation by the measurement unit, the data regarding maintenance of the measurement unit, and the data regarding manipulation of the measurement unit.

E7. The specimen measurement apparatus of embodiment E1, wherein
the interface allows, on the basis of a command created by the application software in accordance with a predetermined rule, the application software to execute at least one of acquisition of the data, registration of the data, update of the data, and deletion of the data.

E8. The specimen measurement apparatus of embodiment E1, wherein
the interface allows, on the basis of a predetermined rule being in common with respect to a plurality of kinds of the application software that respectively operate on a plurality of kinds of operating systems, the plurality of kinds of the application software to utilize the data.

E9. The specimen measurement apparatus of embodiment E1, wherein
the data management software manages the data on the basis of classification according to a kind of the data.

E10. The specimen measurement apparatus of embodiment E1, wherein

the interface allows the application software to utilize the data, on the basis of a predetermined rule, and
the data management software manages the data on the basis of classification corresponding to the predetermined rule.

E11. The specimen measurement apparatus of embodiment E1, wherein
the interface allows the application software to utilize the data, on the basis of a plurality of predetermined rules respectively corresponding to kinds of the specimen measurement apparatuses.

E12. The specimen measurement apparatus of embodiment E1, wherein
the specimen measurement apparatus has been certified as a medical device.

E13. The specimen measurement apparatus of embodiment E1, wherein
the interface allows the application software being a non-medical device to utilize the data.

E14. The specimen measurement apparatus of embodiment E1, wherein
the control software and the data management software are software independent of the application software.

E15. The specimen measurement apparatus of embodiment E1, wherein

the measurement unit includes a specimen processing part configured to prepare a measurement sample on the basis of the specimen and a reagent, and a detection part configured to measure the prepared measurement sample,
the control software controls operation of the specimen processing part and the detection part linking with the data management software, and

the data management software manages the data including a measurement result obtained through operation of the specimen processing part and the detection part.

E16. The specimen measurement apparatus of embodiment E1, wherein

the measurement unit includes a specimen processing part configured to prepare a measurement sample on the basis of the specimen and a reagent, and a detection part configured to measure the prepared measurement sample,
the control software controls operation of the specimen processing part and the detection part linking with the data management software,
the data management software manages the data including a measurement result obtained through operation of the specimen processing part and the detection part, and
the control software and the data management software are software independent of the application software.

E17. The specimen measurement apparatus of embodiment E1, wherein

the measurement unit includes a specimen processing part configured to prepare a measurement sample on the basis of the specimen and a reagent, and a detection part configured to measure the prepared measurement sample,
the control software controls operation of the specimen processing part and the detection part linking with the data management software,
the data management software manages the data including a measurement result obtained through operation of the specimen processing part and the detection part,
the control software and the data management software are software independent of the application software, and
the data management software is capable of linking with the application software via the interface.

E18. The specimen measurement apparatus of embodiment E1, wherein

the measurement unit includes a specimen processing part configured to prepare a measurement sample on the basis of the specimen and a reagent, and a detection part configured to measure the prepared measurement sample,
the control software controls operation of the specimen processing part and the detection part linking with the data management software,
the data management software manages the data including a measurement result obtained through operation of the specimen processing part and the detection part,
the data management software is capable of linking with the application software via the interface, and
the control software and the data management software are software independent of the application software.

E19. The specimen measurement apparatus of embodiment E1, wherein

the measurement unit includes a specimen processing part configured to prepare a measurement sample on the basis of the specimen and a reagent, and a detection part configured to measure the prepared measurement sample,
the control software controls operation of the specimen processing part and the detection part linking with the data management software,
the data management software manages the data including a measurement result obtained through operation of the specimen processing part and the detection part,
the data management software is capable of linking with the application software via the interface,
the measurement unit, the control software, and at least a part of the data management software provide a function corresponding to an intended use of the specimen measurement apparatus having been certified as a medical device, and
the function of the system including the specimen measurement apparatus is expanded by the application software capable of providing a function different from the function corresponding to the intended use.

E20. The specimen measurement apparatus of any one of embodiments E15 to E19, wherein
operation control, performed by the control software, of the specimen processing part and the detection part includes at least one of:

control of suction operation of a liquid including at least one of the specimen and the reagent;

control of mixing the specimen and the reagent to prepare the measurement sample;

control of causing the detection part to detect the measurement sample; and

control of causing the detection part to detect at least one of optical information and an electric signal regarding the measurement sample, and analyzing a measurement result on the basis of a result of a detection.

DESCRIPTION OF THE REFERENCE CHARACTERS

1 specimen measurement system
2 measuring unit
3 control software
4,4A data management software
5,5A interface
6 application software
11 specimen measurement apparatus
21 sample processing part
22 detection part
61 function providing part
62 requesting part
63 response acceptance unit
C command
R predetermined rule

**Claims**

1. A specimen measurement apparatus operable to optically perform a measurement on a specimen, the specimen measurement apparatus comprising:

   (i) a specimen suction part configured to suction the specimen, the specimen suction part comprising a nozzle to suction the specimen in a specimen container, a movement mechanism configured to move the nozzle into the specimen container and a pump configured to apply a negative pressure so that the nozzle suctions the specimen;
   (ii) a sample preparator configured to prepare a measurement sample by mixing the suctioned specimen and a reagent in a container; and
   (iii) an optical detector configured to detect light from the prepared measurement sample;
   (iv) a controller configured to control, according to a control software, the specimen suction part, the sample preparator and the optical detector so that the specimen measurement apparatus optically performs the measurement on the specimen, wherein the specimen measurement apparatus analyzes, in order to obtain a measurement result, optical information which is derived from the detected light, and manages, according to a management software, data relating to the specimen analyzer, wherein the data includes the measurement result, and
   (v) a computer capable of running an interface which interacts with each of a plurality of kinds of application software each programmed to use the data, wherein the plurality of kinds of application software are independent of the specimen analyzer, wherein the interface interacts with the application software according to a predetermined rule which is in common among the plurality of kinds of application software,

   wherein the interface executes operations including:

   (a) receiving a request from at least one of the plurality of kinds of application software;
   (b) retrieving, according to the predetermined rule, the data corresponding to the request; and
   (c) providing the retrieved data to the at least one of the plurality of kinds of application software.

2. The specimen measurement apparatus of claim 1, wherein
the interface interacts with the at least one of the plurality of kinds of application software on the basis of a command created by the at least one of the plurality of kinds of application software in accordance with the predetermined rule.

**3.** The specimen measurement apparatus of claim 1, wherein
the interface interacts with the at least one of the plurality of kinds of application software on the basis of a command created by the at least one of the plurality of kinds of application software in accordance with the predetermined rule, wherein the data provided by the interface is at least one of the data regarding the specimen measurement apparatus, the data regarding measurement operation by the specimen measurement apparatus, the data regarding maintenance of the specimen measurement apparatus, and the data regarding manipulation of the specimen measurement apparatus.

**4.** The specimen measurement apparatus of claim 1, wherein
the interface interacts with the at least one of the plurality of kinds of application software on the basis of a command created by the at least one of the plurality of kinds of application software in accordance with the predetermined rule, wherein the command includes at least one of acquisition of the data, registration of the data, update of the data, and deletion of the data.

**5.** The specimen measurement apparatus of claim 1, wherein
the interface interacts with the at least one of the plurality of kinds of application software on the basis of the predetermined rule, wherein the plurality of kinds of the application software respectively operates on a plurality of kinds of operating systems.

**6.** The specimen measurement apparatus of claim 1, wherein
the specimen measurement apparatus manages, according to the data management software, the data on the basis of classification according to a kind of the data.

**7.** The specimen measurement apparatus of claim 1, wherein
the specimen measurement apparatus manages, according to the data management software, the data on the basis of classification corresponding to the predetermined rule.

**8.** The specimen measurement apparatus of claim 1, wherein
the interface interacts with the at least one of the plurality of kinds of application software on the basis of a plurality of the predetermined rules respectively corresponding to kinds of the specimen measurement apparatus.

**9.** The specimen measurement apparatus of claim 1, wherein
the specimen measurement apparatus has been certified as a medical device.

**10.** The specimen measurement apparatus of claim 1, wherein
the interface interacts with each of the plurality of kinds of application software being a non-medical device.

**11.** The specimen measurement apparatus of claim 1, wherein
the control software and the data management software are independent of each of the plurality of kinds of application software.

**12.** The specimen measurement apparatus of claim 1, wherein

the controller is configured to control, according to the control software, operation of the specimen suction part, the sample preparator and the optical detector collaboratively with the data management software, and
the specimen measurement apparatus manages, according to the data management software, the data including the measurement result obtained through the operation of the specimen suction part, the sample preparator and the optical detector.

**13.** The specimen measurement apparatus of claim 1, wherein

the controller is configured to control, according to the control software, operation of the specimen suction part, the sample preparator and the optical detector collaboratively with the data management software,
the specimen measurement apparatus manages, according to the data management software, the data including the measurement result obtained through the operation of the specimen suction part, the sample preparator and the optical detector, and
the control software and the data management software are independent of each of the plurality of kinds of application software.

**14.** The specimen measurement apparatus of claim 1, wherein

the controller is configured to control, according to the control software, operation of the specimen suction part, the sample preparator and the optical detector collaboratively with the data management software,
the specimen measurement apparatus manages, according to the data management software, the data including the measurement result obtained through the operation of the specimen suction part, the sample preparator and the optical detector,
the control software and the data management software are independent of each of the plurality of kinds of application software, and
the data management software is capable of collaborating with each of the plurality of kinds of application software via the interface.

**15.** The specimen measurement apparatus of claim 1, wherein

the specimen measurement apparatus comprises the control software and the data management software,
the controller is configured to control, according to the control software, operation of the specimen suction part, the sample preparator and the optical detector collaboratively with the data management software,
the specimen measurement apparatus manages, according to the data management software, the data including the measurement result obtained through the operation of the specimen suction part, the sample preparator and the optical detector,
the data management software is capable of collaborating with each of the plurality of kinds of application software via the interface,
the specimen measurement apparatus, the control software, and the data management software are components of a certified medical device, and
the control software and the data management software are independent of each of the plurality of kinds of application software.

FIG. 1

APPLICATION
NOT HAVING
BEEN ADDED
⌐6

⌐1

ADDED
APPLICATION
⌐6

ADDED
APPLICATION
⌐6

ADDED
APPLICATION
⌐6

...

INTERFACE ⌐5

DATA MANAGEMENT SOFTWARE ⌐4

CONTROL SOFTWARE ⌐3

MEASUREMENT UNIT ⌐2

FIG. 2

COMPUTER 900

PROCESSOR 71

MEMORY 72

BUS 73

STORAGE PART 74

PROGRAM

INTERFACE 751

DISPLAY PART 75

INTERFACE 761

MANIPULATION PART 76

COMMUNICATION PART 78

FIG. 3

SPECIMEN MEASUREMENT APPARATUS

FIG. 4

LABORATORY

1

201

COMMUNICATION NETWORK

101

TERMINAL APPARATUS

APPLICATION 6
APPLICATION 6
APPLICATION 6
...

CONTROL UNIT 7

INTERFACE 5

DATA MANAGEMENT SOFTWARE 4

CONTROL SOFTWARE 3

MEASUREMENT UNIT 2

SPECIMEN MEASUREMENT APPARATUS 11

FIG. 5

APPLICATION  APPLICATION  APPLICATION  ...

TERMINAL APPARATUS

COMMUNICATION NETWORK

LABORATORY

CONTROL UNIT

INTERFACE

DATA MANAGEMENT SOFTWARE

CONTROL SOFTWARE

MEASUREMENT UNIT

SPECIMEN MEASUREMENT APPARATUS

FIG. 6

COMMUNICATION
NETWORK
203

1

**CONTROL UNIT** 7

APPLICATION 6

INTERFACE 5

DATA MANAGEMENT SOFTWARE 4

CONTROL SOFTWARE 3

MEASUREMENT UNIT 2

SPECIMEN MEASUREMENT APPARATUS 11

**CONTROL UNIT** 7

APPLICATION 6

INTERFACE 5

DATA MANAGEMENT SOFTWARE 4

CONTROL SOFTWARE 3

MEASUREMENT UNIT 2

SPECIMEN MEASUREMENT APPARATUS 11

...

FIG. 7

1

**DATA MANAGEMENT SYSTEM** — 15

DATA STORAGE — 18

CONTROLLER — 17

DATA MANAGEMENT SOFTWARE — 4A

INTERFACE — 5A

COMMUNICATION CONTROLLER — 16

DATA UTILIZATION VIA INTERFACE

COMMUNI-CATION NETWORK — 204

ACCUMULATION OF DATA SUCH AS MEASUREMENT DATA

CONTROL UNIT — 7

APPLICATION — 6

INTERFACE — 5

DATA MANAGEMENT SOFTWARE — 4

CONTROL SOFTWARE — 3

MEASUREMENT UNIT — 2

SPECIMEN MEASUREMENT APPARATUS — 11

FIG. 8

FIG. 9

FIG. 10

2:MEASUREMENT UNIT

11:SPECIMEN
MEASUREMENT
APPARATUS

7:CONTROL UNIT

FIG. 11

FIG. 12

CONTROL UNIT _7_

DATA MANAGEMENT SOFTWARE _4_

RESULT PROVIDING PART _41_

OPERATION PART _42_

CONTROL SOFTWARE _3_

ANALYSIS PART _32_

UNIT CONTROLLER _31_

STORAGE PART _74_

MEASUREMENT DATA

ORDER

MEASUREMENT UNIT _2_

EP 4 752 902 A2

FIG. 13

TO DATA MANAGEMENT SYSTEM 15

CONTROL UNIT ⌐7

CONTROL SOFTWARE ⌐3

ANALYSIS PART ⌐32

UNIT CONTROLLER ⌐31

MEASUREMENT DATA

ORDER

MEASUREMENT UNIT ⌐2

FIG. 14

```
                                        2
┌──────────────────────────────────────────────────────┐
│                  MEASUREMENT UNIT                      │
│                                                        │
│          22                            21              │
│  ┌──────────────────┐         ┌──────────────────┐    │
│  │                  │         │     SPECIMEN     │    │
│  │  DETECTION PART  │─────────│ PROCESSING PART  │    │
│  │                  │         │                  │    │
│  └──────────────────┘         └──────────────────┘    │
│                                                        │
└──────────────────────────────────────────────────────┘
```

FIG. 15

```
                                        2
┌──────────────────────────────────────────────────────────────────┐
│                      MEASUREMENT UNIT                              │
│                                                                    │
│        22                              21                          │
│ ┌─────────────────────┐      ┌──────────────────────────┐        │
│ │   DETECTION PART     │      │ SPECIMEN PROCESSING PART │        │
│ │                      │      │                          │        │
│ │        221           │      │          211             │        │
│ │ ┌─────────────────┐  │      │  ┌───────────────────┐   │        │
│ │ │ FCM DETECTION PART │    │  │ SPECIMEN SUCTION  │   │        │
│ │ └─────────────────┘  │      │  │      PART         │   │        │
│ │                      │      │  └───────────────────┘   │        │
│ │        222           │      │                          │        │
│ │ ┌─────────────────┐  │      │          212             │        │
│ │ │ RBC/PLT DETECTION │    │  │  ┌───────────────────┐   │        │
│ │ │      PART        │  │      │  │ SAMPLE PREPARATION│   │        │
│ │ └─────────────────┘  │      │  │      PART         │   │        │
│ │                      │      │  └───────────────────┘   │        │
│ │        223           │      │                          │        │
│ │ ┌─────────────────┐  │      │                          │        │
│ │ │ HGB DETECTION PART │    │                          │        │
│ │ └─────────────────┘  │      │                          │        │
│ │                      │      │                          │        │
│ └─────────────────────┘      └──────────────────────────┘        │
│                                                                    │
└──────────────────────────────────────────────────────────────────┘
```

FIG. 16

SPECIMEN SUCTION PART 211

PUMP 2112

2111

T

SAMPLE PREPARATION PART 212

DIFF HEMOLYTIC AGENT    DIFF STAINING LIQUID

DIFF REACTION CHAMBER
212a

RET HEMOLYTIC AGENT    RET STAINING LIQUID

RET REACTION CHAMBER
212b

WPC HEMOLYTIC AGENT    WPC STAINING LIQUID

WPC REACTION CHAMBER
212c

PLT-F HEMOLYTIC AGENT    PLT-F STAINING LIQUID

PLT-F REACTION CHAMBER
212d

WNR HEMOLYTIC AGENT    WNR STAINING LIQUID

WNR REACTION CHAMBER
212e

FCM DETECTION PART 221

FIG. 17

FIG. 18

SAMPLE PREPARATION PART 212

SPECIMEN SUCTION PART 211

HGB HEMOLYTIC AGENT

2112
PUMP

REACTION CHAMBER
212g

HGB DETECTION PART 223

2111

T

EP 4 752 902 A2

FIG. 19

FIG. 20

FIG. 21

FIG. 22

EP 4 752 902 A2

FIG. 23

FIG. 24

FIG. 25

R1 REAGENT       SPECIMEN      R2 REAGENT                                                                        R3 REAGENT
DISPENSING      DISPENSING    DISPENSING          FIRST        STIRRING        SECOND                            DISPENSING
   STEP            STEP          STEP         WASHING STEP       STEP       WASHING STEP                            STEP

                                                  TRANSPORT                                              TRANSPORT
                                                    STEP                                                   STEP

                    REACTION 1  REACTION 2                                                                      REACTION 3

2181: PRIMARY REACTION PART              2182: PRIMARY BF SEPARATION PART              2183: SECONDARY
                                                                                             REACTION
                                                                                             PART

   R3 REAGENT        FIRST                                                R5 REAGENT
  DISPENSING       WASHING      STIRRING          SECOND                 DISPENSING
     STEP            STEP         STEP         WASHING STEP                  STEP          MEASUREMENT STEP

TRANSPORT                                                              TRANSPORT
  STEP                                                                   STEP

                                                                           REACTION 4

2184: SECONDARY BF SEPARATION PART                          2183: SECONDARY      228: OPTICAL DETECTION
                                                                  REACTION            PART
                                                                  PART

EP 4 752 902 A2

FIG. 26

APPLICATION ⌐6

INTERFACE ⌐5

RESPONSE PART ⌐53

REQUEST ACCEPTANCE PART ⌐51

COLLABORATION PART ⌐52

RESULT PROVIDING PART ⌐41

OPERATION PART ⌐42

DATA MANAGEMENT SOFTWARE

4

FIG. 27

```
                    ┌──────────────────┐
                    │      START       │
                    └──────────────────┘
                              │
      ┌───────────────────────│
      │                       ▼
      │                      ╱╲                    S1
      │                    ╱    ╲
      │          NO      ╱        ╲
      │◄───────────────╱  REQUEST   ╲
                       ╲  RECEIVED?  ╱
                         ╲        ╱
                           ╲    ╱
                             ╲╱
                              │ YES
                              ▼
                    ┌──────────────────┐
                    │  ANALYZE REQUEST │          S2
                    └──────────────────┘
                              │
                              ▼
              ┌──────────────────────────────┐
              │ INSTRUCT EXECUTION OF PROCESS │    S3
              │   CORRESPONDING TO REQUEST    │
              └──────────────────────────────┘
                              │
                              ▼
                    ┌──────────────────┐
                    │     RESPONSE     │          S4
                    └──────────────────┘
                              │
                              ▼
                    ┌──────────────────┐
                    │       END        │
                    └──────────────────┘
```

FIG. 28

FIG. 29

```
PUT http://127.0.0.1:8080/qcdata/qcLotsInfo?lotNumber=QC22421101
{
  "Limits": [
    {
      "parameter": "WBC",
      "lowerLimit": 10.0,
      "upperLimit": 100.0,
    },
    {
      "parameter": "RBC",
      "lowerLimit": 20.0,
      "upperLimit": 200.0,
    },
    {
      "parameter": "HGB",
      "lowerLimit": 30.0,
      "upperLimit": 300.0,
    },
  ]
}
```

FIG. 30

EXAMPLE OF REQUEST FOR INFORMATION REGARDING SPECIMEN
MEASUREMENT APPARATUS AND RESPONSE TO REQUEST

| REQUEST | | RESPONSE | |
|---|---|---|---|
| REQUEST REGARDING INFORMATION UNIQUE TO APPARATUS/ SYSTEM | MODEL NAME OF SPECIMEN MEASUREMENT APPARATUS | RESPOND WITH INFORMATION UNIQUE TO APPARATUS CORRESPOND- ING TO REQUEST | MODEL NAME OF APPARATUS |
| | IDENTIFICATION INFORMATION OF SPECIMEN MEASUREMENT APPARATUS | | SERIAL NUMBER OF APPARATUS |
| | TYPE OF SPECIMEN MEASUREMENT APPARATUS | | CODE CORRESPONDING TO TYPE OF APPARATUS (EXAMPLE  01: HEMATOLOGY) |
| | SYSTEM CONFIGURATION | | SYSTEM CONFIGURATION INFORMATION (EXAMPLE: TYPE OF APPARATUS FORMING THE SYSTEM, NUMBER OF APPARATUSES, DISPOSITION OF APPARATUS) |
| | CALIBRATION VALUE | | CALIBRATION INFORMATION ON APPARATUS |
| | ADJUSTMENT INFORMATION | | ADJUSTMENT INFORMATION (EXAMPLE: SENSITIVITY ADJUSTMENT, POSITION ADJUSTMENT, SENSOR ADJUSTMENT, LASER OUTPUT) |
| | SETTING INFORMATION ON SPECIMEN MEASUREMENT APPARATUS | | INFORMATION REGARDING CONFIGURATION OF APPARATUS |
| REQUEST FOR STATUS INFORMATION ON APPARATUS | ACTIVATION STATUS OF APPARATUS | RESPOND WITH STATUS INFORMATION ON APPARATUS | INFORMATION INDICATING ACTIVATION STATUS OF APPARATUS (EXAMPLE  1: ON, 0: OFF) |
| | ERROR STATUS OF APPARATUS | | INFORMATION INDICATING ERROR STATUS OF APPARATUS (EXAMPLE  1: WITH ERROR, 0: WITHOUT ERROR) |
| | OPERATION STATUS OF APPARATUS | | INFORMATION INDICATING OPERATION STATUS OF APPARATUS (EXAMPLE  0: STANDBY, 1: DURING MEASUREMENT) |
| REQUEST FOR INFORMATION REGARDING APPARATUS OPERATION | OPERATION LOG OF APPARATUS | RESPOND WITH INFORMATION REGARDING APPARATUS OPERATION | OPERATION LOG INFORMATION |
| | INFORMATION ON SENSOR PROVIDED IN APPARATUS | | SENSOR INFORMATION |
| | POWER CONSUMPTION INFORMATION | | POWER CONSUMPTION INFORMATION |
| | ERROR INFORMATION ON APPARATUS MECHANISM | | ERROR INFORMATION |

FIG. 31    EXAMPLE OF REQUEST FOR INFORMATION REGARDING SPECIMEN MEASUREMENT AND
RESPONSE TO REQUEST

| REQUEST | | RESPONSE | |
|---|---|---|---|
| REQUEST REGARDING MEASUREMENT RESULT | REQUEST MEASUREMENT RESULT | RESPOND WITH INFORMATION REGARDING MEASUREMENT RESULT | MEASUREMENT RESULT |
| | REQUEST INFORMATION REGARDING MEASUREMENT ERROR | | ERROR INFORMATION |
| | REQUEST FLAG INFORMATION | | FLAG INFORMATION |
| REQUEST REGARDING QUALITY CONTROL | REQUEST MEASUREMENT RESULT OF QUALITY CONTROL SPECIMEN | RESPOND WITH INFORMATION REGARDING QUALITY CONTROL | MEASUREMENT RESULT |
| | REQUEST CALIBRATION CURVE DATA | | CALIBRATION CURVE DATA |
| | REQUEST LOT OF QUALITY CONTROL SPECIMEN | | LOT INFORMATION |
| REQUEST REGARDING MEASUREMENT ORDER | REQUEST MEASUREMENT ORDER | RESPOND WITH INFORMATION REGARDING MEASUREMENT ORDER | MEASUREMENT ORDER |
| | REQUEST SPECIMEN INFORMATION | | SPECIMEN INFORMATION |
| | REQUEST INFORMATION ON SPECIMEN TO BE RETESTED | | INFORMATION ON RETEST SPECIMEN |

EP 4 752 902 A2

FIG. 32          EXAMPLE OF REQUEST FOR INFORMATION REGARDING MAINTENANCE OF
                 SPECIMEN MEASUREMENT APPARATUS AND RESPONSE TO REQUEST

| REQUEST | | RESPONSE | |
|---|---|---|---|
| REQUEST FOR INFORMATION REGARDING REAGENT | REQUEST REAGENT REMAINING AMOUNT | RESPOND WITH REAGENT INFORMATION | REAGENT REMAINING AMOUNT INFORMATION |
| | REQUEST EXPIRATION DATE OF REAGENT | | EXPIRATION DATE INFORMATION |
| | REQUEST LOT INFORMATION OF REAGENT | | LOT INFORMATION |
| | REQUEST REAGENT NAME | | REAGENT NAME |
| | REQUEST NUMBER OF REMAINING TESTS | | NUMBER OF REMAINING TESTS |
| | DATE AND TIME WHEN REAGENT WAS SET | | DATE AND TIME OF SETTING REAGENT |
| | HISTORY OF REAGENT REPLACEMENT | | HISTORY INFORMATION OF REAGENT REPLACEMENT |
| REQUEST FOR INFORMATION REGARDING MAINTENANCE | REQUEST MAINTENANCE SCHEDULE INFORMATION | RESPOND WITH MAINTENANCE INFORMATION | SCHEDULE INFORMATION |
| | REQUEST MAINTENANCE HISTORY INFORMATION | | MAINTENANCE HISTORY INFORMATION |
| | REQUEST APPARATUS CALIBRATION HISTORY | | CALIBRATION HISTORY |
| | REQUEST REMAINING AMOUNT OF CONSUMABLES | | REMAINING AMOUNT INFORMATION |

# FIG. 33

EXAMPLE OF REQUEST REGARDING MANIPULATION OF
SPECIMEN MEASUREMENT APPARATUS

| REQUEST | | RESPONSE | |
|---|---|---|---|
| REQUEST REGARDING SPECIMEN MEASUREMENT | MEASUREMENT ORDER REGISTRATION | RESPONSE REGARDING WHETHER OR NOT MANIPULATION HAS BEEN COMPLETED | ACK or NACK |
| | REGISTRATION/EDITING/DELETION OF RERUN/REFLEX RULE | | ACK or NACK |
| | REGISTRATION/EDITING/DELETION OF CALIBRATION CURVE | | ACK or NACK |
| REQUEST REGARDING MAINTENANCE | REGISTRATION/EDITING/DELETION OF REAGENT INFORMATION | RESPONSE REGARDING WHETHER OR NOT MANIPULATION HAS BEEN COMPLETED | ACK or NACK |
| | REGISTRATION/EDITING/DELETION OF MAINTENANCE SCHEDULE | | ACK or NACK |
| | REGISTRATION/EDITING/DELETION OF SYSTEM BACKUP SCHEDULE | | ACK or NACK |
| | REGISTRATION/EDITING/DELETION OF CONSUMABLES INFORMATION | | ACK or NACK |
| REQUEST REGARDING APPARATUS MANIPULATION | ACTIVATION/DEACTIVATION OF APPARATUS | RESPONSE REGARDING WHETHER OR NOT MANIPULATION HAS BEEN COMPLETED | ACK or NACK |
| | REGISTRATION/EDITING/DELETION OF APPARATUS SETTING | | ACK or NACK |
| | EXECUTION OF APPARATUS CONTROL, MEASUREMENT EXECUTION, RETEST EXECUTION, QC MEASUREMENT EXECUTION, PROCESSING DURING APPARATUS ERROR, etc. | | ACK or NACK |

FIG. 34

FIG. 35

| EVENT | INFORMATION CORRESPONDING TO EVENT (INFORMATION TO SERVE AS PUSH NOTIFICATION TARGET) |
|---|---|
| COMPLETION OF MEASUREMENT | MEASUREMENT RESULT |
| COMPLETION OF QUALITY CONTROL MEASUREMENT | QC MEASUREMENT RESULT |
| OCCURRENCE OR ELIMINATION OF APPARATUS ERROR | MANIPULATION HISTORY |
| REAGENT REPLACEMENT | REAGENT REPLACEMENT HISTORY/REAGENT INFORMATION |
| MAINTENANCE EXECUTION | MAINTENANCE HISTORY |
| SETTING INFORMATION CHANGE | · SETTING INFORMATION ON DATA MANAGEMENT SOFTWARE<br>· APPARATUS SETTING<br>· RETEST RULE<br>· REFLEX RULE |
| CALIBRATION INFORMATION CHANGE | CALIBRATION INFORMATION |

EP 4 752 902 A2

FIG. 36

FIG. 37

FIG. 38

| Device | REAGENT A | REAGENT B | REAGENT C |
|---|---|---|---|
| XR-1000 HEMATOLOGY ID: xxx1 | LOT: A111<br>REMAINING AMOUNT: 50 TESTS<br>PLACEMENT DATE: 2023/10/14 | LOT: B100<br>REMAINING AMOUNT: 20 TESTS<br>PLACEMENT DATE: 2023/8/31 | LOT: C200<br>REMAINING AMOUNT: 20 TESTS<br>PLACEMENT DATE: 2023/9/15 |
| XR-1000 HEMATOLOGY ID: xxx2 | LOT: A111<br>REMAINING AMOUNT: 70 TESTS<br>PLACEMENT DATE: 2023/11/14 | LOT: B101<br>REMAINING AMOUNT: 80 TESTS<br>PLACEMENT DATE: 2023/9/3 | LOT: C200<br>REMAINING AMOUNT: 30 TESTS<br>PLACEMENT DATE: 2023/9/20 |
| XR-1000 HEMATOLOGY ID: xxx3 | LOT: A111<br>REMAINING AMOUNT: 30 TESTS<br>PLACEMENT DATE: 2023/9/10 | LOT: B101<br>REMAINING AMOUNT: 85 TESTS<br>PLACEMENT DATE: 2023/9/21 | LOT: C200<br>REMAINING AMOUNT: 35 TESTS<br>PLACEMENT DATE: 2023/9/22 |
| XR-1000 HEMATOLOGY ID: xxx4 | LOT: A111<br>REMAINING AMOUNT: 20 TESTS<br>PLACEMENT DATE: 2023/9/10 | LOT: B101<br>REMAINING AMOUNT: 90 TESTS<br>PLACEMENT DATE: 2023/10/1 | LOT: C200<br>REMAINING AMOUNT: 40 TESTS<br>PLACEMENT DATE: 2023/9/29 |

FIG. 39

APPLICATION 6

FUNCTION PROVIDING PART 61

MEASUREMENT DATA PROVIDING PART 615

MEASUREMENT DATA ACQUISITION PART 614

APPARATUS INFORMATION ACQUISITION PART 611

RESPONSE ACCEPTANCE PART 63

REQUESTING PART 62

FIG. 40

| SPECIMEN NUMBER | — | XR-1000 ▼ | 2023/09/30 ~ 2023/11/14 🔍 |

| MEASUREMENT DATE AND TIME 2023/09/10 9:00 | SPECIMEN NUMBER 1234567 | MEASUREMENT RESULT ABC | XR-1000 HEMATOLOGY ID: xxx1 |

| MEASUREMENT DATE AND TIME 2023/09/10 11:00 | SPECIMEN NUMBER 1234568 | MEASUREMENT RESULT BBB | XR-1000 HEMATOLOGY ID: xxx2 |

| MEASUREMENT DATE AND TIME 2023/09/10 12:00 | SPECIMEN NUMBER 1234569 | MEASUREMENT RESULT CCC | XR-1000 HEMATOLOGY ID: xxx3 |

| MEASUREMENT DATE AND TIME 2023/11/14 9:00 | SPECIMEN NUMBER 1234560 | MEASUREMENT RESULT DDD | XR-1000 HEMATOLOGY ID: xxx4 |

FIG. 41

FIG. 42

FIG. 43

FIG. 44

NORMALLY RUNNING

ERROR HAS OCCURRED, STOPPED

## FIG. 45

TEST DATE ◀2023/11/10▶ NUMBER OF CASES 71,600 CASES

| | | | | | | | | | | | | | | |
|---|---|---|---|---|---|---|---|---|---|---|---|---|---|---|
| 1 | DI 02 | SP 02 | DI 01 | SP 01 | XR 08 | XR 07 | XR 06 | XR 05 | XR 04 | XR 03 | XR 02 | XR 01 | BT | RM |
| 2 | DI 04 | SP 04 | DI 03 | SP 03 | XR 16 | XR 15 | XR 14 | XR 13 | XR 12 | XR 11 | XR 10 | XR 09 | BT | RM |
| 3 | DI 06 | SP 06 | DI 05 | SP 05 | XR 24 | XR 23 | XR 22 | XR 21 | XR 20 | XR 19 | XR 18 | XR 17 | BT | RM |
| 4 | DI 08 | SP 08 | DI 07 | SP 07 | XR 32 | XR 31 | XR 30 | XR 29 | XR 28 | XR 27 | XR 26 | XR 25 | BT | RM |
| 5 | DI 10 | SP 10 | DI 09 | SP 09 | XR 40 | XR 39 | XR 38 | XR 37 | XR 36 | XR 35 | XR 34 | XR 33 | BT | RM |
| 6 | DI 12 | SP 12 | DI 11 | SP 11 | XR 48 | XR 47 | XR 46 | XR 45 | XR 44 | XR 43 | XR 42 | XR 41 | BT | RM |
| 7 | DI 14 | SP 14 | DI 13 | SP 13 | XR 56 | XR 55 | XR 54 | XR 53 | XR 52 | XR 51 | XR 50 | XR 49 | BT | RM |

REAGENT REPLACEMENT INFORMATION 4 CASES

[REPLACE] REAGENT HAS ALREADY RUN OUT 1 CASE

| LINE No | APPARATUS NAME | INFORMATION |
|---|---|---|
| 1 | XR-01 | FLUOROCELL WNR |

REAGENT WILL RUN OUT WITHIN 12 HOURS 1 CASE

| LINE No | APPARATUS NAME | INFORMATION | ESTIMATION |
|---|---|---|---|
| 3 | XR-18 | FLUOROCELL WDF | WITHIN 6h |

REAGENT WILL RUN OUT WITHIN 24 HOURS 2 CASES

| LINE No | APPARATUS NAME | INFORMATION | ESTIMATION |
|---|---|---|---|
| 4 | XR-30 | FLUOROCELL RET | WITHIN 18h |
| 3 | XR-18 | FLUOROCELL WNR | WITHIN 22h |

CONSUMABLES REPLACEMENT INFORMATION 2 CASES

[REPLACE] CONSUMABLES HAVE ALREADY RUN OUT 0 CASES

| LINE No | APPARATUS NAME | INFORMATION |
|---|---|---|
| | | |

CONSUMABLES WILL RUN OUT WITHIN 12 HOURS 1 CASE

| LINE No | APPARATUS NAME | INFORMATION |
|---|---|---|
| 3 | DI-05 | IMMERSION OIL SHORTAGE |

CONSUMABLES WILL RUN OUT WITHIN 24 HOURS 1 CASE

| LINE No | APPARATUS NAME | INFORMATION |
|---|---|---|
| 6 | BT-06 | WASH SPECIMEN SHORTAGE |

■ REAGENT/CONSUMABLES HAVE ALREADY RUN OUT

▨ REAGENT/CONSUMABLES WILL RUN OUT WITHIN 12 HOURS

▦ REAGENT/CONSUMABLES WILL RUN OUT WITHIN 24 HOURS

EP 4 752 902 A2

FIG. 46

WEB SERVICE SYSTEM — 301

CONTROLLER — 302

SERVICE FUNCTION PROVIDING PART — 6A

FUNCTION PROVIDING PART — 61A

RESPONSE ACCEPTANCE PART — 63A

REQUESTING PART — 62A

SERVICE FUNCTION PROVIDING PART — 6A

COMMUNICATION NETWORK — 205

CONTROL UNIT — 7

WEB BROWSER — 8

INTERFACE — 5

DATA MANAGEMENT SOFTWARE — 4

CONTROL SOFTWARE — 3

MEASUREMENT UNIT — 2

SPECIMEN MEASUREMENT APPARATUS — 11

FIG. 47

FIG. 48

FIG. 49

APPLICATION PROVIDING SERVER 401

APPLICATION STORAGE 405

CONTROLLER 402

DOWNLOAD REQUEST ACCEPTANCE PART 403

APPLICATION PROVIDING PART 404

FIG. 50

FIG. 51

MDM/MAM SYSTEM — 601

DATABASE — 604

MDM/MAM MANAGEMENT TERMINAL — 701

MANAGEMENT FUNCTION PROVIDING PART — 602

MDM/MAM EXECUTION PART — 603

COMMUNICATION NETWORK — 207

TERMINAL APPARATUS — 101

SPECIMEN MEASUREMENT APPARATUS — 11

FIG. 52

USER IDENTIFICATION INFORMATION

ASSOCIATED ORGANIZATION

DEVICE TYPE

DEVICE IDENTIFICATION INFORMATION

POLICY

APPLICATION LIST

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024035313 A
- US 2024051365 A
- US 2024051366 A
- WO 2013094485 A